# EUROPEAN PATENT SPECIFICATION

(11) **EP 1 159 415 B1**
(45) Date of publication and mention of the grant of the patent: **23.12.2009**
(21) Application number: 00907792.6
(22) Date of filing: 03.03.2000
(51) Int. Cl.: C12N 15/10, C12N 15/90, C12N 15/88, C12N 5/06, A01K 67/027

(54) **GENETIC MODIFICATION OF SOMATIC CELLS AND USES THEREOF**
GENETISCHE VERÄNDERUNG VON SOMATISCHEN ZELLEN UND DEREN VERWENDUNGEN
MODIFICATION GENETIQUE DE CELLULES SOMATIQUES ET LEURS UTILISATIONS

(30) Priority: 04.03.1999 GB 9905033; 20.07.1999 GB 9917023; 30.12.1999 US 475674
(43) Date of publication of application: 05.12.2001
(73) Proprietor: REVIVICOR, INC., Blacksburg, VA 24060 (US)
(72) Inventor: COLMAN, Alan, Midlothian EH25 9PP (GB); SCHNIEKE, Angelika Elisabeth, Midlothian EH25 9PP (GB); KIND, Alexander Jarvis, Midlothian EH25 9PP (GB); AYARES, David Lee, Blacksburg, VA 24060 (US); DAI, Yifan, Blacksburg, VA 24060 (US)
(74) Representative: Howard, Paul Nicholas
(86) International application number: PCT/GB2000/000778
(87) International publication number: WO 2000/051424

(56) References cited:
- WO-A-97/07668
- WO-A-97/07669
- WO-A-98/30683
- WO-A-98/37183
- WO-A-98/56903
- POLEJAEVA, I.A. AND CAMPBELL, K.H.S.: "New advances in somatic cell nuclear transfer: Application in transgenesis" THERIOGENOLOGY, vol. 53, no. 1, 1 January 2000 (2000-01-01), pages 117-126, XP000940942
- SCHNIEKE, A.E. ET AL.: "Human factor IX transgenic sheep produced by transfer of nuclei from transfected fetal fibroblasts" SCIENCE, vol. 278, 19 December 1997 (1997-12-19), pages 2130-2133, XP002067036 cited in the application
- CIBELLI, J.B. ET AL.: "Embryonic stem cells: Bovine chimeric offspring produced by transgenic embryonic stem cells generated from somatic cell nuclear transfer embryos" ANNUAL CONFERENCE OF THE INTERNATIONAL EMBRYO TRANSFER SOCIETY, vol. 49, no. 1, 18 January 1998 (1998-01-18), page 236 XP000764040
- ROBL. J.M. ET AL.: "Somatic cell nuclear transplantation in cattle" ANNUAL MEETING SOC. STUDY REPROD, vol. 58, 8 August 1998 (1998-08-08), page 25 XP000791151
- CAMPBELL, K.H.S. AND WILMUT, I.: "Totipotency or multipotentiality of cultured cells: Applications and progress" THERIOGENOLOGY, vol. 47, 1997, pages 63-72, XP000940776
- WAKAYAMA, T. ET AL.: "Full-term development of mice from enucleated oocytes injected with cumulus cell nuclei" NATURE, vol. 394, 23 July 1998 (1998-07-23), pages 369-374, XP002900702 cited in the application
- MCCREATH, K.J. ET AL.: "Production of gene-targeted sheep by nuclear transfer from cultured somatic cells" NATURE, vol. 405, 29 July 2000 (2000-07-29), pages 1066-1069, XP000919025

## Description

This invention describes the production of genetically modified animals in which the genetic modifications are engineered in somatic cells cultured *in vitro* using the technique of gene targeting. Genetically modified cells are then used as nuclear donors to produce *inter alia*, live animals.

The methods described can also be used to validate loci in animal chromosomes which are suitable sites for transgene addition to cells. The method is particularly useful for cells that are required to have no unanticipated detrimental effect, such as cells destined for autologous transplantation.

The technique of nuclear transfer allows the production of offspring by the reconstruction of an early embryo. Genetic material from a donor cell or karyoplast is transferred to a suitable recipient cell from which the nuclear or genomic genetic material has been removed. In the first demonstrations of this technique, successful development was only obtained when the donor genetic material was taken from blastomeres from early embryos. Subsequently, development has been obtained using donor genetic material from differentiated cells maintained in culture and isolated from embryonic (Campbell et al., Nature 380, 64-66, 1996), fetal and adult tissues (Wilmut et al., Nature 385, 810-813, 1997); these reports form the basis of patent applications WO 97/07669 and WO 97/07668 which are incorporated into the present application in full, including all tables and diagrams.

Methods of nuclear transfer have also been described in published patent applications WO98/39416, WO98/30683, WO98/07841, WO97/37009, WO98/27214, WO99/01163 and WO99/01164.

Live offspring have been obtained in the mouse using quiescent cell populations derived directly *ex vivo* as nuclear donors (Wakayama et al., Nature 394, 369-373 1998). The successful use of differentiated cells has also been demonstrated in sheep (Wilmut et al., Nature 385, 810-813, 1997), cattle (Kato et al., Science 282, 2095-2098, 1998; Wells, et al., Theriogenology 1, 217, 1999; Zakhartchenko, et al., Theriogenology 1, 218, 1999; Vignon, et al., Theriogenology 1, 216, 1999) and mice (Wakayama et al., Nature 394, 369-373).

In all the above cited references the nuclear donor and the recipient cell are taken from the same species. However, there has been success reported in achieving development from embryos reconstructed using nuclear donor and recipient cells from different species (Dominko, et al., Theriogenology 49, 385, 1998; Mitalipova, et al., Theriogenology 49, 389, 1998).

The use of nuclear transfer technology has many proven and potential benefits and uses in the production of mammalian embryos, fetuses and offspring. These include but are not limited to;
1. The ability to carry out genetic modification of cultured cells to be used as nuclear donors prior to embryo reconstruction.
2. The ability to carry out multiple genetic modifications in a single animal either by multiple genetic modifications of a cell population in culture or by sequential genetic modification, nuclear transfer and re-isolation of a cell population from the embryo, fetus or animal so produced.
3. The ability to increase the lifespan of cultured cell populations to be used for genetic modification by nuclear transfer and re-isolation of a cell population from the embryo, fetus or adult animal so produced.
4. The ability to produce multiple copies of an animal from a genetically modified, selected and cloned cell population.
5. The ability to produce multiple copies of any embryo, fetus or adult animal by nuclear transfer from cells taken directly *ex vivo,* or cell populations derived from any tissues taken from any of these stages with or without culture *in vitro.*
6. The ability to produce true clones (which share not only nuclear genetic identity, but also mitochondrial genetic identity) by utilising oocytes from the maternal line of the cell donor as cytoplast recipients for embryo reconstruction.
7. The ability to store intact genomes for long periods (e.g. by freezing cell populations in liquid N₂) and to use these stored cells subsequently for the production of offspring by nuclear transfer.
8. The ability to dedifferentiate somatic nuclei and to produce undifferentiated cells that may be used to produce chimeric embryos, fetuses and adult animals by embryo aggregation, or injection. This can also be used to produce populations of embryonic stem, or embryonic germ cell populations.
9. The ability to dedifferentiate any somatic cell type by nuclear transfer and to isolate from the embryo so produced, embryonic stem cells, or any other desired specialised or unspecialised cell type e.g. neurones.
10. The possibility of achieving any of the objectives outlined in 1-9, by using nuclear donor and recipient cells from different species.

This process may be coupled with genetic manipulation techniques for the production of transgenic offspring (Schnieke et al., Science 278, 2130-2133, 1997). The use of nuclear transfer coupled to genetic modification of cells in culture and their selection prior to animal production has a number of proven advantages, including;
1. Production of non-mosaic animals ensuring germ line transmission of the genetic modification/s (Schnieke *et al., Supra*).
2. An increased efficiency in the production of such genetically modified animals (Schnieke *et al., Supra*).
3. The production of multiple copies of the offspring thereby reducing the generation interval to produce flocks or herds of commercially important animals or increasing the numbers of animals for dissemination of genetic modification into the population as a whole (Cibelli, et al.,. Nat. Biotechnol. 16, 642-6, 1998)

The existing and published nuclear transfer technology coupled to genetic modification of cells in culture provides animals containing single and multiple genetic modifications where the transgenes are incorporated at unselected locations within the host genome. However, the production of cultured cells which incorporate a desired genetic modification engineered at a precise and predetermined location in the host genome (gene targeting) has only been described previously in the art for the mouse using ES cells and the published methods do not involve nuclear transfer technology. No such equivalent methods exist for other mammals. Such modifications would have a number of advantages when applied to a variety of animal species including cattle, sheep, goats, horses, camels, rabbits and rodents. Such advantages include, but are not limited to:
1. The production of transgenic animals with superior transgene expression characteristics by placing the transgene at a predetermined site.
2. The removal, modification, inactivation or replacement of a chosen endogenous gene or genes and /or its control sequences.
3. If such modifications are found to have no unanticipated, detrimental effect on the resulting animal that could be ascribed to, for example, disruption of endogenous genes, activation of oncogenes, etc., this would constitute validation of the chosen locus as a preferred site for genetic modification, particularly if the site also conferred good expression of transgenes placed at the locus.

It is an objective of this patent specification to rectify this situation and describe for the first time, methods which can be utilised to produce such modified cells which can then optionally be used to make, *inter alia* whole animals by nuclear transfer. These methods (and others) can also be used to identify specific chromosomal loci as preferred targets for transgene addition as part of the process of *ex-vivo* gene therapy.

The present invention provides, according to a first aspect, a method of nuclear transfer comprising: a preparing a non-human somatic cell for nuclear transfer by a method comprising modifying the genetic material of the non-human somatic cell by a genetic targeting event at an endogenous locus; and (b) transferring the genetic material from the non-human somatic cell to a non-human recipient cell. Preferably the non-human somatic cell is a primary somatic cell. Preferably the somatic cell is a vertebrate animal somatic cell. Preferably the cell is not an immortalised cell. Traditionally, cells can be defined as either "somatic", or "germ-line". Some cells, e.g. ES cells may not fall easily in either of these two traditional categories because they are derived from embryos before distinct somatic and germ lineages can be distinguished. Their functional equivalent, EG (Embryonic germ) cells are more easily defined as "germ-line" cells because they are derived from primordial germ cells. In the present text, the term "somatic" does not cover ES or EG cells.

The use of this method is not restricted to a particular donor cell type. Suitable cells include embryonic, fetal and adult somatic cells (of normal karyotype). In this text an "adult" cell or an "adult" animal is a cell or animal which is born. Thus an animal and its cells are deemed "adult" from birth. Such adult animals, in fact, cover animals from birth onwards, and thus include "babies" and "juveniles". The invention includes the use of undifferentiated e.g. somatic stem cells such as haematopoietic stem cells, at least partially differentiated and fully differentiated cells. Examples of partially differentiated cells include cells of embryonic lineage or precursor cells (e.g. neural precursor cells).

Suitable somatic cells according to the first aspect of the invention are preferably, but not necessarily, in culture. Suitable somatic cells include fibroblasts, epithelial cells, endothelial cells, neural cells, keratinocytes, hematopoietic cells, melanocytes, chondrocytes, lymphocytes (B and T), avian erythrocytes, macrophages, monocytes, mononuclear cells, cardiac muscle cells, other muscle cells, granulosa cells, cumulus cells and epidermal cells. The cells for the method of the first aspect of the invention may be obtained from a variety of different organs such as skin, mesenchyme, lung, pancreas, heart, intestine, stomach, bladder, major blood vessels kidney, urethra, reproductive organs etc. or a disaggregated preparation of a whole or part of a fetus or embryo.

Suitable cells may be obtained or derived from any non-human animal, including birds such as domestic fowl, amphibian species and fish species. In practice however, it is mammalian animals that the greatest commercial advantage is envisaged. A preferred animal from which the somatic cell is obtained is an ungulate, selected from a bovid, ovid, cervid, suid, equid or camelid. In particular, the ungulate is a cow or bull, sheep, goat, bison, water buffalo or pig. Also contemplated by the present invention are somatic cells obtained or derived from a horse, camel, rodent (e.g. rat, mice) or a lagomorph (e.g. rabbit).

Suitable sources for non-human recipient cells in the nuclear transfer process are not limiting. The recipient cell is preferably an non-human oocyte, a non-human fertilised zygote or a non-human two cell embryo, all of which have been enucleated.

The genetic targeting event may be any which enables modification of the genetic material of the somatic cell at a predetermined site. Such a genetic targeting event includes inactivation, removal or modification of genetic material: upregulation of the function of genetic material; replacement of genetic material; and introduction of genetic material. The genetic material in particular comprises a gene or a part thereof or a region which influences the expression of a gene or genes. Accordingly, the genetic target events may include inactivation, removal or modification of a gene, upregulation of a gene, gene replacement or transgene replacement at a predetermined locus.

Preferably the genetic targeting event occurs by homologous recombination.

Preferably, the derivation of gene targeted cell clones occurs at high efficiency. According to this aspect of the invention, high efficiency is defined as a ratio of gene targeted:randomly transfected cell clones within the same selected cell population which is equal to or greater than 1:100.

The precise details of genetic targeting can be varied to improve the efficiency. In accordance with the variety of modifications, genetic targeting of a somatic cell suitable for nuclear transfer can be designed to maximise the frequency of homologous targeting. One factor which can be used to increase genetic targeting by homologous recombination is the effect of transcription on the target genetic material. Preferably, the genetic target is actively transcribed or is adjacent to a genetic locus which is actively transcribed. Such genetic targets or loci can be identified depending on the type of somatic cell used, and also possibly on the stage of such a cell. Such genes can be identified on the basis of the abundance of the corresponding mRNA molecules. Suitable genes would produce mRNAs which fall into the arbitrarily defined intermediate, or abundant class of mRNAs which are present at 300 or more copies of each molecule per cell (Alberts et al. 1994, Molecular Biology of the Cell, Garland Publishing, New York and London). An example of a suitable gene target in fibroblasts is any gene or locus encoding collagen, in particular the COLIA1 or the COLIA2 gene loci. However many suitable loci are present in a cell's genetic material, such as milk protein genes, for example in mammary epithelial cells; immunoglobulins in lymphocytes; HSA and transferrin in liver cells; and type VII collagen in keratinocytes.

According to this aspect of the invention, efficient means of transfection which maximises the number of cell clones derived which have undergone a gene targeting event may be used. For example, lipid mediated transfection reagents e.g. "LipofectaAMINE" (Gibco / Life Sciences) or "GenePorter" (Gene Therapy Systems) may be used.

Alternatively, or in addition, long regions of homology to the target locus may be included in the gene targeting vector. The total length of homology present in the targeting vector is preferably greater than 7kb.

Alternatively, or in addition, non-linear gene targeting vector DNA, *i.e.* DNA which has not been linearised by restriction enzyme cleavage, may be used. In this embodiment of the invention, the DNA transfected is predominantly in a circular form, which may be substantially supercoiled or substantially relaxed.

The above features of the methods of transfection may be used alone or in combination with each other.

A particularly useful strategy to increase genetic targeting is the artificial induction of gene expression or induction of chromatin changes in a somatic cell type. Preferably the genetic targeting event is facilitated by an agent which inhibits histone deacetylation or by a factor which stimulates transcription at the target locus. The factor may be expressed in the host cell. This strategy is described in more detail in the remainder of this text.

In accordance with the genetic targeting of the present invention, it may be useful or necessary, at some point after the event and preferably before any nuclear transfer to remove portions of genetic material from the cell. Such material may be a selectable marker, or for example, an introduced genetic transcription activator. Such removal can be carried out by procedures described hereinafter , or by other procedures well known in the art.

The preparation of the somatic cell (by modification of the genetic material of the cell) may be a precursor step to nuclear transfer. The present invention provides, for the first time, a method by which a somatic cell can be genetically modified and which also supports successful nuclear transfer. Supporting successful nuclear transfer requires that the reconstituted embryo proceeds to produce either a live born viable animal, or an embryo or fetus which can be used as a source of tissue, including EG cells and ES cells.

The method of nuclear transfer for the present invention is not limited. Any method of nuclear transfer may be used. The nuclear transfer may include genetic material from one animal species or one animal type to a recipient cell of the same or different animal species or type.

The recipient cells may be any suitable recipient cell for any method of nuclear transfer, including an oocyte, a fertilised zygote or a two cell embryo which has been enucleated.

The transfer of the genetic material from a somatic cell to a recipient cell provides an animal embryo (the animal embryo comprises the single cell result of the nuclear transfer through to any and all multi-cell stage).

The method of the invention may also comprise the production of cloned totipotent or pluripotent cells from an embryo or fetus derived by nuclear transfer. Methods for the derivation of totipotent or pluripotent cells, such as stem cells, from embryos (ES cells and ES-like cells) and from primordial germ cells of later stage embryos and fetuses (EG cells) have been described by many authors as cited later in this text. Pluripotent stem cells are particularly useful for the production of differentiated cells and their precursors *in vitro* which may provide a source of cells for transplantation (preferably for humans).

Cells (including somatic cells) derived from embryos or fetuses (or adults) may also be used for further rounds of nuclear transfer. Rederivation of cells from an embryo or fetus produced by nuclear transfer may facilitate multiple or sequential genetic manipulations which are otherwise not possible in the initial primary cells.

Alternatively, the method of obtaining an animal embryo according to the invention may further comprise causing an animal to develop to term from the embryo. In such a method, the animal embryo is preferably developed to term *in vivo.* If development up to blastocyst has taken place *in vitro,* then transfer into a surrogate animal takes place at this state. If blastocyst development has taken place *in vivo,* although in principal the blastocyst can be allowed to developed to term in the pre-blastocyst host, in practice the blastocyst will usually be moved from the temporary pre-blastocyst recipient and, after dissection from the protective medium, will be transferred to the permanent post blastocyst recipient. Development from the embryo to an adult goes through the stage of a fetus.

A second aspect of the invention provides a transgenic embryo or fetus obtained by a method according to the invention. Preferred features of aspect one also apply to the second aspect.

A third aspect of the invention provides a method for preparing a transgenic animal comprising causing a non-human animal to develop to term from a non-human embryo or fetus according to the second aspect of the invention. Optionally, the transgenic non-human animal of the third aspect may breed and such offspring (including embryos, fetuses and adults) are also encompassed by the present invention. Preferred features of aspects one and two also apply to the third aspect.

A fourth aspect of the invention provides a transgenic animal, or offspring thereof, according to the third aspect of the invention. Preferred features of aspects one to three also apply to the fourth aspect.

The nuclear transfer methods of the present invention encompass, but are not limited to, a double nuclear transfer method. Such a method, as well as details of single-step nuclear transfer methods, is described in WO 0042174 and is described below.

The invention does not relate to human reproductive cloning.
Thus the "recipient cell", "totipotent cell population" "transgenic embryo", "transgenic cell", "somatic cell" and "animal embryo" used with the invention do not include human cells or embyos

In general, oocytes arrested at metaphase of the second meiotic division have been used as cytoplast recipients. The donor genetic material has been introduced into the recipient cell cytoplasm by the processes of 1) cell fusion 2) injection of intact cells, lysed cells or nuclei. Transfer of genetic material may occur either at the time of activation, preceding activation (see patent applications WO 97/07669 and WO 97/07668 or following activation (Campbell et al., Biol. Reprod. 49 9330942 (1993); Campbell et al Biol. Reprod. 50 1385-1393 (1994)). In each of these instances the ploidy of the reconstructed embryo must be maintained by the use of donor genetic material at an appropriate stage of the cell cycle (for review see Campbell et al., Reviews of Reproduction 1:40-46 (1996)). This process may be coupled with genetic manipulation techniques for the production of transgenic offspring (Schnieke et al., Science 278:2130-2133 (1997)). The use of nuclear transfer coupled to genetic modification of cells in culture and their selection prior to animal production has a number of advantages as described above.

In pigs, the use of nuclear transfer for the production of live offspring has been associated with difficulties. Although offspring have been produced by swapping the pronuclei of fertilised zygotes, only 1 piglet has been produced from a later developmental stage (4 cell) (Prather et al., Biol Reprod 1989 Sep 41:3 414-8).

The process of embryo reconstruction and production of viable offspring by nuclear transfer is a multistep procedure. Each of these steps will now be described in more detail. The description below is intended to give explanation to the steps and is not an exhaustive nor limiting description.

### The Recipient Cell or Cytoplast.

Oocytes, fertilised zygotes and two cell embryos have been used as cytoplast recipients for nuclear transfer. In general oocytes arrested at metaphase of the second meiotic division (also termed unfertilised eggs) have become the cytoplast of choice. At this point in oocyte development the genetic material is arranged upon the meiotic spindle and is easily removed using mechanical means. Several reports have demonstrated that during maturation i.e. between the germinal vesicle stage (prophase of the first meiotic division) and arrest at metaphase of the second meiotic division genomic DNA can be removed and the resulting cytoplast used for nuclear transfer (Kato Y., Tsunoda Y., Mol Reprod Dev (1993) Oct 36:2 276-8). The use of fertilised zygotes as cytoplast recipients has been reported in mouse (Kwon O.Y., Kono T., Proc Natl Acad Sci U.S.A (1996) Nov 12, 93:23 13010-3), cattle (Prather R.S., First N.L., J Reprod Fertil Suppl (1990) 41:), and pigs (Prather et al., Biol Reprod (1989) Sep 41:3, 414-8). In cattle and pigs, development of embryos reconstructed using zygotes as cytoplast recipients is low and on the whole restricted to the exchange of pronuclei, suggesting that factors essential for successful development are removed with the pronuclei. The double nuclear transfer method produces a pronucleus-like structure (including a pronucleus) by transfer of a suitable nucleus, preferably to an enucleated Metaphase II oocyte. The pronucleus-like structure thus formed will contain factors, which are generally removed during zygote enucleation. This pronucleus-like structure is then used as a donor of genetic material for a second nuclear transfer embryo reconstruction. Nuclei or cells of any cell cycle stage may be used as donors of genetic material preferably however; the cell cycle stage of the recipient cytoplast must be controlled accordingly.

### Preparation of a Cytoplast Recipient by Removal of the Genomic Genetic Material.

This process has in general been termed enucleation. In the majority of recipients utilised, the genomic DNA is not enclosed within a nuclear membrane at the time of removal. The removal of the genetic material according to the present invention and described by the term "enucleation" does not require that the genetic material is present in a nuclear membrane (it may or may not be, or may partially be). Enucleation may be achieved physically by actual removal of the nucleus, pronuclei or metaphase plate (depending on the recipient cell), or functionally, such as by the application of ultra-violet radiation or another enucleating influence. Removal of the genetic material is possible by physical and/or chemical means. In the early reports of nuclear transfer, MII oocytes were simply cut in half on the basis that one half would contain the genetic material and the other would not. Modifications to this approach have been made in order to reduce the volume of cytoplasm, which was removed. This may be achieved by aspiration of a small amount of cytoplasm from directly beneath the first polar body using glass micropipettes or by using a knife to cut away that part of the oocyte beneath the polar body. To facilitate plasticity of the oocyte it may be pre-treated with the microtubule inhibitor Cytochalasin B or other such agent that disrupts the cytoskeleton. In contrast to physical enucleation, chemical treatment has been demonstrated to cause complete removal of the genetic material in the mouse.

Treatment of maturing oocytes with the Topoisomerase inhibitor ectoposide results in the expulsion of all genomic material with the first polar body (Elsheikh A.S. et al Jpn J Vet Res (1998) Feb 45:4, 217-20), however no development to term has been described using cytoplast recipients produced by this method and there are no reports of this procedure in other species. Centrifugation of MII oocytes combined with Cytochalasin B treatment has been reported to cause enucleation in hamster and cattle oocytes (Tatham et al., Hum Reprod (1996) Jul 11:7 1499-503). The development of embryos reconstructed from such cytoplasts has been reported in cattle however the frequency of development is low.

When using zygotes, the genetic material may be removed by mechanical aspiration of both pronuclei. Dependent upon species, in order to facilitate visualisation of the pronuclei the zygotes may be centrifuged prior to enucleation.

### Introduction of genetic material (embryo reconstruction).

Having prepared a suitable recipient cell or cytoplast the donor genetic material must be introduced. Various techniques have been reported including;
1. Cell fusion induced by chemical, viral or electrical means.
2. Injection of an intact cell by any method
3. Injection of a lysed or damaged cell.
4. Injection of a nucleus.
   Any of these methods may be used in any species with some modifications of individual protocols.

### Activation of the reconstructed embryo.

In addition to the transfer of donor genetic material from the karyoplast to the cytoplast, the cytoplast must be stimulated to initiate development. When using a fertilised zygote as a cytoplast recipient development has already been initiated by sperm entry at fertilisation. When using MII oocytes as cytoplast recipients the oocyte must be activated by other stimuli. Various treatments have been reported to induce oocyte activation and promote early embryonic development including, but not limited to; application of a DC electric stimulus, treatment with ethanol, ionomycin, Inositol tris-phosphate (IP₃), calcium ionophore A23187, treatment with extracts of sperm or any other treatment which induces calcium entry into the oocyte or release of internal calcium stores and results in initiation of development. In addition any of these treatments, alone or in combination, their application at the same or different times or in combination with inhibitors of protein synthesis (i.e. cycloheximide or puromycin) or inhibitors of serine threonine protein kinases (i.e. 6-DMAP) may be applied.

### Culture of reconstructed embryos.

Nuclear transfer reconstructed embryos may be cultured *in vitro* to a stage suitable for transfer to a final recipient using any suitable culture medium or culture process. Alternatively, embryos may be cultured *in vivo* in the ligated oviduct of a suitable host animal (in general sheep) until a stage suitable for transfer to a final surrogate recipient is reached. Embryos from cattle, sheep and other species may be cultured in a trans species recipient, for simplicity a sheep provides a suitable recipient for bovine, ovine and porcine species. In order to prevent mechanical damage or attack by macrophages to the reconstructed embryos whilst in the oviduct of the temporary recipient it is usual to embed the embryos in a protective layer of agar or similar material.

The *in vitro* culture systems presently available for porcine embryos support development to the blastocyst stage, however, the frequency of live births from such embryos is low (Machaty et al., Biology of Reproduction, (1998) 59, 451-455). In the double nuclear transfer method, the first nuclear transfer reconstructed embryo may be cultured by any of these methods until the formation of a pronuclear-like body occurs and the embryo is used for the second nuclear transfer reconstruction. In practice it is presently preferred that this first culture period is carried out using a suitable medium *in vitro.*

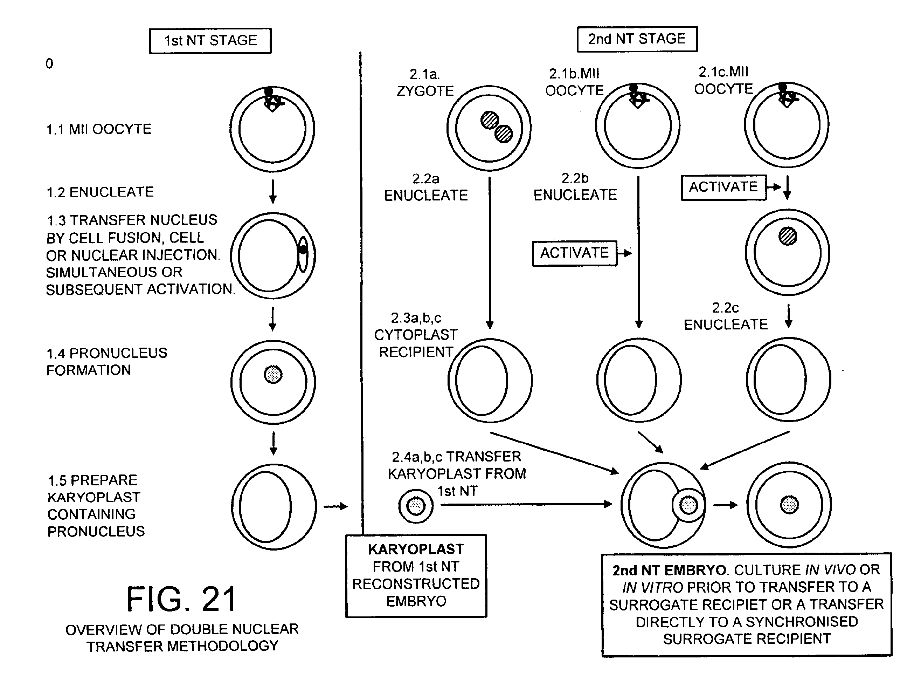

Each of the steps involved in the reconstruction of mammalian embryos by nuclear transfer is inefficient. In pigs this is particularly evident by the lack of reports on the production of live offspring from nuclear transfer reconstructed embryos. The basis of the double nuclear transfer is to minimise the inefficient steps involved in the reconstruction process using a multistep embryo reconstruction procedure and culturing the embryos in the final surrogate recipient. The double nuclear transfer method provides a method of reconstituting an animal embryo. The process comprises transferring a nucleus into a first oocyte followed by removing and transferring said nucleus from said oocyte to a further recipient cell. Preferably, the nucleus transferred from the first oocyte is in the form of a pronucleus-like structure. Preferably, the second recipient cell is an oocyte or an enucleated fertilised zygote. This involves a double nuclear transfer procedure; The first step of this procedure is to produce a pronucleus-like structure by transfer of a donor nucleus to an oocyte. Preferably the oocyte is a mature metaphase II oocyte (unfertilised egg), which has been previously or is simultaneously or subsequently activated, or an activated MII oocyte (the activation is usually by physical or chemical input). An example of the first step is shown in Figure 21 (for simplicity's sake, the use of an activated recipient in Figure 21 is not shown, but this is documented in Table 1 below). In the second step of the procedure the pronucleus-like structure generated by the first step is removed from the first reconstructed embryo and transferred to an oocyte or to an activated parthenote or enucleated fertilised zygote (Figure 21). Where the transfer is to an oocyte, preferably the pronucleus-like structure is removed from the first reconstructed embryo and transferred to an oocyte which is an enucleated MII oocyte prior to, concomitant with or following activation. Following the second nuclear transfer the reconstructed embryo may be transferred directly to a final surrogate recipient for development to term.

**Table 1. Possible cell cycle combinations of donor and recipient cells for first NT in order to maintain ploidy of the nucleus/i of the reconstructed zygotes.**

| DONOR CELL CYCLE STAGE | RECIPIENT CYTOPLAST | OTHER TREATMENT |
|---|---|---|
| G0 | MII CONCOMITANT with ACTIVATION | |
| G0 | MII POST-ACTIVATION | CHEMICAL MAINTENANCE OF PLOIDY (i.e. Nocodazole, Colcemid, DMAP or other serine threonine kinase inhibitors). |
| G0 | MII PRE-ACTIVATION | |
| G1 | MII CONCOMITANT with ACTIVATION | |
| G1 | MII POST-ACTIVATION | CHEMICAL MAINTENANCE OF PLOIDY (i.e. Nocodazole, Colcemid, DMAP, or other serine threonine kinase inhibitors). |
| G1 | MII PRE-ACTIVATION | |
| S | PRE-ACTIVATION | |
| G2 | MII enucleated POST-ACTIVATION | Tetraploid PN Transfer to late G2/ mitotic 1 cell zygote |
| G2 | MII unenucleated POST-ACTIVATION | Subsequent enucleation *Suppression of PB formation if required to form 2 diploid pronuclei |
| G2 | MII PRE-ACTIVATION | |
| S | MII PRE-ACTIVATION | |
| M | MII enucleated POST-ACTIVATION | Depolymerisation of spindle if required i.e nocodazole to form a single tetraploid pronucleus. Transfer to late G2/mitotic I cell zygote |
| M | MII enucleated PRE-ACTIVATION LATE G2 (MPF rising or high) | |
| M | MII enucleated POST-ACTIVATION | Tetraploid PN Transfer to late G2/mitotic 1 cell zygote |
| M | MII unenucleated POST-ACTIVATION | Subsequent enucleation *Suppression of PB formation if required to form 2 diploid pronuclei |

| | | |
|---|---|---|
| *Dependent upon species | | |

The donor genetic material for embryo reconstruction can be provided by any differentiated, partially differentiated or undifferentiated cell taken directly from any stage in an animal development, such as an embryo, foetus, juvenile or adult animal or any cell line derived from such. In previous reports the cell cycle stage of the donor nucleus has been shown to be critical for development. In the present invention a cell at any stage of the cell cycle can be used as donor of genetic material for the first reconstruction process. However, the combination of cell cycle stages of the donor nucleus and the recipient cytoplasm will vary dependent upon the cell cycle stage of the cell to be used as donor of genetic material at the time of transfer. The donor genetic material and the recipient cells, are not required to be from the same animal species, although this may be preferred.

### PREPARATION OF DONOR CELLS.

Methods and materials for the culture, synchronisation and selection of donor cells to be used as nuclear donors for the reconstruction of embryos by nuclear transfer are well known to anyone skilled in the art. Any cell population obtained directly from any stage in the life of an animal (for example, embryo, foetus or adult animal) may be used as a donor genetic material, alternatively a cell from any population may be used (for example an undifferentiated, partially differentiated or differentiated cell population) derived from any stage (for example embryo, foetus, juvenile or adult animal) and maintained in culture or any cell (for example undifferentiated, partially differentiated or differentiated cell) resulting during culture of primary isolates or as a result of treatment of the cultured cell population with hormones, growth factors or chemicals which alter the differentiated state. The cell cycle stage of cells maintained in culture may be manipulated to ensure co-ordination with the recipient cytoplast using any means available to any one skilled in the art. The following examples are non-limiting and are merely representative of various aspects of the art.

### G0-phase

Any method which results in the production of quiescent (non-growing) diploid cells which have arrested the cell cycle following mitosis and cell division but prior to the onset of DNA Replication or S-phase may be used. Suitable methods include but are not restricted to;
I. Quiescence induced by contact inhibition of cultured cell populations
II. Quiescence induced by removal or reduction in concentration of serum or other essential nutrient in the culture medium i.e. leucine or other amino acid, specific growth factor/s.
III. Quiescence induced by senescence
IV. Quiescence induced by addition of a specific growth factor or other substance.
V Quiescence induced by any physical means such as heat shock, hyperbaric pressure or treatment with chemical compounds which induce a heat shock or stress response e.g. puromycin, azacytidine.

### G1-phase

Donor nuclei may be arrested in the G1 phase of the cell cycle by treatment with any chemical, hormone, growth factor or other substance which causes arrest or by any physical stress which induces cell cycle arrest i.e. induction of a heat shock response as a result or elevated temperature or treatment with compounds which induce such a response i.e. puromycin, azacytidine. Alternatively G1 phase cells may be obtained by synchronisation of the cell population by any means and selection of G1 cells at an appropriate time. For example cells may be arrested in the M-phase of the cell cycle by treatment with microtubule disrupting drugs such as Colcemid or Nocodazole, mitotic cells may then be selected from the population by shake-off and placed into a separate culture vessel. G1 cells may then be selected at an appropriate time following shake off. As a variation of this technique, mitotic cells may also be selected from an unsynchronised population. G1 cells may be enriched by causing cells to exit the growth cycle and arrest in a quiescent or G0 phase (as above) and then restimulating growth by re-addition of the restricting factor. G1 cells can then be selected at a time following restimulation to be found by experimentation for each individual cell type/population. This technique may be combined with any technique which then arrests cells in either the G1 phase of the cell cycle, or at the G1/S-phase border i.e. treatment with hydroxyurea or aphidicolin.

### S-phase

S-phase cells can be selected or enriched for by treatment with any chemical agent which interferes with any point of the replication procedure e.g. aphidicolin which inhibits chain elongation in a concentration dependent manner, Hydroxyurea which inhibits the enzyme ribonucleotide reductase. Cells synchronised in any other stage of the cell cycle may be released and the timing of S-phase after release determined experimentally. In addition these two processes may be combined or any other selection or synchronisation procedure or combination thereof utilised.

### G2-phase

G2 phase cells may be selected from a growing population that has been synchronised by any physical, chemical or selective method. Alternatively G2 cells may be enriched for by treatment of a synchronised or unsynchronised cell population with any agents that specifically arrest the cell cycle following the completion of S-phase and prior to the onset of mitosis.

### M-phase

Mitotic cells may be selected from an unsynchronised population using any means possible including but not being limited to, mitotic shake off, elutriation, FAC's (fluorescence activated cell sorting). Alternatively cells may be arrested in mitosis by treatment with microtubule disrupting agents such as colcemid or nocodazole, by treatment with DMAP or any other serine threonine protein kinase inhibitors or by any other chemical or physical means which disrupts normal growth progression and causes arrest in mitosis.

### Unsynchronised cell populations.

Unsynchronised cell populations may be obtained from any embryonic foetal or adult tissue and cultured *in vitro* or any cell removed directly from an embryo, foetus, juvenile or adult animal. An unsynchronised cell is defined as a cell, the cell cycle stage of which is unknown at the time of transfer to a recipient cytoplast.

### NUCLEAR TRANSFER.

### I) Oocytes.

Oocytes to act as suitable cytoplast recipients can be obtained using a variety of techniques and protocols, these include but are not limited to
i) *In vitro* maturation following follicle aspiration from ovaries obtained at slaughter or removed surgically.
ii) Transvaginal follicle puncture followed by maturation *in vitro*
iii) *In vivo* matured but collected by transvaginal follicle puncture prior to ovulation, maturation is then completed *in vitro.*
iv) *In vivo* matured and surgically recovered.
v) *In vivo* matured and recovered at slaughter.

It is preferable, but not essential, that all *in vivo* matured oocytes are harvested by flushing from the oviduct in calcium magnesium free phosphate buffered saline (PBS) (or other suitable medium devoid of calcium and magnesium ions) containing 1.0% foetal calf serum (FCS). Similarly *in vitro* matured oocytes are harvested and transferred to suitable medium devoid of calcium and magnesium ions (for example M2 medium prepared without addition of calcium and magnesium ions). The chosen medium may be supplemented with FCS, BSA or other protein source. Oocytes are denuded of cumulus cells and enucleated as previously described (Campbell *et al.,* 1993,1994) with the exception that calcium free medium, although not essential is preferred for all procedures. Fusion procedures are modifications of those previously reported (Campbell *et al*., 1993,1994) and are as described in the relevant section below, alternatively the nucleus may be introduced by manual injection of the donor cell into the enucleated oocyte (Ritchie and Campbell, J. Reproduction and Fertility Abstract Series No. 15, p60) or by using a commercially available piezzo injection apparatus as demonstrated in the mouse (Wakayama *et al*., 1998). The timing of these events is dependent upon the species. These protocols defme the use of *in vivo* and *in vitro* matured ovine, bovine and porcine oocytes coupled to the use of *in vitro* or *in vivo* produced zygotes. The procedure is not limited to these defined protocols.

### II) Zygotes.

To act as recipients for the second nuclear transfer fertilised zygotes or oocytes (prior to, concomitant with or following activation) are required. At the present time fertilised zygotes are preferred, although not essential. Matured oocytes may be obtained by any of the procedures outlined above. The genetic material may be removed from the oocyte as previously described. Oocytes may be activated by treating the matured oocytes with any physical or chemical stimulus or combination thereof, which causes activation and stimulates development. The timing of application of the activation stimulus will vary somewhat upon both the species and the method of maturation. Fertilised zygotes may be obtained by *in vitro* fertilisation of matured oocytes obtained from any of the above sources or alternatively recovered *ex vivo* following mating or AI using stimulated or non-stimulated donor animals.

### FIRST STAGE NUCLEAR TRANSFER EMBRYO RECONSTRUCTION.

Donor cells at different stages of the cell cycle may be used for embryo reconstruction. The cytoplast recipient used and any additional treatments required will vary somewhat, dependent upon the cell cycle stage of the donor and recipient cells. Table A gives a series of possible combinations.

In fact, any method may be used which results in the production of one or more pronucleus-like structure, preferably of normal diploidy or tetraploidy.

### SECOND STAGE NUCLEAR TRANSFER:

In the second stage, a pronucleus-like structure (preferably identified as a swollen form of the nucleus) from the first nuclear transfer reconstructed embryo is transferred to an enucleated MII (prior to, concomitant with or following activation) or to an activated parthenote or fertilised zygote which have been enucleated by removal of the pronuclei. Although each of these cytoplast recipients are suitable for the second nuclear transfer in practice an enucleated fertilised zygote is the preferred choice. The zygote to act as cytoplast recipient for the second nuclear transfer may be produced *in vivo* or *in vitro,* although in practice the use of *in vivo* produced zygotes is the method of choice at present. Alternatively any cell resulting from development of the first nuclear transfer embryo or cultured cell population derived from this embryo may be used as a donor of genetic material for production of the second nuclear transfer embryo.

### Source of activated oocyte or zygote:

Oocytes obtained from any *in vitro* or *in vivo* source and subsequently matured *in vitro,* or oocytes matured *in vivo* may be used as recipients for the second nuclear transfer following enucleation and subsequent activation or activation and subsequent enucleation. In practice it is preferred that the recipient cytoplast for the second nuclear transfer is prepared from a fertilised zygote. The zygote required for production of a cytoplast recipient for the second nuclear transfer may be obtained from any suitable source which may include but is not limited to *in vivo* produced fertilised zygotes that are recovered from a suitable donor animal. Zygotes may be produced by natural mating, by natural mating following superovulation or by AI using fresh or frozen semen. Alternatively, fertilised zygotes may be produced *in vitro. In vitro* fertilisation may be carried out on oocytes matured *in vivo* from suitable donors with or without superovulation or by using oocytes matured *in vitro* following recovery from slaughterhouse material or aspiration of follicles *in vivo.* In practice it is preferred that the zygote for the second nuclear transfer is obtained following maturation and fertilisation *in vivo.*

### Enucleation of zygotes.

Following successful fertilisation the male and female chromatin in the newly formed zygote decondenses and forms two pronuclei. The genetic material may be removed at any point following fertilisation. However, in practice the present method of choice is to remove the genetic material after formation of the pronuclei. Due to the nature of the cytoplasm it is often difficult to visualise and remove the pronuclei from a fertilised zygote. Visualisation of the pronuclei may be aided by centrifugation of the zygotes prior to their manipulation, although this is not essential.

### Preparation of a karyoplast donor from first nuclear transfer embryo.

The pronucleus/i formed in the first nuclear transfer reconstructed embryos must be removed and transferred to the second cytoplast recipient. To visualise the pronucleus/i the embryos are preferably centrifuged. Aspiration of a pronucleus surrounded by cytoplasm and enveloped in a cell membrane is then carried out, preferably by microsurgery. To facilitate this process, although not a necessity, the embryo may be rendered more *pliable* by treatment with one or more chemical compounds, which disrupt the cytoskeleton e.g. Cytochalasin B, or Nocodazole. The centrifuged embryo is preincubated in the agent/s of choice and then transferred to a manipulation chamber. Using a fine glass pipette a karyoplast is aspirated which contains a pronucleus.

### Reconstruction of second nuclear transfer embryo.

The karyoplast formed from the first nuclear transfer embryo is used to transfer the genetic material now contained within the pronucleus like structure to the enucleated second recipient cytoplast. Any means of transfer including fusion of the karyoplast to the cytoplast by viral, chemical or electrical means may be used or alternatively the pronucleus may be injected into the recipient cell. Due to the size of the pronucleus it is preferred but not essential that the genetic material is transferred by the process of cell fusion. For cell fusion the karyoplast is transferred below the zona pellucida of the second cytoplast recipient and placed into contact with the enucleated zygote. Any physical, chemical or viral means of inducing cell fusion may then be applied. However, at the present time it is preferable, but not essential, to use electrical fusion. For electrical fusion the couplet is placed into a suitable medium in a suitable fusion chamber. A DC electrical pulse is then passed through the couplet at right angles to the plane of contact between the cytoplast and karyoplast. An AC electrical pulse may first be used to align and/or increase contact between the cytoplast and karyoplast. The duration strength and timing of the electrical pulse/s all affect the frequency of fusion and can be optimised by experimentation.

### Culture of second nuclear transfer embryos.

Following reconstruction of the second nuclear transfer embryo, further development may be carried out by culturing in suitable medium *in vitro* until a developmental stage suitable for transfer to a final surrogate recipient is reached. Alternatively, reconstructed embryos may be cultured in the ligated oviduct of a suitable, synchronised, intermediate recipient for sufficient time to reach a developmental stage suitable for transfer to a final surrogate recipient. Or as is the method of choice at present in porcine species, the second nuclear transfer reconstructed embryo can be transferred directly to the final surrogate recipient and pregnancy maintained by hormonal treatment (see below).

### Transfer to final surrogate recipient.

Reconstructed embryos may be cultured by any means *in vitro* or *in vivo* to any stage suitable for transfer to a synchronised final recipient for development to term. Alternatively reconstructed embryos may be transferred as soon as possible to a synchronised recipient for development to term. The latter method is presently the method of choice in porcine species. In this situation induction and maintenance of pregnancy in the final recipient can be achieved by either of the following procedures; At the time of transfer *in vivo* produced embryos at the same developmental stage as the nuclear transfer reconstructed embryos may be transferred with the nuclear transfer. Alternatively, the recipient may be treated with a combination of hormones in order to maintain pregnancy (Christenson et al., J. Animal Science 1971; 32:282-286).

The nuclear transfer methods encompass genetic modification as known in the art to provide transgenic animals of all stages. The term "transgenic" refers broadly to any animal stage whose germ line has been the subject of technical intervention by recombinant DNA technology. This includes introduction of genetic material from another species as well as an animal in whose germ line an endogenous gene has been deleted, duplicated, activated or modified.

The genetic material (donor nucleus) can be modified at any stage in the double nuclear transfer methodology. Most preferably, the recombinant intervention is carried out before the first nuclear transfer step. The recombinant intervention may be carried out between the first and second nuclear transfer steps.

For recombinant intervention before the first nuclear transfer step, the genetic material is preferably modified as part of a cell line modification or taken from a previously modified animal. A level of selection can be introduced to identify donor nuclei for the nuclear transfer step. Alternatively, or in addition, recombinant intervention can be carried out after the first nuclear transfer step and before the second. In this scenario, the cell obtained may be genetically modified by the same or a different method.

The present invention includes the use of unenucleated oocytes as cytoplast recipients for embryo reconstruction by nuclear transfer.

The development of embryos reconstructed by nuclear transfer is dependent upon many factors that include the cell cycle stage of both the recipient cytoplasm and the donor nucleus. The ability to synchronise the blastomeres of early murine embryos has allowed detailed studies of cytoplast/karyoplast cell cycle combinations. Reports in the mouse have demonstrated the use of mitotically arrested cells as donors of genetic material (Kwon and Kono, . Proc. Natl. Acad. Sci. U. S. A.93, 13010-13013).

When using somatic cells as nuclear donors it has been shown that cells arrested in the G0 phase (quiescent) of the cell cycle are able to direct development to term of nuclear transfer reconstructed embryos. The chromatin of quiescent cells has been reported to undergo condensation, these cells also show a reduction in transcription and translation, mRNA is actively degraded and changes occur in the polyribosomes Whitfield, J. F., et al (1985) In Control of Animal cell proliferation (Eds Boynton A. L. and Leffert, H. L., p331-365, Academic Press Inc: London.) Quiescent cells reduce their metabolism to that which is required to maintain viability.

More recently, other researchers have shown that transcriptionally inactive genes localise to centromeric heterochromatin in the nuclei of cycling but not quiescent primary B-lymphocytes. Stimulation of quiescent cells to re-enter the growth cycle causes repositioning of inactive loci to the centromeric regions (Brown *et al, Mol Cell Biol.* **3**, 207-217). Thus the chromatin of quiescent cells may be more amenable to structural changes after nuclear transfer which are associated with 'reprogramming' of gene expression.

The ability to utilise different cell cycle combinations of donor and recipient cells is dependent upon species. In the mouse, an intact organised spindle is obtained when G2 or M phase nuclei are transferred into enucleated MII oocytes. On activation a "pseudo-mitotic/meiotic" event occurs; a polar body is extruded and a diploid nucleus formed. Alternatively suppression of polar body expulsion by treatment with Cytochalasin B results in a tetraploid embryo with 2 diploid pronuclei. Each of these pronuclei can be used (successfully) as a nuclear donor to an enucleated zygote. In cattle, sheep and pigs, following transfer of a nucleus from a diploid (G1) blastomere or a somatic cell into an enucleated MII oocyte, no organised spindle is observed and there are no reports of polar body extrusion suggesting that no organised spindle is in fact formed. Similarly in cattle, when a G2 nucleus is transferred into an enucleated MII oocyte no organised spindle is observed, however if the oocyte is not enucleated 2 organised spindles are observed, one the original oocyte MII and the second organising the chromosomes derived from the donor nucleus.

These differences in spindle organisation can be used to advantage. Nuclei in the G2 or M-phases of the cell cycle can be transferred to a maturing oocyte either I) at a point following metaphase of the first meiotic division i.e. between MI and MII or ii) at MII. At MII there would be 2 spindles present 1 from the maternal DNA and the other from the transferred DNA. Activation of the recipient oocyte can be prevented by methods known in the art i.e. removal of calcium from the culture, manipulation or fusion medium. The transferred chromatin which is condensed into chromosomes may then be allowed to 'be conditioned' in the recipient cytoplasm for a period dependent upon the species and the age of the recipient oocyte. The maternal chromatin may be removed following formation of an intact spindle containing the donor chromatin, or alternatively following activation of the reconstructed oocyte. Activation may be achieved by methods common to the art with the exception that spindle integrity must be maintained. On activation the transferred DNA undergoes a pseudo-mitotic/meiotic division and extrudes a polar body thus giving a diploid embryo. Alternatively the extrusion of the polar body could be prevented with compounds common to the art e.g. Cytochalasin B and the resulting zygote would have 2 diploid pronuclei each of which could be used for transfer to an enucleated zygote or an activated enucleated oocyte.

The original oocyte or maternal DNA could be selectively removed following identification by a number of means:
1. By observation of the 1^{st} polar body and aspiration of cytoplasm and the meiotic spindle from directly beneath the polar body prior to activation - this depends on the age of the recipient oocyte as the polar body breaks down on increasing age.
2. Either the maternal or donor chromatin may be selectively stained prior to reconstruction with a DNA specific dye that will then allow selective removal of the stained or unstained chromosomes prior to activation.
3. As in 3 with subsequent removal of the chromosomes or the subsequent pronucleus following activation.
4. By use of transgenic donor cells which contain a specific marker gene driven by a G0 specific promoter sequence.

### Examples of the use of unenucleated oocytes:

The protocols described for the production of oocytes as recipients for 1^{st} nuclear transfer procedures as described above are all suitable for the use of unenucleated oocytes. Transfer of the donor nucleus can be achieved as previously described using electrical, chemical or virally induced fusion or injection by any means including the use of a piezzo. Enucleation following transfer of the donor nucleus can be achieved by any methods known to the art. The timing of the various procedures will vary between species and be dependent upon the source of oocytes and will be determined experimentally.

The donor cells should be placed in a suitable DNA specific fluorochrome to stain the DNA - i.e. 1 microgram of Hoescht 3332 for 10 minutes in culture medium followed by incubation in medium lacking the stain. Transfer of the nucleus can be carried out at any time after the appearance of telophase of the 1^{st} meiotic division which may be observed microscopically by the appearance of a small protuberance on the cell wall of the oocyte which is well known to those skilled in the art. Manipulation and reconstruction of the recipient oocytes is preferred in a medium that lacks calcium in order to prevent activation. The reconstructed oocytes can then be cultured until the maternal DNA is removed. Sufficient time is allowed for formation of the mitotic spindle (this will vary between species and culture conditions and also on the timing of reconstruction). In the case of Telophase 1 recipients sufficient time is allowed for the oocyte maternal chromatin to reach MII. Enucleation of the maternal chromatin may be achieved at MII by brief exposure to UV light for visualisation and identification of the stained chromatin. Alternatively, the oocytes may be activated and then exposed to UV for identification of the donor chromatin at any time prior to and following pronuclear formation. Following removal of the maternal chromatin and activation the presumptive zygotes are cultured by any means known to the art to a stage suitable for transfer to a synchronised final recipient for development to term. The unenucleated oocyte is preferably used in the first nuclear transfer step. It may be used in the second.

A fifth aspect of the invention provides a method for obtaining clonal pluripotent or totipotent cells (including a clonal pluripotent or totipotent cell population), the method comprising culturing a cell from a non-human transgenic embryo or non-human transgenic fetus, according to the second aspect of the invention, or from an adult developed from such an embryo or fetus. Preferred features of aspects one to four also apply to the fifth aspect.

In this aspect of the invention "pluripotent" and "totipotent" refer to the ability of the cells, under the appropriate stimuli, to differentiate into some (pluripotent) or all (totipotent) the tissue types displayed in an adult animal.

A sixth aspect of the invention provides a clonal pluripotent or totipotent cell or cell population obtainable by a method according to the fifth aspect of the invention. Preferred features of aspects one to fifth also apply to the sixth aspect.

For all aspects of the invention, it is clear that more than one gene targeting event may take place. For example, in a single primary cell there may be a gene targeting event to remove or inactivate genetic material and a gene targeting event to introduce a transgene. More than two gene targeting events are also envisaged. The more than one gene targeting event may take place simultaneously, subsequently or sequentially in the same passage or a different passage. Alternatively a second or further gene targeting event may take place in a cell derived from an embryo, fetus or adult which was developed from an original somatic cell according to the present invention.

The process of embryo reconstruction and production of viable offspring by nuclear transfer is a multistep procedure, each of these will now be described in detail. Also described are more details with respect to gene targeting according to the present invention. All details which follow apply to the invention herein described. In addition, the following terms are referred to in this text and their full expressions are set out here:
ES cell - embryonic stem cell;
EG cell - embryonic germ cell;
MII - metaphase II;
PCR polymerase chain reaction;
HR - homologous recombination;
AAV - adeno associated virus;
DAF - decay accelerating factor;
MCP - membrane cofactor protein;
CD59 - membrane inhibitor of reactive lysis;
HPRT - hypoxanthine phosphoribosyltransferase;
gpt - xanthine guanine phosphoribosyltransferase;
LIF - Leukaemia inhibitory factor;
GFP - green fluorescent protein;
IRES -internal ribosomal entry site
AAT- alpha 1 antitrypsin
BLG- beta lactoglobulin
a1,3 GT- α 1,3-galactosyltransferase

### The Recipient Cell or Cytoplast.

Oocytes, fertilised zygotes and two cell embryos have been used as cytoplast recipients for nuclear transfer. In general, oocytes arrested at metaphase of the second meiotic division (also termed unfertilised eggs, or MII oocytes) have become the cytoplast of choice. At this point in oocyte development the genetic material is arranged upon the meiotic spindle and is easily removed using mechanical means. Several reports have demonstrated that during maturation i.e. between the germinal vesicle stage (prophase of the first meiotic division) and arrest at metaphase of the second meiotic division genomic DNA can be removed and the resulting cytoplast used for nuclear transfer (Kato et al., Mol Reprod Dev 36, 276-8, 1993). The use of fertilised zygotes as cytoplast recipients has been reported in mouse (Kwon et al., Proc Natl Acad Sci USA, 93, 13010-3, 1996), cattle (Prather et al., J. Reprod Fertil Suppl 41, 1990), and pigs (Prather et al., Biol. Reprod. 41, 414-8, 1989). In cattle and pigs, development of embryos reconstructed using zygotes as cytoplast recipients is low and on the whole restricted to the exchange of pronuclei suggesting that factors essential for successful development are removed with the pronuclei.

### Preparation of a Cytoplast Recipient by Removal of the Genomic Genetic Material.

This process has in general been termed enucleation. In the majority of recipients utilised, the genomic DNA is not enclosed within a nuclear membrane at the time of removal. The removal of the genetic material according to the present invention and described by the term "enucleation" does not require that the genetic material is present in a nuclear membrane (it may or may not be, or may partially be). Enucleation may be achieved physically by actual removal of the nucleus, pronuclei or metaphase plate (depending on the recipient cell), or functionally, such as by the application of ultra-violet radiation or other enucleating influence. Removal of the genetic material is possible by physical and or chemical means. In the early reports of nuclear transfer, MII oocytes were simply cut in half on the basis that one half would contain the genetic material and the other would not. Modifications to this approach have been made in order to reduce the volume of cytoplasm, which was removed. This may be achieved by aspiration of a small amount of cytoplasm from directly beneath the 1^{st} polar body using glass micropipettes or by using a knife to cut away that part of the oocyte beneath the polar body. To facilitate plasticity of the oocyte it may be pre-treated with cytochalasin B or other such agent that disrupts the cytoskeleton. In contrast to physical enucleation, chemical treatment has been demonstrated to cause complete removal of the genetic material in the mouse. Treatment of maturing oocytes with the topoisomerase inhibitor ectoposide results in the expulsion of all genomic material with the 1^{st} polar body (Elsheikh et al., Jpn J Vet Res 45, 217-20, 1998), however no development to term has been described using cytoplast recipients produced by this method and there are no reports of this procedure in other species. Centrifugation of MII oocytes combined with Cytochalasin B treatment has been reported to cause enucleation in hamster and cattle oocytes (Tatham et al., Hum Reprod 11, 1499-503, 1996). The development of embryos reconstructed from such cytoplasts has been reported in cattle however the frequency of development is low.

When using zygotes the genetic material may be removed by mechanical aspiration of both pronuclei. Dependent upon species, in order to facilitate visualisation of the pronuclei the zygotes may be centrifuged prior to enucleation.

### Introduction of genetic material (embryo reconstruction).

Having prepared a suitable recipient cell or cytoplast the donor genetic material must be introduced. Various techniques have been reported including;
1. Cell fusion induced by chemical, viral or electrical means.
2. Injection of an intact cell by any method
3. Injection of a lysed or damaged cell.
4. Injection of a nucleus.
   Any of these methods may be used in any species or in a combination of species with some modifications of individual protocols.

### Activation of the reconstructed embryo.

In addition to the transfer of donor genetic material from the karyoplast to the cytoplast, the cytoplast must be stimulated to initiate development. When using a fertilised zygote as a cytoplast recipient, development has already been initiated by sperm entry at fertilisation. When using MII oocytes as cytoplast recipients the oocyte must be activated by other stimuli. Various treatments have been reported to induce oocyte activation and promote early embryonic development including but not limited to; application of a DC electric stimulus, treatment with ethanol, ionomycin, inositol tris-phosphate (IP₃), calcium ionophore A23187, treatment with extracts of sperm or any other treatment which induces calcium entry into the oocyte or release of internal calcium stores and results in initiation of development. In addition any of these treatments in combination, their application at the same or different times or in combination with inhibitors of protein synthesis (i.e. cycloheximide or puromycin) or inhibitors of serine threonine protein kinases (i.e. 6-DMAP) may be applied.

### Culture of reconstructed embryos.

Nuclear transfer reconstructed embryos may be cultured *in vitro* to a stage suitable for transfer to a final recipient using any suitable culture medium or culture process. The reconstituted embryos may otherwise be cultured *in vitro* until they are used in the production of cloned totipotent or pluripotent cells, according to the second aspect of the invention. Alternatively, embryos may be cultured *in vivo* in the ligated oviduct of a suitable host animal (in general, sheep) until a stage suitable for transfer to a final surrogate recipient is reached in order for the animal to be grown to term. Embryos from cattle, sheep and other species may be cultured in a trans species recipient; for simplicity a sheep provides a suitable recipient for bovine, ovine and porcine species. It is usual to embed the embryos in a protective layer of agar or similar material to prevent mechanical damage to the reconstructed embryos or attack by macrophages whilst in the oviduct of the temporary recipient.

### Gene targeting and embryonic stem cells

Gene targeting by homologous recombination between an exogenous DNA construct and cognate chromosomal sequences allows precise modifications to be made at predetermined sites in the genome. Gene targeting is well established in mouse embryonic stem (ES) cells, and has been used to effect modifications in a large number of murine genes (summarised by Brandon et al., Curr. Biol. 5, 625-634, 758-765, 873-881, 1995). This has been facilitated by the ease with which genetic modifications engineered in ES cells *in vitro* can be transferred to whole mice and the consequences of gene targeting studied (reviewed by Papaioannou and Johnson, In: Gene targeting: a practical approach. Ed Joyner, A.L. Oxford University Press, 1992; and by Ramirez-Solis and Bradley Curr Opin. Biotech. 5, 528-533, 1994). Mouse ES cells are pluripotent cells derived from early embryos (Evans et al., Nature 292, 154-156. 1981) which can be grown and manipulated *in vitro* then reintroduced into the preimplantation embryo where they can contribute to all cell types of a chimeric animal, including germ cells (Robertson, E.J., (Ed.) 1987. Teratocarcinomas and embryonic stem cells. A practical approach. IRL Press, Oxford.). The potential benefits of engineering precise genetic modifications by gene targeting in species other than mice, e.g. livestock, have been described many times (e.g. Colman and Garner, Pharmaceutical Forum 5, 4-7, 1996). These include, but are not limited to, the production of human therapeutic proteins in the body fluids, disease prevention, increasing required production traits, cell based therapies, cell based delivery systems for genetic therapy, tissue and organ transplantation.

Great efforts have therefore been made to derive ES lines from a wide variety of species. However, definitive ES cell lines i.e. capable of contributing to the germ line of a chimeric animal, have not been demonstrated from any mammalian species other than mouse. The status of human ES cells (Thomson et al., Science 282, 1145-7, 1998), remains unknown in this respect because of ethical constraints. There are reports of ES or rather ES-like cell lines derived from hamster, mink, sheep (Piedrahita et al., Theriogenology 34, 879-901, 1990), cattle (Stice et al., Biol. Reprod. 54, 100-110, 1996), pig (Gerfen et al., Anim. Biotechnol. 6, 1-14, 1995) and rhesus monkey (Thomson et al., Proc. Natl. Acad. Sci. USA 92, 7844-7848, 1995). In all of these cases, the more limited definition of "cells which under the appropriate *in vitro* conditions, can differentiate along at least three different lineages" has been used to support claims that the cells derived represent ES cells. The production of pig (Wheeler, Reprod. Fertil. Dev. 6, 1-6, 1994) and rat chimeras have also been reported, although in neither case has ES contribution to the germ line been demonstrated.

Embryonic germ (EG) cells are undifferentiated cells functionally equivalent to ES cells, that is they can be cultured and transfected *in vitro* then contribute to somatic and germ cell lineages of a chimera (Stewart, Dev. Biol. 161, 626-628, 1994). EG cells are derived by culture of primordial germ cells, the progenitors of the gametes, with a combination of growth factors: leukaemia inhibitory factor, steel factor and basic fibroblast growth factor (Matsui et al., Cell 70, 841-847,1992; Resnick et al., Nature 359, 550-551, 1992).

Several attempts have been made to isolate EG lines from primordial germ cells in cattle (Cherny, et al., Theriogenology 41, 175, 1994; Stokes, et al., Theriogenology 41, 303, 1994), pig (Shim, et al., Biol. Reprod. 57, 1089-1095, 1997; Piedrahita et al., Biol. Reprod. 58, 1321-1329,1998) and rat (Mitani, et al., Theriogenology 41, 258, 1994). Blastocyst injection of cultured EG cells led to production of mid-gestation chimeric bovine embryos (Stokes, Theriogenology 41, 303, 1994). More recently chimeric male piglets have been produced from both genetically manipulated (Piedrahita, et al., Biol. Reprod. 58, 1321-1329,1998) and normal EG cells (Shim, et al., Biol. Reprod. 57, 1089-1095, 1997). In both instances EG cell contribution to the testes was detected. Unfortunately the ability of this approach to achieve germline transmission could not be established, as one of the animals was stillborn and the other failed to thrive and was sacrificed.

The lack of fully functional large animal ES, or EG cells has however been circumvented by developments in nuclear transfer which allow genetic modifications to be made to somatic cells in culture and then those cells used as nuclear donors to produce a whole animal, as demonstrated by Schnieke *et al*. (*Supra*).

### Gene targeting in somatic cells

There has been considerably less work on gene targeting in somatic cells. The efficiency with which gene targeted clones can be derived is a function of the frequency of homologous recombination (HR) events and the frequency of random integration events. In all mammalian cell types, HR events are significantly less frequent than random events. Distinguishing HR events against a background of random integrants represents a major obstacle to the isolation of gene targeted clones. The results of published experiments indicates that gene targeting in somatic cells is infrequent, estimates of the ratio of HR to random events range from 1:50 or 1:230 in fibrosarcoma (Itzhaki et al., Nat. Genet. 15, 258-265,1997), 1:241 in myeloid leukemia cells (Zhen et al., Proc. Natl. Acad. Sci. USA 90, 9832-9836, 1993), 1:1000 in bladder carcinoma (Smithies et al., Nature 317, 230-234, 1985), to 1:9700 in erythroleukemia /lymphoblast fusion cells (Shesely et al., Proc. Natl. Acad. Sci. USA 88, 4294-4298, 1991). However, these data were obtained from a range of immortalised cell lines and describe the targeting of different gene loci with different gene targeting constructs.

In those few cases where the same construct has been used to target the same locus in both ES cells and somatic cells, the comparative data indicates that the ratio of HR to random integration events may be significantly lower in somatic cells (Arbones et al., Nat. Genet. 6, 90-97, 1994). Recombinogenic cells of the immune system, such as B cells, represent a special exception (Buerstedde et al., Cell 67, 179-188, 1991; Takata, M. et al., EMBO J. 13, 1341-1349, 1994).

Comparison of the frequency of homologous recombination between immortalised and primary somatic cells indicates that homologous recombination is less frequent in primary cells than in immortalised cell lines (Finn et al., Mol. Cell. Biol. 9, 4009-4017, 1989; Thyagarajan et al., Nuc. Acids Res. 24, 4084-4091, 1996). This is important to the production of animals by nuclear transfer because normal, euploid, non-immortalised cells are preferred as nuclear donors because of the risk that immortalised cells may not support development or may lead to tumours in the resultant animal.

An undesirable consequence of a low frequency of homologous recombination is that it is necessary to transfect and screen large populations of the chosen cell type to ensure derivation of gene targeted cell clones. Although ES and EG cells can undergo extensive periods in culture and still be used to derive whole animals, primary cells have a limited lifespan in culture and their competence as nuclear donors may be reduced by genetic alterations which occur in vitro. This precludes the provision of large populations of primary cells by expansion in culture and extensive periods of culture to allow identification of gene targeted cell clones amongst a high background of random transfectants. Therefore there was an expectation that if gene targeting events did occur in primary cells the frequency of such events would be too rare to allow the practical use of gene targeted cell clones to derive whole animals by nuclear transfer.

### Factors affecting the frequency of gene targeting.

Numerous factors have been identified as affecting the frequency of gene targeting. As stated above, there are less data available regarding factors critical to targeting in somatic cells than ES cells.

The frequency of gene targeting in somatic cells has been shown to increase dramatically with the length of the region of homology in the targeting vector (Scheerer, et al., Mol. Cell. Biol. 14, 6663-6673, 1994).

The use of DNA isogenic to the host cell has been demonstrated to improve the frequency of gene targeting in embryonic stem cells (Deng et al., Mol Cell Biol. 12, 3365- 3371, 1992; te Riele et al., Proc. Natl. Acad Sci. USA 89, 5128-5132, 1992). However this has not been investigated in somatic cells.

The effect of transcription of the target gene on the frequency of homologous recombination is the subject of some dispute. Some of the earliest reports of gene targeting demonstrated targeting modifications made to genes which are inactive in the cell type used (Smithies et al., Nature, 317, 230-234, 1985; Johnson, et al., Science 245, 1234- 1236, 1989). However, it has been proposed that homologous recombination at a gene locus is more frequent when that gene is actively transcribed than when it is inactive (Nickolof et al., Mol. Cell. Biol. 10, 4837-4845, 1990; Thyagarajan et al., Nucleic Acids Res. 23, 2784-2790, 1995). This proposal has been disputed by Yanez and Porter, who report no correlation between gene targeting frequency and the transcriptional status of the interferon inducible 6-16 gene in human HT1080 cells (Gene Therapy, 5, 149-159, 1998).

The presence of double strand breaks at the target locus has been shown to stimulate gene targeting in CHO cells (Liang et al., Proc. Natl. Acad Sci. USA 93, 8929-8933, 1996) but not mouse Ltk⁻ cells (Lukacsovich et al., Nuc. Acids Res. 22, 5649-5657, 1994). However double strand breaks have also been shown to stimulate the level of illegitimate recombination in CHO cells (Sargent et al., Mol Cell Biol. 17. 267-277, 1997). The consequent risk of introducing genetic aberrations reduces the usefulness of this approach for cells destined for nuclear transfer. This also applies to other methods of stimulating homologous recombination by induction of DNA damage, e.g. chemical carcinogens, UV radiation, gamma radiation and photoreactive molecules (reviewed by Yanez and Porter, Gene Therapy, 5, 149-159, 1998).

There is a widespread perception by researchers in the field that to achieve gene targeting, vector DNA should be introduced into the host cell by either electroporation, or more rarely by microinjection. A survey of the extensive literature describing gene targeting (summarised by Brandon *et al*., supra) indicates that electroporation is by far the method preferred in the art.

The art also teaches that linearising the gene targeting vector before it is introduced into the host cell dramatically increases the frequency of gene targeting (reviewed by Yanez and Porter, supra). Again a survey of the literature describing gene targeting indicates that gene targeting vectors are used in linear form. Therefore the expectation was that use of linear DNA would be required for gene targeting in somatic cells for all target loci in somatic cells.

Yanez and Porter (supra) also review other factors which affect the rate of homologous recombination. These include the growth conditions of the host cell culture, the stage of the cell cycle of the host cell, modification of the ends of linearised transfected DNA, the inactivation of the MSH2 gene and the inhibition of poly ADP ribose polymerase enzyme activity.

### Methods of enriching or identifying gene targeting events

There are several methods whereby cells carrying possibly rare targeting events can be enriched, or identified from a transfected population.

Transfectants can be divided into pools and the presence of targeted clones within each pool screened, typically by the polymerase chain reaction. Positive pools are then progressively subdivided and rescreened until a single clone has been isolated (Shesely, et al., Proc. Natl. Acad. Sci. USA 88, 4294-4298, 1991). However, as mentioned previously, such schemes are unsuited to primary cells destined for nuclear transfer, because repeated rounds of purification extend the time in culture. Extended culture is undesirable because primary cells may become senescent or acquire genetic aberrations.

The use of gene targeting vectors which maximise the target frequency and allow selection of targeted clones without increasing the time spent in culture is more appropriate for cells destined for nuclear transfer. Gene targeting strategies such as the enhancer trap, promoter trap and polyadenylation trap are described in detail by Hasty and Bradley (In: Gene targeting: a practical approach. Ed Joyner, A.L. Oxford University Press, 1992) and are discussed later in this text.

### Other gene targeting methods

Very high rates of homologous recombination have been reported using chimeric RNA/DNA oligonucleotides for targeting the β-globin gene in lymphoblastoid cells (Cole-Strauss, Science 273, 1386-1389, 1996) and the Factor IX gene both *in vitro* and *in vivo* (Kren, et al., Nature Med. 4, 285-290, 1998). Unfortunately the technology does not seem to transfer easily and other researchers have failed to apply it to their gene of choice (Strauss, Nature Medicine 4, 274-275, 1998).

High targeting frequencies have also been observed with gene targeting vectors based on the adeno-associated virus (AAV). Russell et al. (Nature Genetics 18, 325-330, 1998) report 11 out of 13 transfected primary human fibroblasts had a correctly targeted hypoxanthine phosphoribosyltransferase gene.

Both of these methods are limited in the type of modification that can be engineered, because only a few nucleotide changes can be made at the target locus. While this allows gene inactivation e.g. by the insertion of stop codons, or subtle modification by the substitution of individual amino acids, these methods do not allow the insertion or replacement of larger regions which are necessary for gene replacement or transgene placement.

In this text, there is described the generation, identification and isolation of somatic cells carrying predetermined genetic modifications at a defined locus, "gene targeting" while maintaining the ability of those cells to support production of, following nuclear transfer, either i) a fetus or ii) a viable animal or iii) pluripotent cell populations.

While the method is applicable to all mammalian species, the preferred species are sheep, cattle (cow and bull), goat, pig, horse, camel, rabbit, rodent and human. This invention does not relate to human reproductive cloning. It does cover human tissue cells and, where applicable, human embryos, in particular, embryos under 14 days old.

Preferably, gene targeting is carried out at, or adjacent to, a gene locus which is actively transcribed, or otherwise capable of supporting gene targeting at high frequency. Also preferred is the use of an efficient lipid mediated transfection system e.g. "GenePorter" (Gene Therapy Systems). The use of a gene targeting vector DNA in supercoiled or circular form is also preferred. Long regions of homology to the target locus may be included in the vector. Combinations of these preferred methods of gene targeting are within the scope of the invention. For example, in a particularly preferred embodiment, the use of a lipid mediated transfection system in combination with a gene targeting vector in circular form (relaxed and/or supercoiled) can achieve efficient gene targeting at gene loci which are poorly transcribed or inactive in the host cell.

Many somatic cell types are capable of supporting nuclear transfer and are thus suitable according to the present invention (e.g. mammary epithelial cells, muscle cells, fetal fibroblasts, adult fibroblasts, oviduct epithelial cells, granulosa cells and cumulus cells). Many other cell types will also support nuclear transfer, including embryonic stem cells and their differentiated derivatives, endothelial cells and sub-endothelial cells. Gene targeting of somatic cells in combination with nuclear transfer allows more flexibility than gene targeting using ES cells alone. ES cells are a single cell type which display a single pattern of gene expression. The wide choice of somatic cells available for nuclear transfer allows a cell type to be chosen for gene targeting in which the gene locus of interest is preferably transcriptionally active, or capable of supporting gene targeting at high frequency. Thus, the frequency of homologous recombination can be maximised.

This strategy can be extended to include the artificial induction of gene expression or induction of chromatin changes in a somatic cell type to either enable or increase the frequency of gene targeting. Induction of chromatin changes may involve the use of agents which inhibit histone deacetylation (e.g. sodium butyrate or trichostatin A) to induce an open chromatin configuration and/or gene expression at the target locus before gene targeting.

### Gene expression and histone acetylation

There is strong evidence that gene activity is correlated with the acetylation of core histones (reviewed by Jeppeson, Bioessays, 19, 67-74, 1997; Pazin and Kadonaga, Cell 89, 325-328, 1997) and that histone deacetylation regulates gene transcriptional activity (Wolffe, Science 272, 371-372, 1996). Detailed mechanisms of repression involving proteins with histone deacetylase activity have been elucidated for several genes (e.g. Brehm et al., Nature 391, 597-601, 1998; Magnagi-Jaulin et al., Nature 391, 601-604, 1998; lavarone et al., Mol Cell Biol 19, 916-922, 1999). The inhibition of histone deacetylase activity by chemical reagents such as sodium butyrate, or trichostatin A has been shown to reactivate silent genes (Chen et al., Proc. Natl. Acad. Sci. USA 94, 5798-5803, 1997) and inhibit transcriptional repression (Brehm et al., Nature 391, 597-601, 1998; Magnagi-Jaulin et al., Nature 391, 601-604, 1998). The effect of inhibitors of histone deacetylase on the frequency of gene targeting has not been described in the art.

Alternatively, particular genes or classes of genes can be activated using particular transcriptional activators. Such transcriptional activators may be termed "factors". For example, Weintraub et al (Proc. Nat. Acad. Sci. USA, 86, 5434-5438, 1989) show that primary human and rat fibroblasts, when transfected with the transcriptional activator, MyoD, turn on a variety of muscle genes, and in the case of the rat cells, can form myotubes. Likewise, Tontonoz et al (Cell, 79, 1147-1156, 1994) have shown that the regulators PPARg and C/EBPa synergise to powerfully promote adipogenesis in fibroblasts. In the context of the protocols described in this text, targeting to genes which are normally active in muscle or fat cells can be effected by the introduction of the type of factors described above. However, it is important that the method used to introduce a transcription factor does not compromise the ability of the cells to support nuclear transfer.

Expression of transcription factors can be achieved by transient transfection of an expression construct prior to gene targeting. Because only a small proportion of cells transiently transfected actually integrate the exogenous DNA into their genome, a transcription activator designed to effect activation of a gene at the target locus can be expressed prior to gene targeting and will in the majority of cells leave no integrated DNA. The presence or absence of integrated transcription activator construct DNA can be determined in individual clones and those which lack integrated copies used for nuclear transfer. Where necessary, integrated copies of transcription activator expression constructs can be removed if they are flanked by recognition sites for site specific recombinase enzymes e.g. the *lox*P sites for Cre or the FRT sites for FLP recombinase (Kilby et al., Trends in Genetics, 9, 413-421, 1993).

The choice of target locus may be made on the basis of the ability of that locus to support gene targeting at high frequency, or on the basis of the function of the gene. Gene targeting at the target locus may be carried out so that the expression of the target gene is reduced or ablated, increased, or left unaffected. Gene targeting does not necessarily have to affect the function of the target gene, indeed in some circumstances it may be preferable that expression and function of the target locus is left unaffected.

Gene targeting of primary somatic cells is carried out to achieve gene inactivation, gene upregulation, gene modification, gene replacement, or transgene placement at the target locus. Examples of preferred target loci and modifications include, but are not limited to:
1. inactivation, removal or modification
   - of genes responsible for the presence of antigens which are xenoreactive to humans (e.g. α-1,3 galactosyltransferase),
   - of the PrP locus responsible for the production of the prion protein and its normal counterpart in non human animals,
   - of genes which in humans are responsible for genetic disease and which in modified, inactivated or deleted form could provide a model of that disease in animals, e.g. the cystic fibrosis transmembrane conductance regulator gene.
   - of regulatory regions or genes to alter the expression of one or more genes, e.g. RFX transactivator genes which are responsible for regulation of major histocompatibility class II molecules
   - of endogenous viral sequences
   - of genes responsible for substances which provoke food intolerance or allergy.
   - of genes responsible for the presence of particular carbohydrate residues on glycoproteins, e.g. the cytidine monophospho-N- acetyl neuraminic acid hydroxylase gene in non-human animals
   - of genes responsible for the somatic rearrangement of immunoglobulin genes, e.g. RAG1, RAG2
2. upregulation
   - of genes responsible for suppression of complement mediated lysis (e.g. porcine CD59, DAF, MCP)
   - of gene expression by the introduction of a response element to allow experimental modulation of gene expression
3. gene replacement
   - replacement of genes responsible for production of blood constituents (e.g. serum albumin) with their human counterpart.
   - replacement of genes responsible for substances which provoke food intolerance or allergy with a more benign (e.g. human) counterpart
   - replacement of immunoglobulin genes with their human counterpart.
   - replacement of genes responsible for surface antigens with their human counterpart.
4. transgene placement at a predetermined locus:
   - placement of a transgene at a gene locus which may offer advantageous transgene expression
   - placement of a transgene at a site which places it under the control of an endogenous regulatory region.

The gene targeting vector and the experimental procedure is designed so that the time taken to identify, isolate, analyse and expand primary cell clones carrying targeted events is minimised. This is an important aspect of the invention because reduction of the time in culture increases the likelihood that cells used as nuclear donors are viable, normal and euploid. Recognised risks associated with *in vitro* culture of primary cells which are detrimental to the outcome of nuclear transfer include: senescence due to limited lifespan, acquisition of genetic damage, loss of a normal complement of chromosomes and rapid erosion of chromosomal telomeres.

Precautions which can optionally be taken to minimise the time in culture are:
1. Cryopreservation of cell samples destined for use as nuclear donors at the earliest possible stage.
2. The use of a gene targeting vector which allows direct selection or identification of homologous recombinants relative to random integrants.
3. The use of culture conditions designed to reduce the metabolic insults suffered by the primary cells. For example, by the use of a reduced oxygen atmosphere, or the inclusion of antioxidants to minimise the extent of oxidative damage. However, the invention is not limited to the use of such conditions.

The use of promoter trap, or polyadenylation trap strategies (reviewed by Hasty and Bradley In: Gene targeting: a practical approach. Ed Joyner, A.L. IRL press Oxford, 1992) are preferred embodiments of the invention. In each case, the gene targeting vector is designed such that homologous recombination between the gene targeting vector and the target locus renders a marker gene active, while in the majority of random integrants it is inactive. Marker genes may include genes which confer resistance to drugs (e.g. neomycin, G418, hygromycin, zeocin, blasticidin, histidinol) or other selectable markers (e.g. HPRT, gpt), visible markers (e.g. GFP) or other selection systems (e.g. single chain antibody /hapten systems; Griffiths et al., Nature 312, 271-275, 1984). Conversely, strategies may be designed such that homologous recombination results in removal of a negatively selectable marker gene. For example a vector could be designed such that in correctly targeted cells a site specific recombinase gene e.g. the Cre or FLP recombinase (Kilby et al., Trends in Genetics, 9, 413-421, 1993) is activated e.g. by a promoter or polyadenylation trap, and the recombinase results in the deletion of a toxin gene flanked by appropriate recognition sites (e.g. *loxP* or FRT sites) The gene targeting vector is designed to maximise the frequency of homologous targeting relative to random integration events. This is achieved by incorporating large regions of DNA homologous to the target locus into the gene targeting vector. While it is desirable to maximise the size of these homologous regions, in practice these are limited by the constraints of construct production and the requirement for a reliable, simple genetic screen (e.g. by PCR amplification) to identify targeted events. Homologous DNA in the gene targeting vector may, or may not be isogenic to the host cell, in that it may or may not be derived from the host cell, or from other cells of the same individual.

Gene targeting can be carried out such that a transgene is cointegrated with a selectable marker gene at the target locus. This could be used to provide a suitable site for achieving high expression of the transgene. A preferred embodiment of the invention is the placement of a transgene designed to express a foreign protein in the milk of a transgenic animal at a locus known, or predicted to support abundant expression in the mammary gland. This can be achieved by, for example, placing a structural gene adjacent to the endogenous promoter of a milk protein gene. Another example is the placement of a gene adjacent to the endogenous promoter of a collagen gene. Where the presence of the marker gene is undesirable in the final animal, it may be removed by the action of specific recombination systems e.g. the Cre/*lox*P system or the FLP/FRT system (Kilby *et al*., *Supra*) if it is flanked by recombination recognition sites. Removal of the marker gene may either be achieved in the cells before nuclear transfer, or in cells derived from animals (including fetuses) produced by nuclear transfer, or in oocytes, zygotes, or embryos which carry the genetic modification and are from a lineage which started with an animal made by nuclear transfer or during subsequent mating of animals derived by nuclear transfer. A method whereby Cre recombinase can be specifically activated and used to effect recombination during the production of male gametes has been described by O'Gorman et al., (Proc. Natl. Acad. Sci. USA. 94, 14602-14607, 1997).

Multiple rounds of gene targeting may be carried out, either in cells before nuclear transfer or in cells derived from an animal produced by nuclear transfer. Retargeting of a locus may be facilitated by the inclusion of a marker gene in the first round of targeting such that its loss on subsequent targeting allows ready identification or selection of clones carrying subsequent targeted events.

As stated earlier, one part of the present invention provides for the generation of animals which contain targeted modifications to their genomes. Not all regions of the genome are suitable sites for modification because of their proximity to gene silencing elements such as those seen in heterochromatin and because modification to certain sites can have disruptive effects on endogenous gene expression. These potential undesirable consequences can be examined in any animals resulting from the nuclear transfer process, particularly those homozygous at the targeted locus (loci). This can be achieved by backcrossing or by using nuclear donors where the same targeting event has been achieved at both alleles. It has been claimed that a significant proportion of a preferred embodiment of the invention is the validation of target loci as preferred sites for genetic modification because of the absence of collateral genetic damage and their ability to confer good levels of transgene expression as assessed in nuclear transfer animals. Such a validation would be extremely valuable for those contemplating *ex-vivo* gene therapy.

### Ex-vivo gene therapy.

*Ex vivo* gene therapy is a means of delivering therapeutic gene products to a patient in which cells derived by biopsy, are manipulated *in vitro* and then returned to the same patient. Gene therapy by transplantation of *ex vivo* genetically engineered cells has been used for both local and systemic delivery of proteins to experimental animals. For example, Hortelano et al., (Human Gene Therapy 10, 1281-8, 1999) and Regulier et al., (Gene Therapy 5, 1014-1022, 1998) describe the transplantation into mice of myoblasts expressing human clotting Factor IX and erythropoietin respectively. Cao et al (J. Mol Med 76, 782-789, 1998) and Muller-Ladner et al. (Arthritis. Rheum. 42, 490-497, 1999) describe the transplantation into mice of fibroblasts expressing cytokines and cytokine inhibitors respectively.

To date, the choice of cell used for ex-vivo gene therapy is dictated by its availability from the patient , the ease with which genetic manipulation can be carried out, etc. If adult stem cells were available more readily, these would provide suitable targets for gene manipulation, either because of their abilities to form a self sustaining population or because they can be induced to differentiate in vitro into cell type difficult to access from the patient e.g. neurons (see Smith, Current Biology R802-804, 1998). More recently, there have been proposals that a similar objective could be obtained using therapeutic cloning (Trounson and Pera Reprod Fertil Dev 10 121-125 1998). Therapeutic cloning is an as yet untried application of nuclear transfer which promises to provide cells, tissues, and organs for a human patient requiring replacement or supplementation of diseased or damaged tissue. In outline, somatic cells are obtained by biopsy, their nuclei transferred into an oocyte, human blastocysts derived and used to produce embryonic stem (ES) cells, or other pluripotent cells, which are then induced to differentiate into the cell type required for transplantation. If nuclear donor cells are obtained from the patient requiring the transplant this would provide an autologous graft. Preferably, gene targeting would be performed on the somatic donor population prior to therapeutic cloning since all cells from the disaggregated embryo would have the desired alteration thereby minimising time needed in culture to expand cell populations prior to transplantation.

### Genetic modification of cells destined for human transplantation.

Although, in some instances, healthy, unmanipulated cells would suffice, the function and efficacy of tissue produced by either therapeutic cloning, or ex vivo gene therapy would require, or be enhanced by genetic modification. Genetic modification could be used to effect changes to endogenous genes in situ e.g., to repair or remove a genetic defect, or to add a transgene. Transgenes may be added to achieve a variety of therapeutic goals, the precise nature of which would depend on the condition to be treated and the cells to be transplanted. For example, a transgene could be designed to:
- aid the longevity of the transplanted cells
- aid the interaction of the transplanted cells with the host tissue
- aid terminal differentiation of transplanted precursor cells
- induce regeneration of host tissue, e.g. by expression of a growth factor
- express a factor which is deficient, absent or sub-functional in the patient
- facilitate controlled ablation of transplanted cells if required
- to act as a source of agent toxic to cells close to the site of transplantation, e.g. for cancer treatment.

While effecting changes in endogenous genes can only be achieved by gene targeting, transgene addition can be achieved by either random integration, or gene targeting. However, gene targeting offers several important advantages over random integration as a means of adding a transgene to cells destined for human transplantation.

Transgenes integrated at random in a host genome are subject to the well documented "position effect". This can lead to suboptimal transgene expression and may subject the transgene to influences capable of disturbing the tissue or temporal pattern of gene expression and which are necessarily unpredictable. In contrast, gene targeting allows the precise placement of a transgene at a predetermined locus which can be chosen to provide desirable expression characteristics.

Integration of a transgene into some sites in the host genome may physically disrupt structural gene sequences, or lead to aberrant up or down-regulation of endogenous gene expression. Random integration therefore carries a risk of causing a deleterious phenotype in the host cell, including tumorigenesis. Gene targeting, however, allows the placement of a transgene at a predetermined site which can be chosen on the basis of its safety in this respect.

Although the frequency of gene targeting in somatic cells is generally regarded as very low compared to the frequency of random integration, we demonstrate in this patent specification that an appropriate combination of target locus, host cell type and targeting vector can lead to a very high frequency of targeting.

Desirable criteria for a target locus for transgene placement can therefore be summarised as below:
1. The locus should support transgene expression at abundant levels should it be required.
2. The locus should not unduly influence the tissue specific regulation of the transgene.
3. Transgene integration at the target locus should have minimal or no effect on cell phenotype.
4. The target locus should support gene targeting at high frequency, preferably in cells which are readily obtained by biopsy and which can be cultured, transfected and drug selected. A high frequency of gene targeting is desirable as it simplifies the experimental procedures and would minimise the amount of tissue required from the patient by biopsy and would minimise the time needed for in vitro culture.

Our findings, as fully described in the examples, indicates that the COLIA1 locus represents an example of a good target locus in these respects.

A major application of *ex vivo* gene therapy is the use of genetically modified transplanted cells as a means of providing a protein lacking in the patient, for example blood clotting factors for haemophiliac patients. A primary objective of genetic manipulation of such cells is to achieve a suitable level of expression of the therapeutic product in the transplanted cells. This can be accomplished by the placement of a transgene as a discrete transcription unit at a target locus under the control of a promoter optimally active in the cell type in which transgene expression is desired. However, an alternative approach is to place the transgene under the control of an endogenous gene promoter, a promoter which directs abundant expression in the cell type transplanted would be particularly suitable for expression of a protein required in large amounts or where the number of cells transplanted into the patient is restricted. For example, the endogenous promoter may direct expression in fibroblast cells or in endothelial cells. The transgene can be placed under the control of the endogenous promoter without disturbing the expression of the endogenous gene by inserting the transgene sequence within the 3' untranslated region of the mRNA such that it forms part of a bicistronic message. Translation of the transgene coding region can be achieved by insertion of an internal ribosomal entry site. The successful placement of a marker transgene as part of the COLIA1 mRNA is described in the examples.

### Nuclear transfer as a test of a cells normality after gene targeting

An important aspect of this invention is the demonstration that normal viable animals can be derived by nuclear transfer from cell clones which have undergone transgene placement by gene targeting at the COLIA1 locus.

Production of an animal by nuclear transfer provides a very stringent test of the normality of the donor cell. Thus, nuclear transfer in animals such as sheep provides a rigorous means of testing gene targeting procedures before they are carried out in human cells destined for transplantation therapy. We have identified that gene targeting and nuclear transfer in animals such as sheep offers a means of identifying target loci suitable for human gene targeting. The value of nuclear transfer in a particular animal species as a test of the safety of an equivalent human locus would be greatest in regions where a high degree of synteny exists between the human and animal genome.

Means of identifying human target loci suitable for transgene placement and those target loci are encompassed within the scope of this invention. Because the same loci could be used to place different transgenes in a range of therapeutic applications, a set of candidate target loci identified by animal nuclear transfer animal can be characterised further, for example in human cell culture before they are used for human therapy.

This information can be applied to the choice of targeting site for gene therapy. Whatever the choice of cell type used, it will be imperative that the population used all have the same, pre-selected modification. This will involve cell cloning from correctly modified primary differentiated or stem cell types. Where therapeutic cloning is used all the cells will by origin, have the same genotype.

All preferred features of each aspect of the invention apply to all other aspects *mutatis mutandis.*

The following drawings are referred to in the present text:

### Figure Legends

### Figure 1

Structure of the COLT-1 gene targeting vector and the targeted ovine COLIA1 locus

The upper portion shows a diagram of the COLT-1 vector with the 5' and 3' regions homologous to the ovine COLIA1 locus, the IRES neo region and the bacterial plasmid indicated.

A double crossover event at the ovine COLIA1 locus (middle) results in the structure of the targeted locus as indicated in the lower portion of the figure. Although it is not possible to determine the precise sites where recombination between the COLT-1 vector and the target locus recombination occur, regions at the target locus homologous to the COLT-1 vector are shown in darker shading.

### Figure 2

PCR screening strategy used to identify gene targeting events at the ovine COLIA1 locus.

The upper portion shows the approximate position of the two primers used to amplify a 3.4 fragment from a region unique to the COLT-1 vector to a region unique to the COLIA1 locus.

Sequence A shows a portion of DNA sequence spanning the junction between the region unique to the COLIA1 locus and the 5' region of shared homology with the COLT-1 vector. The position and orientation of the COLTPCR4 primer is shown.

Sequence B shows a portion of DNA sequence spanning the junction between the IRES neo gene and the 5' region of shared homology. The position and orientation of the COLTPCR8 primer is shown.

### Figure 3

Agarose gel electrophoresis of PCR fragments amplified from COLT-1 transfected G418 resistant PDFF-2 cell clones. Lanes containing samples from cell clones 1, 2, 6, 12, 13, 14, and 26 are indicated. The position of the diagnostic 3.4kb amplified fragment is indicated by the arrow.

### Figure 4

Sequence analysis of the 5' junction of the targeted ovine COLIA1 locus in three independently derived targeted cell clones.

The upper portion shows the sequence of the 5' end of the linearised COLT-1 gene targeting vector. Terminal sequence derived from a cloning vector is indicated.

The middle portion shows a portion of the sequence of the diagnostic 3.4 kb fragment amplified from each of the targeted cell clones 6, 13 and 14 spanning the junction between the region unique to the COLIA1 locus and the 5' region of shared homology with the COLT-1 vector.

The lower portion shows the sequence of the PDFF2 COLIA1 locus over the same region.

### Figure 5.

Construction of COLT-2 transgene placement vector

The upper portion of the diagram shows the structure of the BLG driven AAT transgene inserted into the COLT-1 vector. The middle shows the COLT-1 vector and the point at which the AATC2 transgene was inserted. The lower portion of the diagram shows the structure of the COLT-2 vector. Functional regions of each construct are distinguished by patterns and shading and are as indicated.

### Figure 6

Southern analysis of COLIA1 gene targeted lambs.

Lanes indicated in the key contain genomic DNA from lambs derived by transfer of nuclei from cell clones targeted by COLT-1 (PDCOL6) and COLT-2 (PDCAAT90), control DNA samples from a normal lamb, cell clone PDCAAT90 and non transfected PDFF2 cells. The positions of the 7kb fragment derived from the non-targeted COLIA1 locus and the 4.73 kb fragment derived from the targeted COLIA1 locus are indicated by arrows.

### Figure 7

The structure of the ovine COLIA1 locus and the COLT-1 and 2 targeting vectors and COLT-1 and 2 targeted loci are shown.

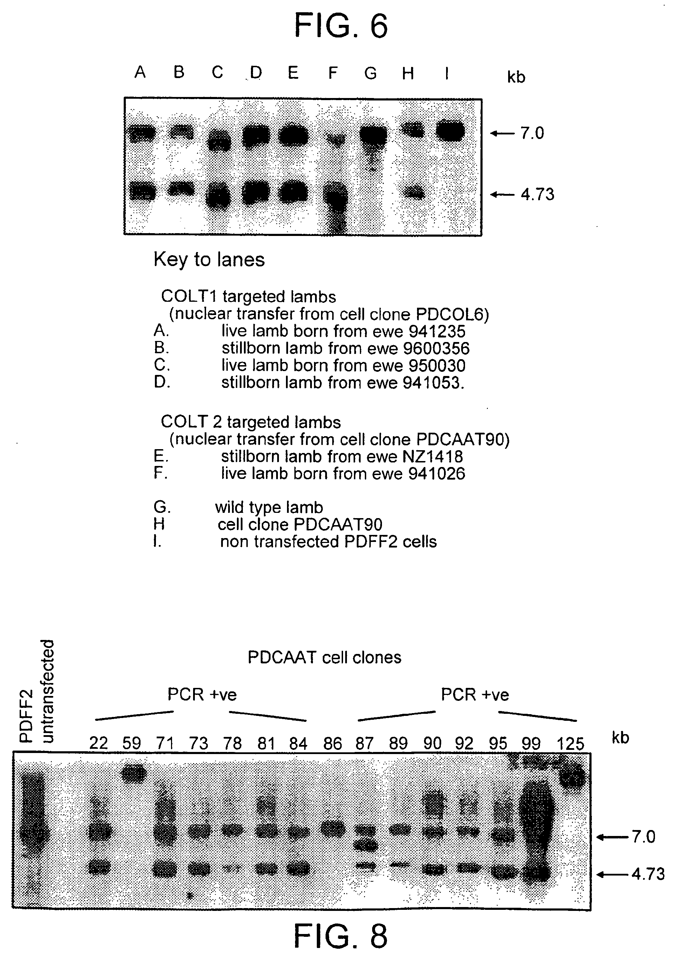

The diagram of the COLIA1 locus shows the position of the translational stop and polyadenylation sites and the direction of COLIA1 transcription as an arrow. The asterisk shows the position of the SspI restriction site where the targeted gene insertions were made. The position of the COLIA1 probe used for Southern analysis in Figures 6 and 8 is shown.

The IRES neo cassette is indicated by a shaded box, the bacterial vector pSL1180 is indicated by an open box and the BLG driven AAT transgene by a striped box. The diagrams of the targeted loci show the direction and predicted extent of the COLIAI / IRES neo bicistronic message and also the AAT message as arrows.

The scale of the diagram is indicated by the 2kb scale bar. Restriction enzyme sites shown are: K, KpnI; A, AspI; Sc, SacII; S, SalI; B, BamHI.

### Figure 8

Southern analysis of COLT-2 transfected (PDCAAT) cell clones.

The identity of each cell clone is shown above each lane. Those which provided a positive signal by PCR are indicated. Also included are non transfected PDFF-2 cells. The positions of the 7kb fragment derived from the non targeted COLIA1 locus and the 4.73 kb fragment derived from the targeted COLIA1 locus are indicated by arrows.

### Figure 9

Northern analysis of total RNA derived from PDCAAT cell clones 81 and 90 and non transfected PDFF-2 cells.

The source of each RNA sample is indicated over each lane. The left upper autoradiograph shows hybridisation to a neomycin phosphotransferase (neo) probe, the right upper autoradiograph shows hybridisation to a human COLIA1 probe. The lower two panels show hybridisation to a mouse β-actin probe. The position of the 288 and 18S ribosomal RNA bands are indicated.

### Figure 10

Northern analysis of primary ovine mammary epithelial (POME) cells in culture.

POME cells were grown for a total of 4 and 9 days in culture on either fibronectin (FN) or type I collagen coated dishes. Cells were either grown continuously in Proliferation Medium (uninduced) or grown for the final three days before harvest in Induction Medium (induced). Total RNA was extracted and subjected to Northern analysis using standard procedures. The autoradiograph shows the result of hybridisation with an ovine beta lactoglobulin (BLG) probe. The source of each RNA is indicated above each lane. Total RNA prepared from the mammary gland from which the cells were derived is also included. The size and position of the ovine BLG mRNA is indicated by the arrow.

### Figure 11

The structure of the ovine beta lactoglobulin (BLG) locus and the BLAT-3 targeting vector are shown.

The diagram of the BLG locus shows the position of the BLG translational start (ATG), stop and polyadenylation sites and the direction of transcription from the BLG promoter. Exons are indicated by black boxes. Exon 3 at which the targeted insertion is designed to take place is marked. The position of the primers used to PCR amplify a fragment from the targeted locus is indicated. The 5' and 3' regions of the BLAT-3 vector homologous to the ovine BLG locus are marked. The IRES neo cassette is indicated by a shaded box and the bacterial vector pBS (Bluescript) indicated by an open box. The scale of the diagram is indicated by the 2kb scale bar.

### Figure 12

Agarose gel electrophoresis of PCR fragments amplified from BLAT-3 transfected G418 resistant POME cell clones. Lanes containing samples from BLAT-3 transfected POME cell clones 1-16 are indicated. Also included is a fragment amplified from a plasmid constructed to mimic the 5' structure of the BLAT-3 targeted BLG locus, marked as "positive control". Non transfected POME cells are also indicated. The position of the 2.375kb diagnostic fragment is shown by the arrow.

### Figure 13

Schematic map of the porcine α 1,3 GT promoter-trap knockout vector pPL501 and 502. Porcine α 1,3 GT genomic structure was based on the report by Katayama *et al* (Katayama, A et al, Glycoconjugate J , 15, 583-589, 1998). The gene is 24 kb in size, and contains 6 exons and 5 introns; The start codon is located in exon 4.

### Figure 14

Outline of the PCR screening strategy for the detection of homologous recombination events at the porcine α1,3 GT locus. Small arrows indicate the positions of the two PCR primers. The subcloned 2.4 kb PCR fragment was sequenced from both ends.

### Figure 15

Sequence analysis over the 3' junction region of the PCR fragment amplified from porcine α1,3 GT targeted cell clones.

The upper portion shows the sequence of the portion of the circular gene targeting vectors pPL501 and 502 at the 3' end of the 3' homologous arm or each. The junction between the 3' region of homology to the porcine α1,3GT gene and bacterial plasmid sequence is indicated.

The lower portion shows the sequence of the diagnostic 2.65 kb fragment amplified from four of the targeted cell clones spanning the junction between the region unique to the 1,3-GT locus and the 3' region of shared homology with the α1,3-GT gene targeting vector. The sequence derived from each clone was identical.

### Figure 16

Schematic map of bovine BLG poly A-trap knockout vector. 5' homologous recombination arm is a 10kb fragment of BLG promoter. 3' homologous recombination arm is a 1.9kb fragment containing BLG exon 2 to intron 3.

### Figure 17

PCR screening and DNA sequencing of BLG knockout clones. The 5' primer, Neo442s, is located at 3' end of neo gene. The 3' primer, BLG3'1, is approximately 260bp 3' of the 3' end of the 3' homologous arm. The PCR product from the targeted locus is 2.3kb.

### Figure 18

Sequence analysis over the 3' junction region of the PCR fragment amplified from three bovine BLG targeted cell clones.

The upper portion shows the sequence of the portion of the circular gene targeting vector pPL522 at the 3' end of the 3' homologous arm of each. The junction between the 3' region of homology to the bovine BLG gene and bacterial plasmid sequence is indicated.

### The lower portion shows the sequence of the diagnostic 2.3kb fragment amplified from three of the targeted cell clones spanning the junction between the region unique to the BLG locus and the 3' region of shared homology with the BLG gene targeting vector. The sequence derived from each clone was identical.

### Figure 19

Western blot of sample milks to identify presence of transgenic AAT.

### Figure 20

Graph of change in transgenic AAT expression over the course of sample milkings.

### Figure 21

Overview of double nuclear transfer methodology.

We have demonstrated gene targeting at three gene loci in three species. In each case the frequency of gene targeting achieved was significantly in excess of the rate that could be expected in primary cells based on published data.

The sheep COLIA1 locus has been targeted by placement of a selectable marker gene alone and in combination with a transgene. The COLIA1 gene expresses the alpha-1(I) procollagen gene at high level in the fibroblast cells used for targeting. The frequency of gene targeting obtained with the marker gene was slightly greater than 1 targeted event per 10 drug resistant cell clones. This is significantly in excess of the rate of gene targeting that could be expected in primary cells based on published data. Furthermore, the cells carrying the targeted modification retained their ability to support nuclear transfer and the production of viable lambs has been demonstrated. This therefore facilitates the practical use of somatic cells coupled with nuclear transfer as a means of transferring precise predetermined genetic modifications wrought *in vitro* to whole animals.

The sheep beta lactoglobulin gene has been targeted at high efficiency in primary cells chosen because they provided abundant expression at the target locus.

The pig alpha 1,3-galactosyltransferase gene has been targeted in primary fibroblasts which were estimated to express at the target locus at low level. Targeting was achieved using a novel method of transfection suited to gene targeting at gene loci which are transcribed at low level, or are inactive in the host cell type.

The bovine beta lactoglobulin gene has been targeted at high efficiency in primary fibroblasts which do not detectably transcribe the target locus, using the same novel method of transfection.

The present invention is exemplified by the following, non-limiting examples.

### Example 1

Placement of a neo marker gene at the COLIA1 locus in primary ovine fetal fibroblast cells by gene targeting

The Poll Dorset primary fetal fibroblast primary culture PDFF2 has been described previously (Schnieke *et al*., *Supra*). PDFF2 genomic DNA was used to generate a library of cloned DNA fragments from which a molecular clone corresponding to a portion of the ovine COLIA1 gene from exon 44 to approximately 14 kb 3' of the translational stop site was isolated. The ovine COLIA1 molecular clone was used as a source of DNA for construction of the COLT-1 gene targeting vector.

COLT-1 is designed to place the neo selectable marker gene downstream of the COLIA1 gene without disrupting the expression of the COLIA1 gene.

Gene targeting with the COLT-1 vector was designed to maximise the frequency of homologous recombination in PDFF2 fetal fibroblasts:
a. The choice of target locus, COLIA1 gene locus is highly expressed in fibroblasts.
b. The COLT-1 construct was designed as a promoter trap such that the construct alone does not confer G418 resistance. Homologous recombination at the COLIA1 locus introduces a neo gene lacking a promoter but which has at the 5' end an internal ribosomal entry site (IRES) to facilitate translation. Homologous recombination results in insertion of the IRES neo gene into the COLIA1 transcribed region downstream of the COLIA1 translational stop site. Transcription of the targeted COLIA1 locus produces a bicistronic message with translation of the neo coding region occurring by initiation at the internal ribosomal entry site.
c. The length of the regions within COLT-1 homologous to the COLIA1 gene was maximised.

### COLT-1 consists of:

A 3 kb region of the 3' end of the ovine COLIA1 gene, from a position approximately 2.9kb 5' of the translational stop site to an SspI site 131 bases 3' of the stop site.
A 0.6 kb internal ribosomal entry site (IRES) region corresponding to bases 1247 to 1856 of the IRES hygro vector (Clontech, Genbank accession number: U89672)
A 1.7 kbp region containing the bacterial neo gene and a portion of the 3' end of the human growth hormone gene containing the polyadenylation site essentially the same as that described by McWhir et al.,(Nature Genetics 14, 223-226, 1996)
A 8.3 kb region of the 3' end and flanking region of ovine COLIA1 gene, from a SspI site 131 bases 3' of the translational stop site to a XhoI site approximately 8.4kb 3' of the stop site.
The bacterial cloning vector pSL1180 (Pharmacia)

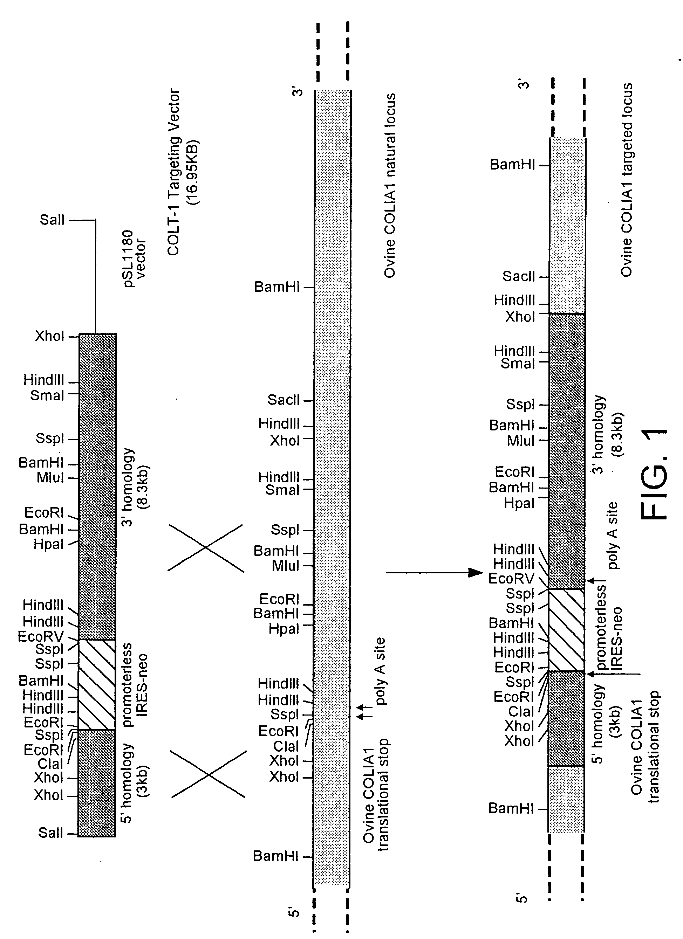

The structure of the COLT-1 Vector and the targeted COLIA 1 locus are shown in Figure 1.

COLT-1 DNA linearised with the restriction enzyme SalI was transfected into early passage PDFF2 cells and G418 resistant cell clones were isolated as summarised below:
Day 0: 5x10⁵ cells PDFF2 cells at passage 3 were transfected with 6µg linearised COLT-1 DNA using lipofectAMINE following the procedure recommended by the manufacturers (Gibco, BRL Life Technologies).
Day 2: Transfected cells were split into two sets of eight (10cm) dishes and G418 added to the medium at either 0.8 mg/ml, or 3.0 mg/ml.
Day 11-18: Colonies were isolated and replated into 6 well dishes. Samples were taken for PCR analysis.
Day 15- 19 Colonies were expanded into 25cm² flasks. Further samples were taken for PCR analysis
Day 20-25 Expanded clones were cryopreserved.

Throughout, PDFF-2 cells were grown in BHK 21 (Glasgow MEM) medium supplemented with L-glutamine (2mM), sodium pyruvate (1mM) and 10% fetal calf serum in standard tissue culture flasks and dishes in a humidified tissue culture incubator using an atmosphere composed of 2% O₂, 5% CO₂, 93% N₂. Cells were passaged by standard trypsinisation.

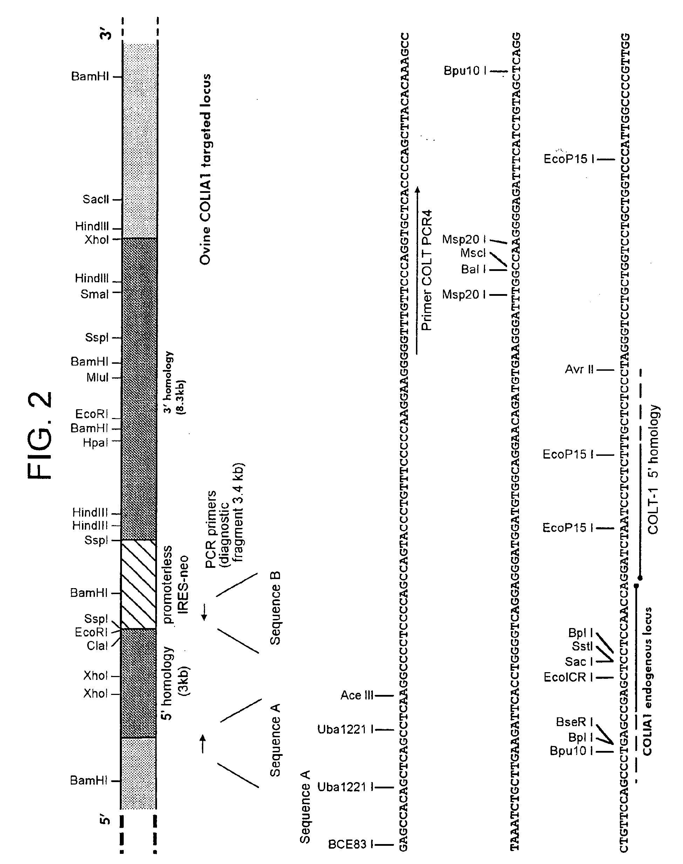

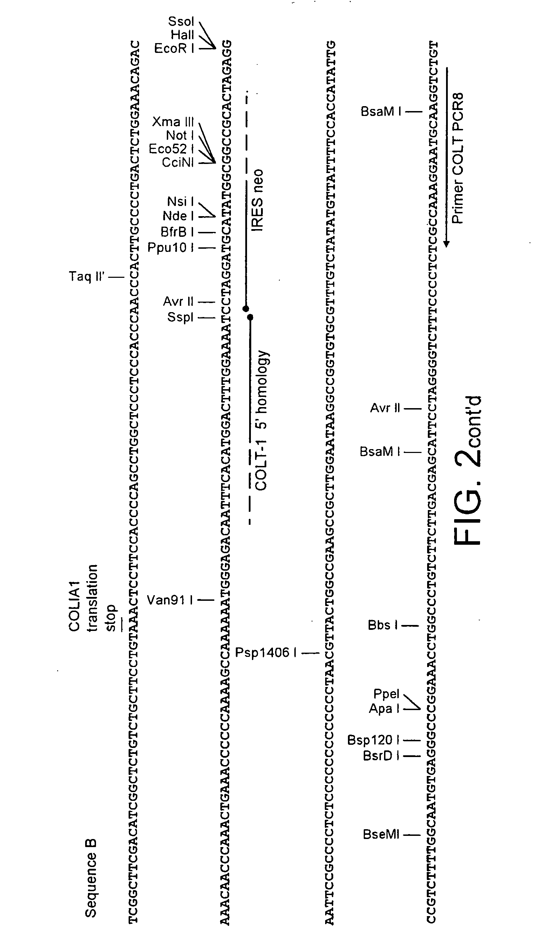

PCR analysis was used to distinguish between random integrated copies of COLT-1 and homologous recombinants at the COLIA1 locus. The PCR scheme used is shown in Figure 2. The upper part of Figure 2 shows the predicted structure of the targeted locus and the positions of the two primers used to amplify a fragment of 3.4kb if the IRES neo cassette is inserted at the correct position in the COLIA1 gene. The DNA sequences A and B, in the lower part of the figure, show the precise positions of each primer relative to the structure of the targeted locus.

Samples of cells to be screened were lysed in PCR lysis buffer (50mM KCI, 1.5 mM MgCl_{2,} 10mM Tris pH8.5, 0.5% NP40, 0.5% Tween) plus proteinase K, and incubated at 65°C for 30min. Proteinase K was inactivated at 95°C for 10 min and polymerase chain reaction carried out using the "Expand long template PCR system" (Boehringer) following the manufacturers recommended conditions. Thermal cycling conditions were as below:

| | | |
|---|---|---|
| | 94°C | 2 min |
| 10 cycles of | | |
| | 94°C | 10 sec |
| | 55°C | 30 sec |
| | 68°C | 2 min |
| 20 cycles of | | |
| | 94°C | 10 sec |
| | 60°C | 30 sec |
| | 68°C | 2 min + 20 sec/cycle |
| | | |
| | 68°C | 7 min |

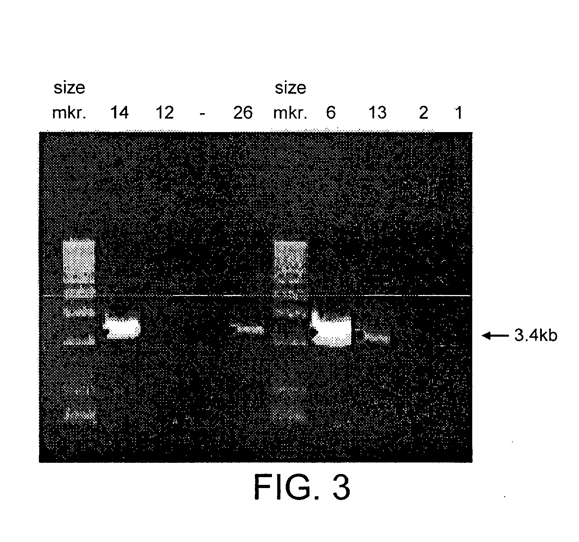

Figure 3 shows a representative agarose gel electropherogram of PCR products amplified from seven G418 resistant cell clones. Four of these (clones 6, 13, 14, 26) show the presence of the diagnostic 3.4 kb fragment indicative of integration of the IRES neo gene into the COLIA1 locus by homologous recombination.

### In total 63 clones were analysed by PCR, 7 of which (11%) were positive.

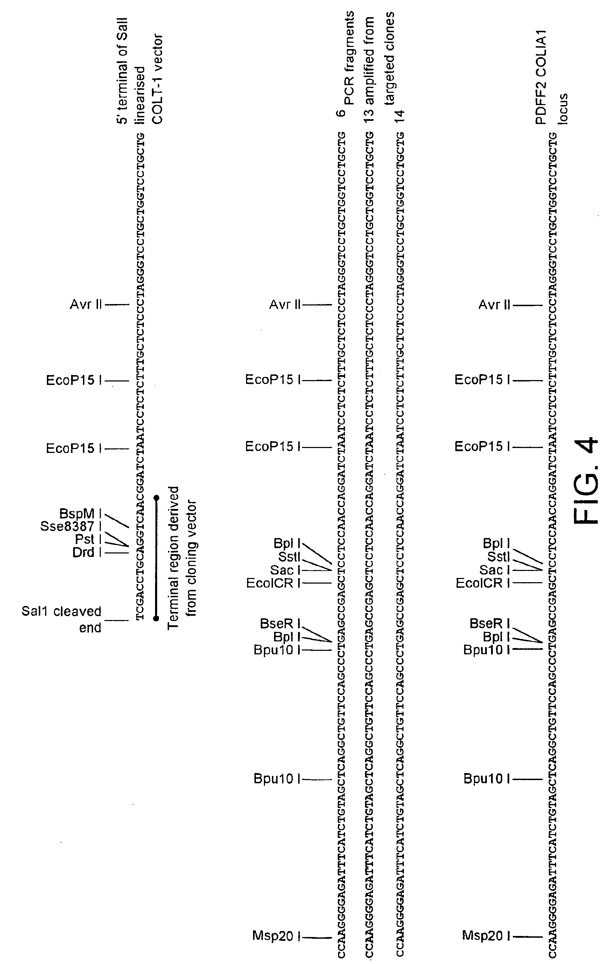

The nucleotide sequence of fragments amplified from three clones (6, 13, 14) was determined and compared with the sequence of the ovine COLIA1 gene. Portions of the sequence of the fragment amplified from each clone is shown in Figure 4. It can be seen that in each case the sequence of the fragment amplified from each clone is identical over the 5' junction of the targeted locus and is consistent with a homologous recombination event occurring between the COLT-1 plasmid and the endogenous COLIA1 gene. That is, the PCR fragment contains sequence from the COLIA1 gene 5' of that present within the COLT-1 construct and does not contain the sequence known to be present at the extreme 5' end of the linearised COLT-1 construct.

### Example 2. Generation of COLT-1 gene targeted sheep

Targeted clones 6 and 13 were selected for nuclear transfer primarily because they grew most readily in culture. The chromosome number of these clones was determined as a basic requirement for competence as nuclear donors. In each case the gross chromosome number indicated that each clone was euploid.

**Table 2 shows the chromosome number of clones 6 and 13**

| Clone | number spreads counted | number spreads 53/54¹ |
|---|---|---|
| 6 | 28 | 17 |
| 13 | 42 | 28 |

| | | |
|---|---|---|
| 1 Number of spreads with a chromosome count of either 53 or 54. | | |

It was calculated that the total period of culture these cells had undergone since disaggregation of the original fetus up to the point of preparing multiple cryopreserved stocks ready for rounds of nuclear transfer was 30 days. This period was made up of four stages: 2 days and 3 days for the initial establishment of the PDFF-2 working stock of primary cells, 20 days during which time gene targeting was carried out, and 5 days for the expansion of the clones and provision of multiple stocks for nuclear transfer. Between these stages cells were stored by cryopreservation.

Cryopreserved stocks of each cell clone were thawed, grown for 1-2 days then prepared for nuclear transfer as below.

### Preparation of cells to act as nuclear donors.

Donor cells were plated at 2 X 10⁴ cells per well of a 6 well tissue culture dish and cultured for 16-24 hours in BHK 21 (Glasgow MEM) medium supplemented with L-glutamine (2mM), sodium pyruvate (1mM) containing 10% fetal calf serum. At 24 hours the medium was aspirated and the cell monolayer was washed three times in medium containing 0.5% fetal calf serum. Cultures were then incubated at 37°C in low serum medium (0.5%) for 2-5 days until they had exited the cell cycle as determined by staining for proliferating cell nuclear antigen (PCNA) in control cultures.

Cells were harvested by trypsinisation and stored in suspension in medium containing 10% fetal calf serum for 2-3 hours prior to use as nuclear donors.

### Superstimulation of donor ewes and recovery of oocytes

Ewes to act as oocyte donors were synchronised with progestagen sponges for 14 days (Veramix, Upjohn, UK) and induced to superovulate with single injections of 3.0 mg/day (total 6.0 mg) ovine follicle-stimulating hormone (FSH) (Ovagen, Immunochemical Products Ltd. New Zealand) on two successive days. Ovulation was induced with an 8 mg single dose of a gonadotropin-releasing hormone analogue (GnRH Receptal, Hoechst. UK.) 24 hours after the second injection of FSH.

Unfertilised metaphase II oocytes were recovered by flushing from the oviduct at 24-29 hours after GnRH injection using Dulbecco's phosphate buffered saline containing 1.0% fetal calf serum (FCS) maintained at 37°C until use.

### Oocyte manipulation.

Recovered oocytes were maintained at 37°C, washed in phosphate buffered saline (PBS) 1.0% FCS and transferred to calcium free M2 medium containing 10% Foetal Calf Serum (FCS), at 37°C. To remove the chromosomes (enucleation) oocytes were placed in calcium free M2 containing 10% FCS, 7.5 µg/ml Cytochalasin B (Sigma) (Cytochalasin B is optional) and 5.0 µg/ml Hoechst 33342 (Sigma) at 37°C for 20 minutes. A small amount of cytoplasm from directly beneath the 1st polar body was then aspirated using a 20 µM glass pipette. Enucleation was confirmed by exposing the aspirated portion of cytoplasm to UV light and checking for the presence of a metaphase plate.

### Embryo reconstruction.

Groups of 10-20 oocytes were enucleated and placed into 20µl drops of calcium free M2 medium at 37°C, 5% C_{O2} under mineral oil (SIGMA). At 32-34 hours post hCG injection, a single cell was placed into contact with the enucleated oocyte. The couplet was transferred to the fusion chamber (see below) in 200µl of 0.3 M mannitol, 0.1mM MgS0₄, 0.001mM CaCl₂ in distilled water. Fusion and activation were induced by application of an AC pulse of 3V for 5 seconds followed by 3 DC pulses of 1.25kV/Cm for 80µsec. Couplets were then washed in TC199 10% FCS (the addition of 7.5 µg/ml Cytochalasin B to this medium is optional) and incubated in this medium for 1 hour at 37°C, 5% CO₂. Couplets were then washed in TC199 10% FCS and cultured in TC199 10% FCS at 37°C, 5% CO₂ overnight. Alternatively, the donor nucleus may be transferred by either manual or piezo-electric aided injection or by any other chemical or physical means of producing cell fusion.

### Embryo culture and assessment.

After the overnight culture period fused couplets were double embedded in 1% and 1.2% agar (DIFCO) (or any other suitable protective covering material) in PBS and transferred to the ligated oviduct of synchronised ewes. After 6 days recipient ewes were sacrificed and the embryos retrieved by flushing from the oviduct using PBS, 10% FCS. Embryos were dissected from the agar chips using 2 needles and development assessed by microscopy. All embryos which had developed to the morula/blastocyst stage were transferred as soon as possible to the uterine horn of synchronised final recipient ewes.

### Nuclear Transfer: births from COLT-1 targeted sheep.

Results of nuclear transfer using two COLT-1 targeted clones, termed PDCOL6, and 13 are summarised in Table 3.

**Table 3. Nuclear transfer using COLT-1 targeted cell clones**

| | PDCOL 6 | PDCOL 13 |
|---|---|---|
| No. reconstructed embryos | 109 | 154 |
| No. embryos recovered from temp. recipient | 104 | 149 |
| No. embryos developed to morula or blastocyst | 14 | 43 |
| No. embryos transferred to final recipients | 14 | 43 |
| No. recipients | 8 | 22 |
| No. fetuses detected at day 60 by ultrasound scan | 5* | 10* |
| No. liveborn lambs | 4 | 8 |
| No. lambs surviving more than 1 month | 2 | 2 |

| | | |
|---|---|---|
| * one twin pregnancy | | |

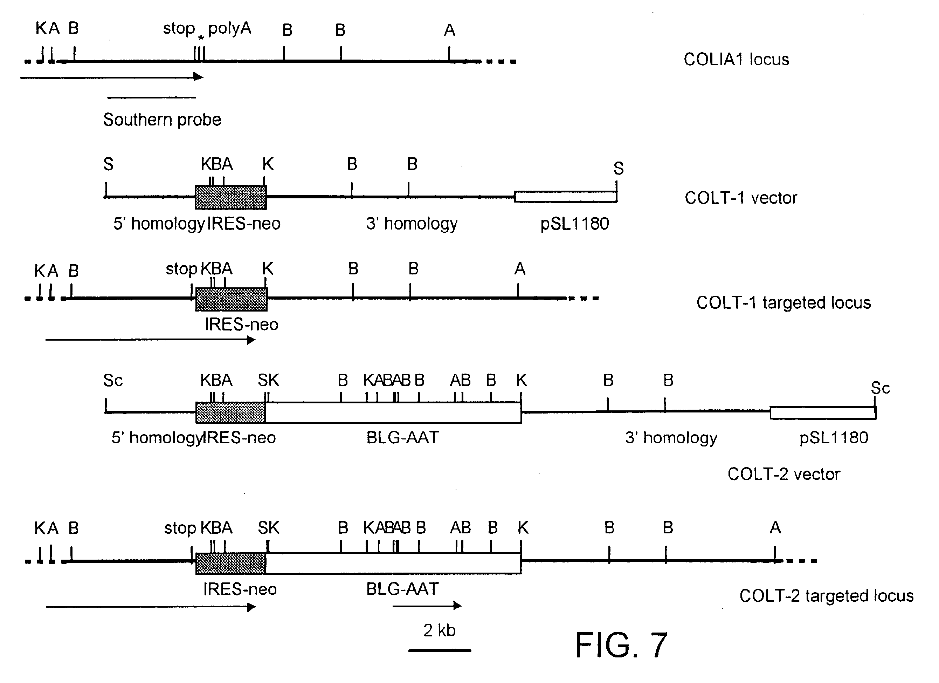

Southern analysis of one still born and one live lamb derived from cell clone PDCOL6 are shown in Figure 6. The hybridisation probe used was a 3kb COLIA1 fragment which constitutes the 5' region of homology present in vectors COLT-1 and 2 which reveals a 4.73 band diagnostic of the targeted allele and a 7kb band from the non-targeted allele. The upper part of Figure 7 shows the structure of the COLT-1 targeted locus showing the position of the hybridisation probe used for Southern analysis and the origin of the fragments seen in Figure 6.

These data indicate that each lamb analysed showed the presence of both wild type and targeted alleles.

### Example 3. Placement of a transgene at the COLIA1 locus in primary ovine fetal fibroblast cells by gene targeting.

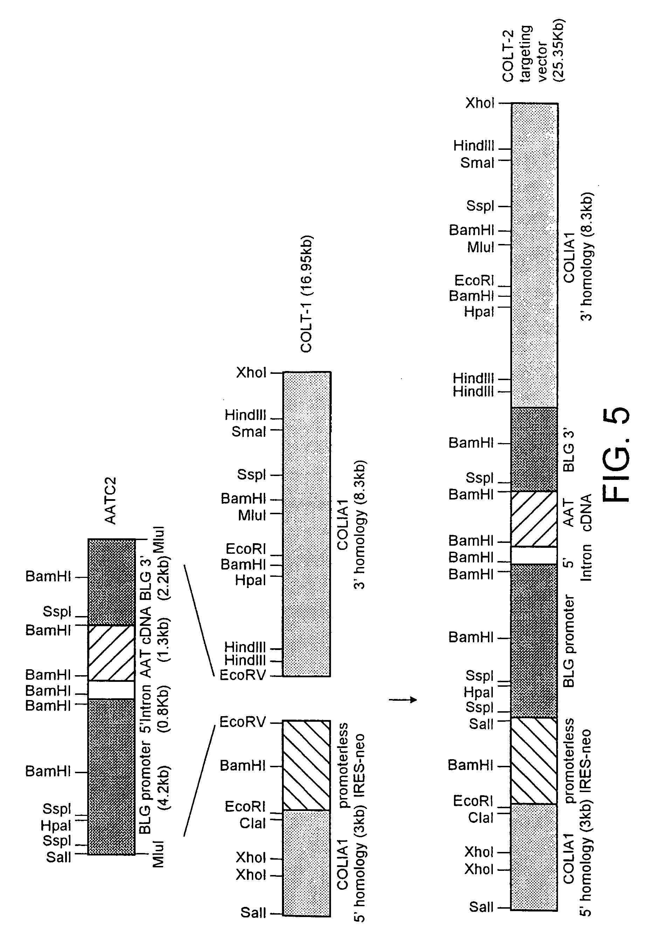

The construct COLT-2 is shown in Figure 5. COLT-2 was generated by insertion of a MluI fragment containing the AATC2 transgene expression cassette into a unique EcoRV site situated at the 3' end of the IRES neo segment of COLT-1.

AATC2 consists of the 5' portion of the vector pACTMAD6 (described in patent application WO 99/ 03981), comprising the ovine β-lactoglobulin promoter and mouse cardiac actin 1st intron, linked to human α-1 antitrypsin cDNA and the 3' portion of the pMAD vector (described in patent application WO 99/ 03981), comprising the ovine β-lactoglobulin exon 7, polyadenylation site and 3 flanking region.

COLT-2 DNA linearised with the restriction enzyme SacII was transfected into early passage PDFF2 cells and G418 resistant cell clones were isolated in the manner described for COLT-1 transfection in Example 1.

The time course of the experiment is shown below:
- Day 0:: 30 µg COLT-2 DNA transfected into PDFF2 cells at passage 2 in 5 X 25cm² flasks
- Day 2:: transfected cells plated onto 80 X 10cm diameter petri dishes and 0.8 mg/ml G418 selection applied
- Day 11-12:: 384 vigorous G418 resistant colonies picked to individual wells of 6-well dishes.
- Day 15:: 104 clones sampled for PCR analysis

- Day 15:: 15 vigorous PCR +ve clones passed to 25cm² flasks
- Day 18:: 15 clones cryopreserved

Seventy of the 104 cell samples were screened by PCR using the same method as COLT-1. This yielded 46 positive signals, indicating a targeting efficiency of 66%. Cell clones derived from COLT-2 targeting were termed PDCAAT.

Southern analysis of a group of PCR positive PDCAAT cell clones and one PCR negative clone is shown in Figure 8. The autoradiograph shows the results of hybridisation with a 3kb COLIA1 fragment which constitutes the 5' region of homology present in vectors COLT-1 and 2. A diagram of the structure of the COLT-2 targeted locus showing the position of the hybridisation probe and the origin of the fragments is shown in Figure 7.

The COLIA1 probe hybridised to a 4.73 band diagnostic of the targeted allele and a 7kb band from the non-targeted COLIA1 allele. It can be seen that PDCAAT cell clones 22, 71, 73, 81, 84, 89, 90, 92 and 95 show the presence of both fragments in equimolar ratio, consistent with the targeting of one COLIA1 allele. Clone 86 shows only the non-targeted allele consistent with the negative PCR result. Clones 87 and 99 show the presence of the targeted and non-targeted alleles and additional bands which could represent either rearrangements at the target locus, or random integration events. It is not known whether these indicate the presence of both random and targeted events within the same cell clone, or that these cultures are oligoclonal. Clone 78 shows the presence of the targeted fragment at a lower intensity than the non-targeted fragment again perhaps indicating that this is not a pure clone. DNA from clones 59 and 125 was uncut.

### Northern analysis of COLT-2 targeted cell clones

Cell clones PDCAAT 81 and PDCAAT 90 were grown to confluence in 75cm² flasks, total RNA extracted and Northern analysis carried out by standard procedures.

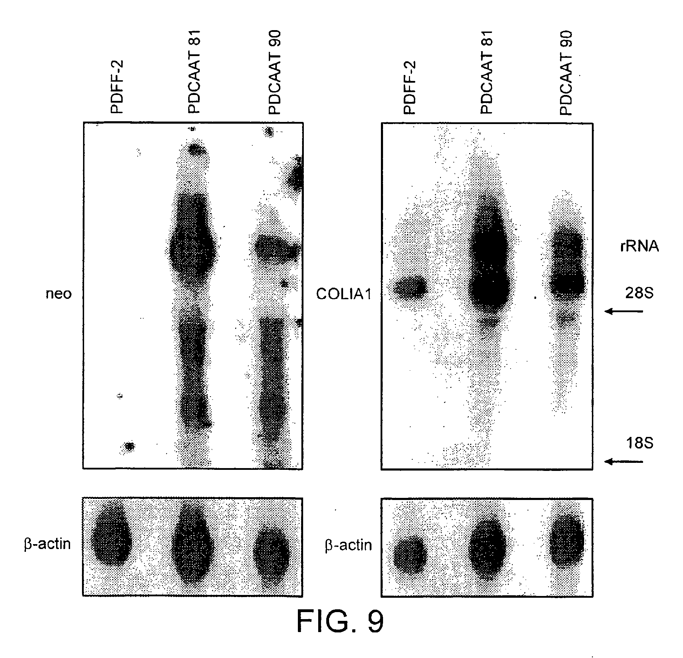

Duplicate samples of 10 µg of each RNA were subjected to gel electrophoresis using a formaldehyde agarose gel and MOPS running buffer. After transfer, the membrane was cut in half to provide two filters for Northern analysis. One half was hybridised with a 4.1 kb human COLIA1 cDNA followed by hybridisation with mouse β-actin cDNA. The other half filter was hybridised with a neomycin phosphotransferase probe and then with mouse β-actin cDNA. Results are shown in Figure 9.

Transcription of the COLT-2 COLIA1 targeted locus was predicted to produce a bicistronic message, with the IRES-neo portion fused to the COLIA1 transcript after the stop codon. The results shown in Figure 9 are consistent with this. The right upper autoradiograph (hybridisation to collagen cDNA) shows a single mRNA species of approximately 4.8 kb, as estimated by the position of the 28s rRNA, corresponding to the endogenous COLIA1 mRNA. Interestingly, although both mice and human show two endogenous COLIA1 mRNA species from different poly A sites, sheep show a single species. A larger species is present in the PDCAAT clones 81 and 90, consistent with the size (∼6.8 kb) predicted for the COLIA1 IRES-neo fusion mRNA. The left upper autoradiograph shows hybridisation to the neo probe. The single mRNA species detected in PDCAAT 81 and 90 is the size predicted for the bicistronic message. No mRNA species were detected with the neo probe in non-targeted PDFF-2 cells. Hybridisation with mouse β-actin cDNA was used as an indication of the amount of RNA loaded in both filters and is shown in the lower two panels.

Northern analysis was also carried out to investigate whether insertion of the AATC2 transgene at the COLIA1 locus led to expression of BLG directed AAT expression in fibroblasts. Hybridisation with an human α1-antitrypsin probe was used to detect expression of the transgene. No AAT mRNA was detectable in PDFF-2, PDCAAT81 or PDCAAT90 cells. It can therefore be concluded that insertion of the transgene at the COLIA1 locus does not induce significant leaky expression of BLG-driven AAT in fibroblasts.

### Example 4. Generation of a sheep carrying a transgene placed at the COLIA1 locus.

Karyotype analysis of three PDCAAT cell clones derived in Example 3 was carried out as a basic requirement for competence as nuclear donors. In each case the gross chromosome number indicated that each clone was euploid. Table 4 shows the chromosome number of cell clones PDCAAT81, 90 and 95.

**Table 4. Chromosome numbers in PDCAAT cell clones**

| Clone | number spreads counted | number spreads 53/54¹ |
|---|---|---|
| 81 | 31 | 26 |
| 90 | 35 | 34 |
| 95 | 24 | 18 |

| | | |
|---|---|---|
| 1 Number of spreads with a chromosome count of either 53 or 54. | | |

PDCAAT cell clones 81 and 90 were used for nuclear transfer using the method described in Example 2. Results of nuclear transfer are summarised in Table 5.

**Table 5. Nuclear transfer using COLT-2 targeted cell clones**

| | PDCAAT 81 | PDCAAT 90 |
|---|---|---|
| No. reconstructed embryos | 71 | 83 |
| No. embryos recovered from temp. recipient | 62 | 78 |
| No. embryos developed to morula or blastocyst | 4 | 19 |
| No. embryos transferred to final recipients | 4 | 19 |
| No. recipients | 2 | 10 |
| No. fetuses detected at day 60 by ultrasound scan | 2 | 3* |
| No. liveborn lambs | 0 | 2 |
| No. lambs surviving more than 1 month | 0 | 1 |

| | | |
|---|---|---|
| * one twin pregnancy | | |

Genomic DNA was prepared from tissue biopsies from lambs obtained by nuclear transfer. Southern analysis of one still born and one live lamb derived from cell clone PDCAAT90 are shown in Figure 6. The diagnostic fragments observed (see Figure 7) confirm the presence of the wild type and targeted allele in each case.

### Example 5. Targeting of a gene locus in cells chosen to provide abundant expression at the target locus.

The ovine beta lactoglobulin (BLG) gene is specifically expressed in the ovine mammary gland in late pregnancy and during lactation. Gene targeting at this locus was achieved by choosing an appropriate host cell type. These were primary mammary epithelial cells which express BLG at high level in culture.

### Preparation of primary ovine mammary epithelial (POME) cells

All procedures were carried out under sterile conditions
1. Mammary tissue was excised from a 115 day pregnant ewe and cut into 5cm pieces removing fat and major blood vessels.
2. The pieces of mammary tissue were washed 3 times with fresh Dissection Medium in a laminar flow cabinet hood and cut into 1cm pieces.
3. The tissue pieces were injected with Dissociation Medium until the tissue became distended.
4. Tissue was minced into a paste and transferred to a conical flask and 4ml Dissociation Medium were added per g of tissue.
5. Tissue was incubated in a rotary shaker (∼200 rpm) at 37 deg. C for 1-5hr until the tissue passed easily through a wide bore Pasteur pipette.
6. A series of differential centrifugation steps were used to enrich the epithelial component of the mammary tissue.
a. Cell suspension centrifuged at 100 rpm, 30 sec
b. Floating fat aspirated and discarded
c. Pellet redigested for a further 30-60 min and step a repeated.
d. Supernatant centrifuged at 800 rpm, 3 min
e. Floating fat aspirated and discarded
f. Pellet resuspended in Proliferation Medium
g. Supernatant centrifuged at 1500 rpm, 10 min
h. Supernatant discarded
i. Pellet resuspended in Proliferation Medium

7. Pellets from steps 6f and i were combined and cells were seeded at 2.5 x 10⁵ cells /cm² onto either collagen (Sigma C8919) or fibronectin (Sigma 4759) coated tissue culture flasks in Proliferation Medium containing 50µg/ml gentamycin (Sigma G1397).
8. Induction of milk gene expression in POME cells was carried out by growth of confluent monolayers for 3 days in Induction Medium

| Media used in POME cell preparation and culture Dissection Medium | |
|---|---|
| M199 (Gibco/BRL 22340-020) | 500ml |
| 100x antibiotic/antimycotic (Gibco/BRL 15240-039) | 10ml |
| 50mg/ml gentamycin (Sigma G1397) | 0.5ml |

| Dissociation Medium | |
|---|---|
| M199 (Gibco/BRL 22340-020) | 500ml |
| NaHCO₃ (Sigma S5761) | 0.6g |
| Trypsin (Gibco/BRL170702-018) | 0.75g |
| Collagenase A (Boehringer 1088-793) | 1.5g |
| Fetal calf serum | 25ml |

| Proliferation Medium | |
|---|---|
| (1:1) DMEM/F12 (Gibco/BRL 4196-039, 21765-029) | 500ml |
| Fetal calf serum | 50ml |
| 5mg/ml bovine insulin (Gibco/BRL 13007-018) | 0.5ml |
| 10 g/ml epidermal growth factor (Sigma E4127) | 0.5ml |
| 5mg/ml linoleic acid (Sigma L8384) | 0.5ml |

| Induction Medium | |
|---|---|
| (1:1) DMEM/F12 (Gibco/BRL 4196-039, 21765-029) | 500ml |
| Fetal calf serum | 50ml |
| 5mg/ml bovine insulin (Gibco/BRL 13007-018) | 0.5ml |
| 5mg/ml linoleic acid (Sigma L8384) | 0.5ml |
| 5mg/ml ovine prolactin (Sigma L6520) | 0.5ml |
| 1mM dexamethasone (Sigma D4902) | 0.5ml |

### BLG expression and induction in cultured POME cells

Expression of BLG was analysed in POME cells grown for a total of 4 and 9 days in culture. Cultures of POME cells were grown on either fibronectin (FN) or type I collagen coated dishes. Cells were either grown continuously in Proliferation Medium or subjected to induction of milk gene expression by growth for three days in Induction Medium. Total RNA was extracted and subjected to Northern analysis using standard procedures.

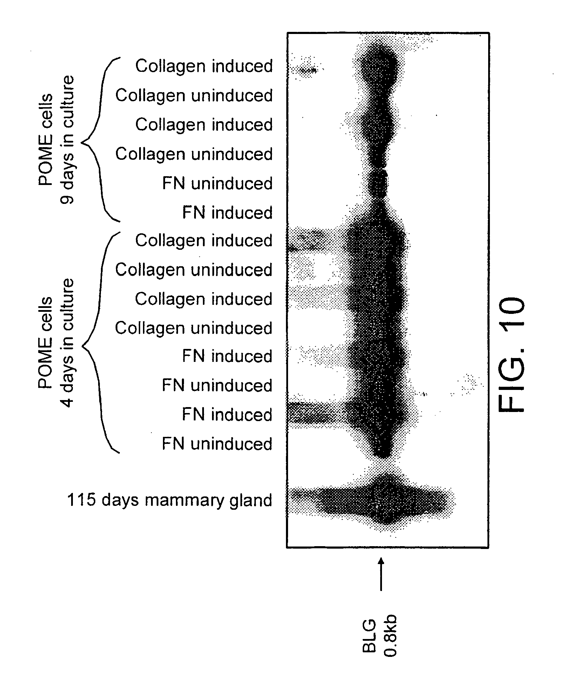

Figure 10 shows Northern analysis of POME cell cultures. The filter shown was hybridised with an ovine BLG probe. It can be seen that all cultures express abundant quantities of BLG mRNA at day 4 with a slight reduction after 9 days in culture. Analysis of POME cells at later stages showed further reductions in BLG expression. This highlighted the need to carry out gene targeting in POME cells within a short period after derivation. POME cells which had been cryopreserved directly after derivation behaved in a similar manner and could be used conveniently.

The mRNA detected in this Northern analysis represents transcriptional activity of the BLG gene in culture and not mRNA residual from the mammary gland because induction significantly increased the amount of BLG message present in each case.

### Generation of a BLG promoter trap vector BLAT-3

BLAT-3 is a promoter trap gene targeting vector designed to disrupt expression of the ovine BLG gene by insertion of an IRES neo cassette into exon 3.

### BLAT3 consists of:

A 1.84kb region of the 5' end of the ovine BLG gene from a SacI site 25 bases 5' of the translational start to a ClaI site within exon 3.
A 0.6 kb internal ribosomal entry site (IRES) region corresponding to bases 1247 to 1856 of the IRES hygro vector (Clontech, Genbank accession number: U89672)
A 1.7 bp region containing the neo gene (encoding neomycin phosphotransferase) and a portion of the 3' end of the human growth hormone gene containing the polyadenylation site, essentially the same as that described by McWhir et al., (Nature Genetics 14, 223-226, 1996)
An approximately 12 kb region of the ovine beta-lactoglobulin gene from a ClaI site within exon 3 to a point approximately 9kb 3' of the polyadenylation site corresponding to the end of a genomic clone in phage lambda.

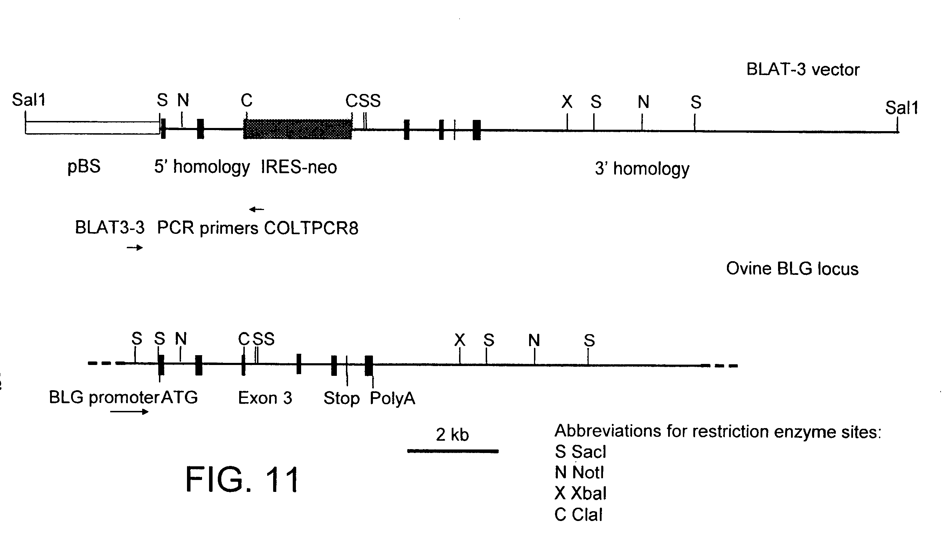

The structure of the BLAT-3 vector and the position of PCR primers used to detect targeting events are shown in Figure 11.

### Targeting of BLG in POME cells using vector BLAT-3

BLAT-3 DNA linearised with SalI was transfected into early passage POME cells and G418 resistant clones derived as described below:
- Day 0: 6µg DNA transfected using lipofectAMINE into 1 X 25cm² flask (precoated with collagen) of POME cells
- Day 1: Transfected cells plated onto 6 X 10cm diameter petri dishes and 0.8 mg/ml G418 selection applied
- Day 14-17: G418 resistant colonies picked to collagen coated 6-well dishes
- Day 21-25: Samples taken for PCR analysis, clones cryopreserved

The PCR scheme used to identify POME cells which had undergone a gene targeting event was based on the use of one primer which hybridised to a region within the IRES region of BLAT3 (primer COLTPCR8) and one which hybridised to a region of the BLG promoter not contained within the vector (primer BLAT3-3). The approximate position of these primers is shown in Figure 11 and the sequence of each primer was as below:
BLAT3-3: TAAGAGGCTGACCCCGGAAGTGTT
COLTPCR8: GACCTTGCATTCCTTTGGCGAGAG

Samples of cells to be screened were lysed in PCR lysis buffer (50mM KCI, 1.5 mM MgCl₂, 10mM Tris pH8.5, 0.5% NP40, 0.5% Tween) plus Proteinase K, and incubated at 65°C for 30min. Proteinase K was inactivated at 95°C for 10 min and polymerase chain reaction carried out using the "Expand long template PCR system" (Boehringer) following the manufacturers recommended conditions. The thermal cycling conditions were as below:

| | | |
|---|---|---|
| | 94°C | 2 min |
| 10 cycles of | | |
| | 94°C | 10 sec |
| | 60°C | 30 sec |
| | 68°C | 2 min |
| 20 cycles of | | |
| | 94°C | 10 sec |
| | 60°C | 30 sec |
| | 68°C | 2 min + 20 sec/cycle |
| | | |
| | 68°C | 5 min |

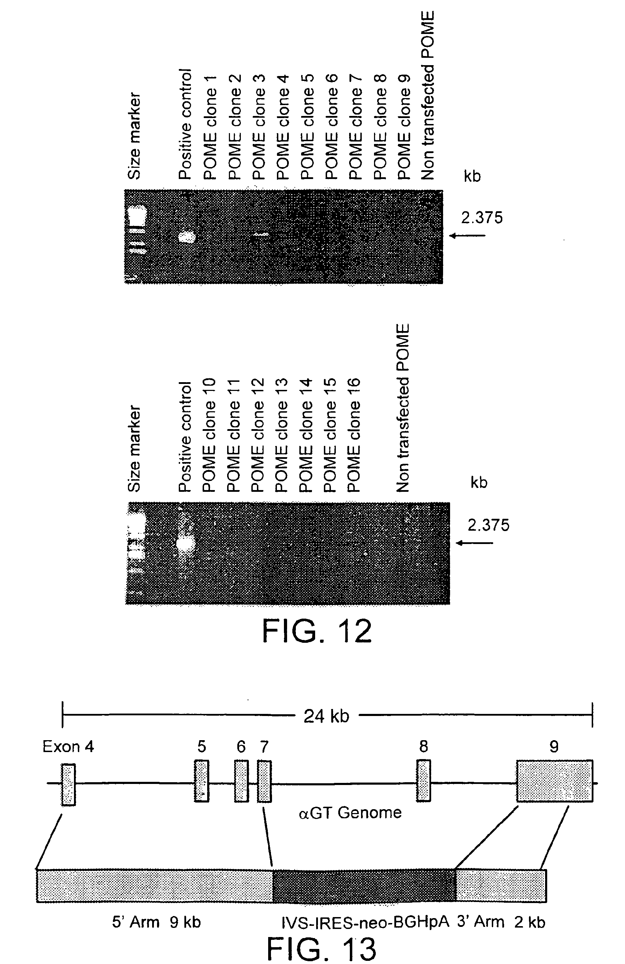

Figure 12 shows an agarose gel electropherogram of PCR products amplified from 16 G418 resistant BLAT-3 transfected POME cell clones. One of these (clone 3) shows the presence of the diagnostic 2.375 kb fragment indicative of integration of the IRES neo gene into the BLG locus by homologous recombination.

### Example 6.

Knockout of the alpha 1,3 galactosyltransferase gene locus in primary porcine fetal fibroblasts by homologous recombination.

A primary obstacle in xenotransplantation is the occurrence of hyperacute rejection (HAR) which destroys vascular endothelial cells in the donor organ by a complement-dependent mechanism. A carbohydrate antigen, Gal α1→3Gal, has been identified as the major antigen responsible for HAR (Sandrin et al., Transplant Rev 8, 134-139, 1994). Although most mammals have the Gal α1→3Gal structure, it is lacking in humans, and human sera have potent antibodies against the antigenic epitope (Galili et al., J Exp Med 160, 1519-1531, 1984). The antigenic carbohydrate structure is formed through the action of an enzyme, α 1,3-galactosyltransferase, which transfers galactose from UDP-galactose to *N*-acetyllactosamine (Galβ1→4GlcNAc), yielding Galα1→3Galβ1→4GlcNAc. Alpha1,3-galactosyltransferase knockout mice have been generated and cells from these mice are less susceptible to cytotoxic attack by human sera (Tearle et al., Transplantation, 61, 13-19, 1996). These results indicate that removal of α1,3-galactosyltransferase activity in porcine cells or organs should dramatically reduce HAR when these tissues are used for xenotransplantation. The porcine α 1,3-galactosyltransferase cDNA has been cloned and transfection analysis confirmed that these clones encoded functional α 1,3-galactosyltransferase (Sandrin et al., Xenotransplantation 1, 81-88, 1994). Recently, the porcine α 1,3-galactosyltransferase (GT) full length cDNA (3.2 Kb) has been cloned and its genomic organisation (6 exons encompassing a 24 kb region) has been reported (Katayama, A et al, Glycoconjugate J , 15, 583-589, 1998). The goal was to knockout the α 1,3 GT gene in primary porcine cells by homologous recombination, and use the knockout cells as nuclear donors for somatic cell nuclear transfer to produce GT-deficient pigs.

### Isolation of Primary Porcine PFF4 fetal fibroblasts

A porcine fetus (Large White x Yorkshire cross) at 33 days gestation was prepared for cell isolation by first removing the head and entrails, washing three times with DMEM, and then mincing the remaining fetal tissue into about 20 ml of DMEM with sterile scissors. Finely minced tissue was then transferred to a 50 ml polypropylene centrifuge tube, and spun to remove the DMEM. The tissue was then digested with collagenase (100u/ml in 40 ml), for 1 hr. at 37°C in a shaking water bath. Tissue clumps were broken apart with vigorous pipetting, and the resulting cell suspension was spun to remove the collagenase solution. The digested cell suspension was then plated in three T-75 flasks in DMEM + 10%FBS + non-essential and essential amino acids. Cells were confluent in two days, and were passaged into six T-75 flasks. After two days, the flasks were harvested, and the cells frozen in 100 1 ml cryovials.

### Construction of the porcine α-1,3 GT knockout vector (pPL501).

Cells from the primary fetal fibroblast culture, PFF4, were used for genomic DNA isolation. Genomic DNA from PFF4 cells was used to PCR clone isogenic α-1,3 GT DNA sequences for use in construction of a porcine α-1,3 GT knockout vector. The knockout vector was designed as a replacement-type vector, incorporating more than 10 kb of homology, in order to maximise the frequency of recombination. Since the α-1,3 GT gene is expressed in fibroblast cells, a promoter trap strategy was utilised to enrich for homologous recombination events.

The porcine α1,3 GT promoter-trap knockout vector, pPL501, was constructed from the following three components (figure 13):
(1) The 5' recombination arm consists of a 9 kb PCR- generated genomic sequence, from the α1,3 GT gene, from porcine primary fetal fibroblasts (PFF4) cells; which consists of 64 base pairs from the 3' end of exon 4, extending from intron 4 to intron 6, and including 81 base pairs from the 5' end of exon 7 ; The PCR primers used to clone this 9 kb region were as follows:
   GAGTGGTTCTGTCAATGCTGCT (5')
   GGAAGCTCTCCTCTGTTGTCTT (3')
(2) A promoter-less selectable neo marker consisting of a 2.057 kb BamHI-XhoI fragment, containing the IVS-IRES-neo-BGHpA expression cassette, (from pIRESneo; Clontech; GenBank Accession #U89673)
(3) A 3' recombination arm consisting of a 1.8 kb PCR-generated α1,3 GT sequence from exon 9 (starting 84 bp from the 5' end of exon 9, and extending 1844 bp to the 3' end of exon 9), also from PFF4 cells. The PCR primers used to clone this 1.8 kb sequence were as follows:
   GGTGGATGATATCTCCAGGATGCCT (5')
   GCTGTTTAGTCATGAGGACTGGGT (3')

Random integration of the promoter trap construct does not confer G418 resistance. Homologous recombination results in insertion of the promoterless neo gene, into exon 7, downstream of the endogenous α-1,3 GT promoter, allowing transcription of a bicistronic message. Translation of the neo coding region, from the targeted locus, initiates at the internal ribosome entry site. Homologous recombination results, not only in insertion of the IRES-neo cassette into exon 7, but deletion of exon 8, thus interrupting the α-1,3 GT coding region, and creating a null allele. During construction of the pPL501 vector, sequencing of the 5' junction of the 9 kb homology region, revealed a sequence polymorphism. It was not known if this was a natural polymorphism between the two PFF4 α-1,3 GT alleles, or a PCR error, therefore, a second knockout vector, pPL502 was made that is identical to pPL501, except using the polymorphic 9 kb 5' recombination arm. Both pPL501 and pPL502 were used, in equimolar amounts, for transfections.

### Transfection of Primary PFF4 cells with the alpha-1,3 GT targeting vector.

Equimolar amounts of the pPL501/502 knockout vectors were transfected in supercoiled and covalently closed circular form into early passage PFF4 cells and G418 resistant clones were isolated as summarised below:
- Day 0:: PFF4 cells at passage 3 were seeded in a 75cm² flask at 40% confluence.
- Day 1:: After 24 hours PFF4 cells were at 80% confluence, and transfected with 8µg pPL501/502 DNA using 40 µl GenePORTER reagent following the procedure recommended by the manufacturers (Gene Therapy Systems).
- Day 2:: Transfected cells were split into eight 48 well plates and eight 24 well plates in medium containing 350µg/ml G418
- Day 16:: 94 G418^{R} colonies were isolated and re-plated into 12 well dishes. 45 of the best-growing colonies were sampled for PCR analysis.
- Day 20-25:: Colonies were expanded into 25cm² flask.
- Day 25-31:: Expanded colonies were cryopreserved.

Throughout, PFF4 cells were grown in DMEM supplemented with Non-Essential Amino Acids (1X), basic fibroblast growth factor (2ng/ml), 10% fetal calf serum in standard tissue culture flasks and dishes, in a humidified tissue culture chamber using an atmosphere composed of 5%O₂, 5%CO₂, and 90% N₂. Cells were passaged by standard trypsinisation.

PCR analysis was performed to screen potential recombinants, using primers designed to distinguish between random integrants and homologous recombinants. The sequence of these primers is shown below.
Neo442s: CATCGCCTTCTATCGCCTTCTT (5')
Alpha-Gte9a2: AGCCCATCGTGCTGAACATCAAGTC (3')

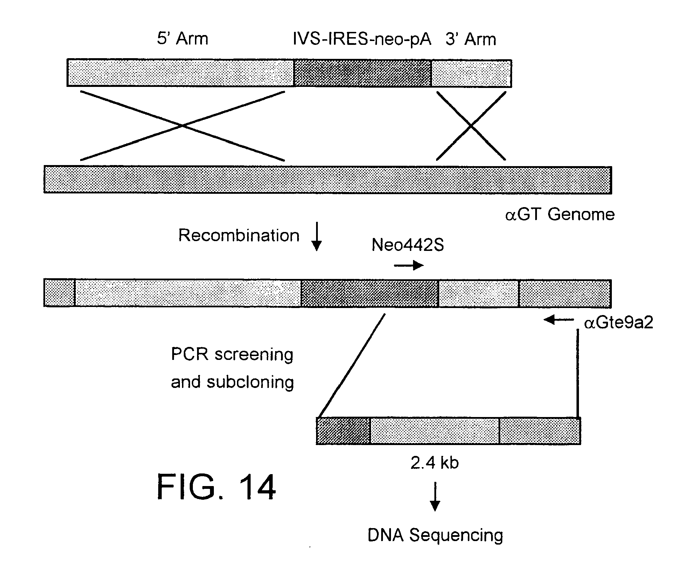

The Neo442s primer was designed to bind with sequences in the 3' end of the neo gene; while the alpha-gte9a2 primer binds to sequences in GT exon 9 outside the region included in the targeting vector. The positions of these primers relative to the predicted structure of the targeted locus is shown in Figure 14.

Cells were harvested from 45 G418^{R} colonies for PCR screening, and were lysed in PCR lysis buffer (40mM Tris-HCl pH8.9, 0.9% Triton X-100, 0.9% Nonidet P40, 400µg/ml proteinase K) at 50°C for 15 minutes. 5 µl of lysed cells (∼2-5000 cells) were incubated at 65° C for 15 minutes, followed by Proteinase K inactivation at 95° C for 10 min. PCR amplification was performed using "Expand High Fidelity" Taq polymerase (Roche Molecular Biochemicals) following the manufacturers recommended conditions. The thermal cycling conditions were as below:

| | |
|---|---|
| | 94 °C 2 min |
| 30 cycles of | |
| | 94 °C 15 sec |
| | 64 °C 30 sec |
| | 68 °C 4 min (with 20sec/cycle increasing time increments in cycles 10-30) |
| | |
| | 72 °C 7 min |

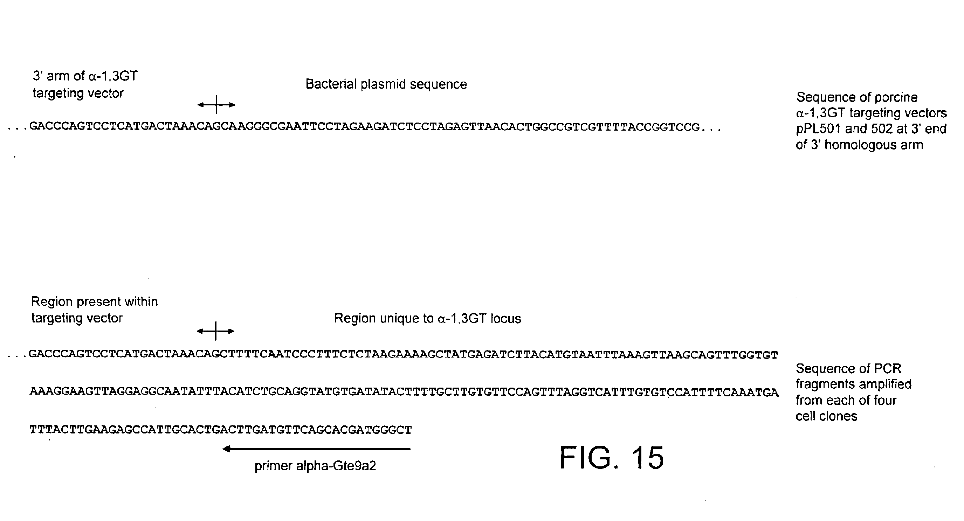

Amplification of a 2.4kb product (the 3' end of which originates in the endogenous gene) indicated that construct integration was due to homologous recombination at the correct site within the alpha1,3 GT locus. Fifteen out 45 G418^{R} colonies (33%) showed the presence of the 2.4 kb PCR fragment, diagnostic of a targeted recombination event. PCR products from four independent colonies were subcloned into the pCR-XL-TOPO vector and sequenced to verify recombination. Results illustrated in Figure 15 demonstrate that the sequences from the 3' end of each of the PCR products sequenced corresponded to the sequence expected of correct homologous recombination.

These α-1,3 GT knockout cells will now serve as nuclear donors for somatic cell nuclear transfer towards the production of pigs deficient in the alpha 1,3 galactosyl transferase enzyme.

### Example 7

Knockout of bovine β-lactoglobulin (BLG) gene locus in primary bovine fetal fibroblasts (HFF5) by homologous recombination

Bovine β-lactoglobulin (BLG) is one of the major allergens involved in human allergic responses to cow's milk proteins. Knockout of the BLG gene in cow cells, in combination with somatic cell nuclear transfer, to produce a BLG-deficient cow, and subsequently BLG-free milk, presents the opportunity to make a less allergenic and more human-like infant formula.

The bovine BLG genomic DNA and cDNA have been cloned (Hyttinen et al, J Biotechnol, 61, 191-198, 1998; Alexander et al, Nucleic Acids Res, 17, 6739, 1989). Using sequence information from GenBank (GenBank Accession # Z48305), portions of the BLG gene were recloned from primary bovine fetal fibroblast cells, for use in construction of the BLG knockout vector. Since the BLG gene is not expressed in primary fetal fibroblasts, the BLG knockout vector was designed to incorporate a polyA-trap strategy to enrich for homologous recombination events.

### Isolation of primary bovine HFF5 fetal fibroblasts

An elite Holstein male fetus at approximately 55 days gestation was collected from a local abattoir and prepared for isolation. The fetus was first removed from the placental membranes and then washed three times with Dulbecco's modified Eagles medium (DMEM) at room temperature. In a fresh 20ml aliquot of DMEM, the surface skin and subdermal tissue was stripped from the skeletal structure using small scissors and then diced as finely as possible. Minced tissue was then spun down and the DMEM was replaced with 50 ml of collagenase at 100units per ml of DMEM. Tissue was digested for 1 hr in a 37°C water bath. Minced tissue was then broken apart with repeated pipetting and the resulting cell suspension was spun to remove the collagenase solution. The cell pellet was then resuspended in complete DMEM plus 10% fetal bovine serum, non essential amino acids (1X) and basic fibroblast growth factor (2ng/ml) plus gentamycin. Cells were seeded into 4 T-75 flasks. Resulting cells were confluent and frozen three days post isolation.

### Poly A-trap Knockout Vector Construction

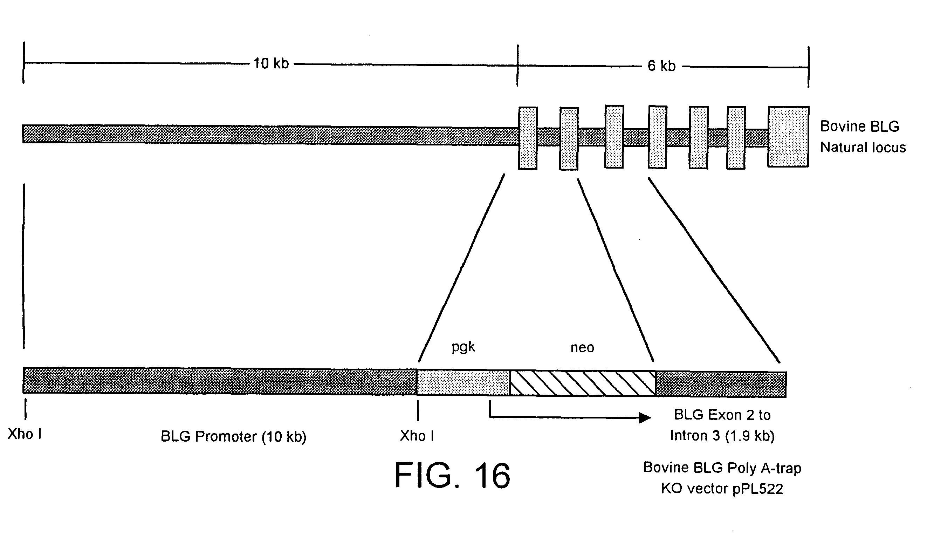

The bovine BLG polyA-trap knockout vector pPL522 was constructed from the following three components (see figure 16):
(1) The 5' recombination arm consists of a 10 kb PCR-generated genomic sequence comprising the BLG promoter region through to 89bp downstream from the first ATG in exon 1, derived from DNA isolated from primary Holstein fetal fibroblasts (HFF5). The primers used to generate the PCR fragment were:
   BoBLGpro: 5' CCA GTG CTG ATT TGA TTT CCT ACT CAC GCC 3'
   BoBLGpro7: 5' ACC TTC TGG ATA TCC AGG CCC TTC ATG GTC 3'
(2) A poly A-less selectable marker comprising a 1415bp Xho I - Sma I fragment containing the mouse phosphoglycerate kinase (PGK) promoter, and a neo gene cassette without a poly A signal, from pPL442 which was derived from pKOTKneoV904 (Lexicon Genetics Inc., Woodlands, Texas);
(3) The 3' recombination arm is a PCR-generated 1935 bp Nco I-BamH I fragment of BLG genomic sequence (GenBank Accession # Z48305) from HFF5 cells. This 3' arm contains BLG sequences comprising 114bp from the 3' end of exon 2, intron 2, exon 3 and 885bp from the 5' end of intron 3.

### Transfection of Primary HFF5 cells with the BLG knockout vector

HFF5 cells were maintained at 37°C, with 5% CO₂, in DMEM medium supplemented with 10% Fetal calf serum, Non-Essential Amino Acids (1X), and basic fibroblast growth factor (2ng/ml). The selection medium contained 350µg/ml (active concentration, Gibco-BRL) of G418.

BLG knockout vector pPL522 DNA was transfected without linearisation by restriction digestion (supercoiled and closed circular form) into HFF5 cells by lipofection, using GenePORTER (Gene Therapy Systems), and the G418 resistant colonies were selected as follows:
Day 0: HFF5 cells at passage 1 (1 million cells per vial) were thawed from liquid nitrogen and seeded into a 25cm² flask
Day 1: Cells at approximately 60% confluence were transfected with 2 ml of transfection media containing 1 µg of undigested pPL522 DNA (>70% supercoiled) and 20 µl of GenePORTER reagent. Cells were incubated in 5% CO₂ at 37° C. After 4 hours, 2ml of growth medium (DMEM + 20% FCS) was added.
Day 2-12: Transfected cells were trypsinised at 24 hrs post transfection and seeded into 24 well plates at a density of 2500 to 5000 cells per well in selection medium with 350 µg/ml of G418. Cells were incubated in 5% CO₂ at 37° C. Medium was changed every 3 days during selection.
Day 13-15: Eighty-five colonies were passed from 24 well plates to 12 well plates. Wells which contained more than one distinguishable colony were harvested as pools (8 to 12 colonies per pool). A total of 21 pools were submitted for PCR analysis.
Day 15-17: Fifty-two well-isolated, single colonies from 12 well plates, upon reaching confluence, were passed to 60mm dishes. An aliquot of each colony was submitted for PCR analysis.
Day 17-22: The remaining cells from each of the 52 single colonies were frozen (2 vials for each colony), and stored at -70° C.

### PCR screening of G418 resistant colonies

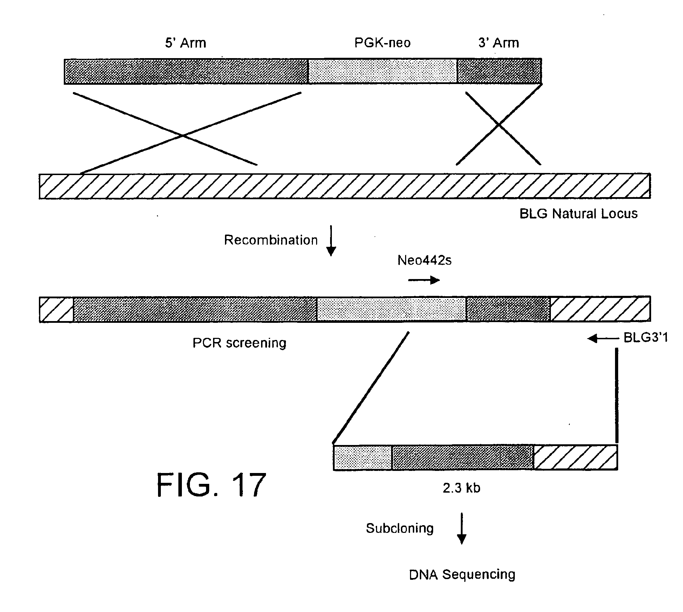

G418 resistant colonies and pools were screened by PCR analysis, using primers designed to distinguish between random integration and homologous recombination events (Figure 17). The 5' primer (Neo442s) binds to the 3' end of the neo gene within the knockout vector. The 3' primer (BLG3'1) binds to a region of bovine BLG exon 4 which is outside of sequences present in the knockout vector (approximately 260 bp 3' of the 3' end of the BLG 3' homologous arm in the vector. The primer sequences are shown below:
Neo442s: 5' CAT CGC CTT CTA TCG CCT TCT T 3'
BLG3'1: 5' CCA GCA CAA GGA CTT TGT TCT C 3'

Cells were lysed in PCR lysis buffer (40mM Tris-HCl pH8.9, 0.9% Triton X-100, 0.9% Nonidet P40, 400µg/ml proteinase K) at 50° C for 15 minutes. Five µl of lysed cells (5000-10000 cells) were incubated at 65°C for 15 minutes, followed by proteinase K inactivation at 95°C for 10minutes. PCR amplification was performed using Expand High Fidelity Taq (Roche Molecular Biochemicals) and the manufacturer's protocol with the following thermal cycling program:

| | |
|---|---|
| 94°C | 1 minute |
| 60°C | 1 minute |
| 72°C | 2 minutes |
| 35 cycles | |

Amplification of a 2.3kb product (the 3' end of which originates in the endogenous gene, not the construct) indicates that construct integration was due to homologous recombination within the BLG locus.

### Subcloning and sequencing of PCR products

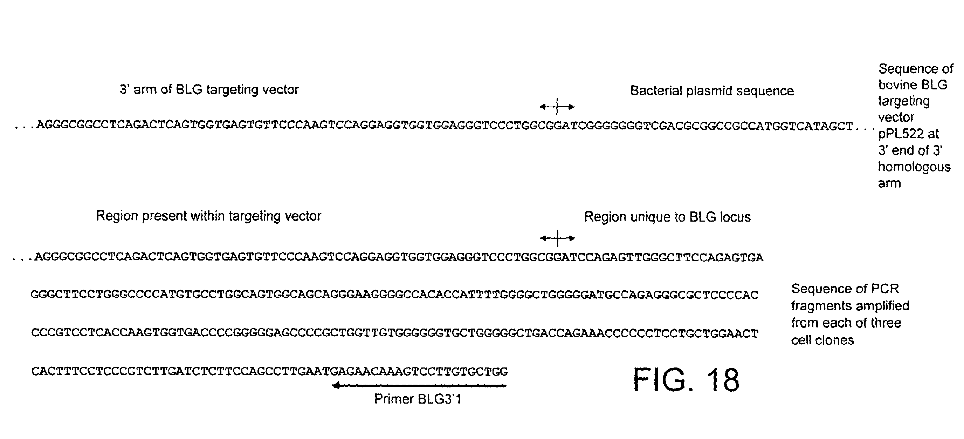

Out of 52 single colonies and 21 pools (each pool comprising 8 to 12 colonies), 5 single colonies (9.6%) and 17 pools (81%) showed positive PCR products. PCR products from 3 single colonies were subcloned into the pCR-XL-TOPO vector (Invitrogen), and two subclones from each of the 3 single colonies (6 clones total) were sequenced, generating more than 400bp of sequence from both ends. Sequencing results showed that all subclones matched the sequence expected after correct homologous recombination (Figure 18).

### Example 8

Detection of transgenic AAT in the milk of collagen locus targeted sheep.

Induced lactation milks from the sheep produced in Example 4 were diluted 1:100 with 50mM Na Phosphate, pH 7.2 and 10µl treated with an equal volume of nonreducing SDS sample buffer before loading onto an 8-16% Tris/Glycine SDS gel. An equivalent amount of control sheep milk (tgAAT negative) was also loaded. 200ng of sheep milk known to include tgAAT at a known concentration was loaded as a positive control.

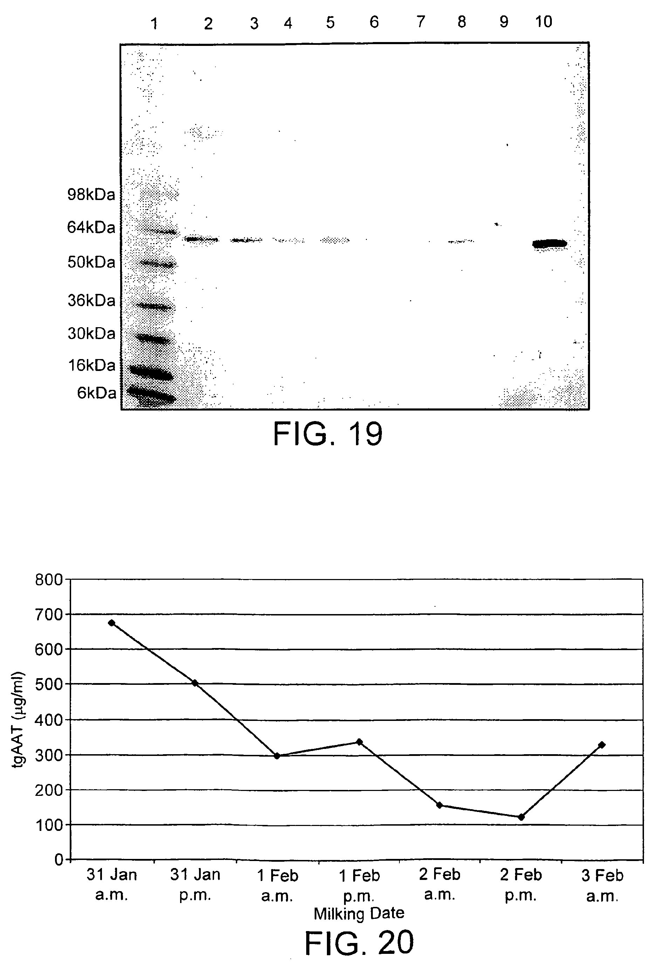

After separation, the proteins were transferred to a PVDF (polyvinyl difluoride) membrane which was blocked with 2%BSA in PBS for 1 hour. The blot was probed for 30min with a rabbit anti-human AAT polyclonal antibody diluted 1:2000 with 1%BSA/PBS/0.1% Tween-20 (PBS-T). After washing 5 times over 30min with PBS a goat anti-rabbit IgG-horseradish peroxidase labelled antibody was added for 30min at a dilution of 1:5000 in 1%BSA/PBS-T. After a final wash with PBS as above, the blot was developed using DAB (diamino benzidine) substrate (Figure 19).

| Lane | Sample | Mass/Volume |
|---|---|---|
| 1 | See Blue markers | 10µl |
| 2 | Jan 31 a.m. | 0.1µl |
| 3 | Jan 31 p.m. | 0.1µl |
| 4 | Feb 01 a.m. | 0.1µl |
| 5 | Feb 01 p.m. | 0.1µl |
| 6 | Feb 02 a.m. | 0.1µl |
| 7 | Feb 02 p.m. | 0.1µl |
| 8 | Feb 03 a.m. | 0.1µl |
| 9 | Control Milk (negative) | 0.1µl |
| 10 | TgAAT (positive) | 200ng |

The developed blot was scanned and densitometry carried out to assess the expression level of tgAAT. Peak areas of the tgAAT bands were used to calculate the amount present in each lane with reference to the known positive standard material. Finally, these values were corrected for the initial dilution to give the expression level in whole milk (Table 6 below). The change in expression level across the milking is shown graphically in Figure 20.

The higher molecular weight band seen in the targeted sheep milk samples corresponds to the mass of IgG and is the likely to be the case if IgG levels are particularly high in these early milking samples (colostrum-like). Their appearance in the blot may be due to a slight cross-species reactivity with the anti-IgG secondary antibody.

**Table 6: expression levels of tgAAT in sample milks as assessed by densitometry with reference to a known positive control sample.**

| Milking | Expression level (whole milk) in µg/ml |
|---|---|
| 31 Jan a.m. | 674.5 |
| 31 Jan p.m. | 502.7 |
| 1 Feb a.m. | 298.3 |
| 1 Feb p.m. | 337.6 |
| 2 Feb a.m. | 154.3 |
| 2 Feb p.m. | 120.8 |
| 3 Feb a.m. | 329.7 |

The sheep is producing tgAAT in its milk. This amount compares favourably with the highest level previously reported for an AAT cDNA transgene in sheep carrying multiple random gene inserts (18 µg/ml) as described in McClenaghan M et al, (Animal Biotechnology 2 161-176 1991). Interestingly and most surprisingly, this finding indicated that the COL1AI locus may provide a permissive environment for transgene expression even though type 1 collagen is not itself actively expressed in mammary epithelium; Warburton et al, (J. Cell Physiol. 128 76-84 1986).

In this example, hormone induction of lactation in the immature ewes was carried out essentially as described by Ebert et al., (Biotechnology 12, 699-702, 1994). The ewe was injected twice daily for seven days with oestradiol benzoate (0.05 mg/kg) and Progesterone (0.1250 mg/kg) to encourage mammary development and prolactin release. Eighteen days after the first injection, the ewe was injected each morning for three days with dexamethasone (5mls/day). Throughout the induction, the ewe was housed under lights which mimic natural daylight. Twenty-one days after the first injection, the udder was inspected and milking took place.

### Example 9

### Description of double nuclear transfer.

### I) PROTOCOL FOR PORCINE NUCLEAR TRANSFER.

### Synchronisation procedure for the collection of in vivo matured Porcine Oocytes.

A suitable procedure for the collection of *in vivo* matured porcine oocytes is described below. In practice any procedure which results in the production of suitable oocytes may be used.

Feed Altrenogest (Regu-Mate, 18 mg/day, 9 AM) for 5 - 9 days, starting on day 12-16 of the cycle (day 0 is heat). If no heats are available, feed Altrenogest for 14 days. Inject gilts with Estrumate (IM, 1.0 ml, 9 AM) during the last Alternogest feeding. Inject eCG (Equine Chorionic Gonadotropin, 1500 IU IM) 14 hrs (11 PM) after the last Altrenogest feeding. Inject hCG (human Chorionic Gonadotropin, 1000 IU, IM) 80 hrs after the eCG injection (7 AM). Collect oocytes 50 hrs after the hCG injection (9 AM).

### FIRST STAGE NUCLEAR TRANSFER EMBRYO RECONSTRUCTION.

A range of protocols are available for the first stage nuclear transfer. Their use is dictated by the cell cycle stage of the donor cell nucleus at the time of transfer.

### 1. "MAGIC" (Metaphase Arrested G1/G0 Accepting Cytoplast).

Enucleated oocytes were maintained in calcium free medium 10% FCS at 39°C. In the first stage nuclear transfer it is presently preferred, but not essential, that the donor genetic material is introduced into the enucleated oocyte by piezzo aided injection. To aid injection the cytoplast recipients may be centrifuged at 13,000xG for 5 minutes. The donor cell is placed into medium containing 12% Polyvinyl Pyrollidone (optional), the cell is drawn into the injection pipette and then injected into the oocyte, the cell membrane may be intentionally damaged by selection of an appropriately sized pipette and/or the application of a burst of piezzo vibration and repeated pipetting. The size of the injection pipette is dependent upon the size of the cell. Alternatively, transfer of the nucleus may be achieved by cell fusion. As soon as possible after enucleation a single cell was placed into contact with the oocyte by using a glass pipette to transfer the cell through the hole previously made in the zona pellucida. The cytoplast/cell couplet was then transferred into fusion medium (0.3 M D-Sorbitol supplemented with0.1mM Mg SO₄ & 0.05 mM CaCl₂ in H₂0, osmolality 280mOsM) in the fusion chamber. The couplet was manually aligned between the electrodes. Fusion was induced by application of two DC pulses of 1kV/cm for 60 µsec at intervals of 5 sec. The couplets were then washed in calcium free medium supplemented with 10% FCS at 37°C and incubated in the same medium under oil at 37°C 5% CO₂. 30 minutes prior to activation the couplets were transferred to calcium free medium 10% FCS containing 5µM Nocodazole. Activation was induced as described below, following activation the reconstructed zygotes were incubated in medium NCSU23, 10% FCS at 37°C 5% CO₂ for a further 3 hours. They were then washed 3 times for 5 minutes at 37°C in the same medium without nocodazole and cultured for a further period of time sufficient for the formation of a pronucleus prior to being used as nuclear donors for the second stage nuclear transfer embryo reconstruction. Alternatively, the donor nucleus may be transferred by either manual or piezzo aided injection or by any other chemical or physical means of producing cell fusion.

### 2. "GOAT" (G0/G1 ACTIVATION AND TRANSFER).

Enucleated oocytes were returned to the maturation medium. At 30 or 42 hours post onset of maturation a single cell was placed into contact with the enucleated oocyte.

The couplet was transferred to the fusion chamber (see below) in fusion medium 0.3 M D-Sorbitol supplemented with 0.1mM MgSO₄ & 0.05 mM CaCl₂ in H₂0, osmolality 280mOsM. Fusion and activation were induced by application of two DC pulses of 1kV/cm for 60 µsec at intervals of 5 sec. Couplets were then washed in NCSU23 10% FCS and incubated at 37°C 5% CO₂ for a further period of time sufficient for the formation of a pronucleus prior to being used as nuclear donors for the second stage nuclear transfer embryo reconstruction. Alternatively, the donor nucleus may be transferred by either manual or piezzo aided injection or by any other chemical or physical means of producing cell fusion.

### 3. "UNIVERSAL RECIPIENT".

Enucleated oocytes were activated (as described below) and cultured in NCSU23, 10% FCS at 37°C 5% CO₂. After the decay of MPF activity, a single cell was then placed into contact with the oocyte and fusion induced as described below. The couplets were then cultured in NCSU23 10% FCS at 37°C 5% CO₂ for a further period of time sufficient for the formation of a pronucleus prior to being used as nuclear donors for the second stage nuclear transfer embryo reconstruction. Alternatively, the donor nucleus may be transferred by either manual or piezzo aided injection or by any other chemical or physical means of producing cell fusion.

### PREPARATION OF A CYTOPLAST FOR THE SECOND NUCLEAR TRANSFER EMBRYO RECONSTRUCTION.

A suitable cytoplast may be prepared by activation of an enucleated oocyte as described in the 'UNIVERSAL RECIPIENT'. Alternatively, as is preferred at present, a cytoplast recipient may be prepared from any *in vivo* or *in vitro* produced zygote. After pronuclear formation the zygote may be centrifuged, in a suitable buffered medium, at 13.000x for 5 minutes, this aids visualisation of the pronuclei but is not essential. The centrifuged zygotes are placed into the manipulation chamber in a suitable medium (as above) and a portion of cytoplasm containing the two pronuclei aspirated using a micropipette.

### SECOND STAGE NUCLEAR TRANSFER EMBRYO RECONSTRUCTION.

Synchronisation procedure for the collection of in vivo fertilised 1-cell porcine zygotes.

A suitable procedure for the collection of *in vivo* matured porcine oocytes is described below. In practice any procedure which results in the production of suitable oocytes may be used.

Feed Altrenogest (Regu-Mate, 18 mg/day, 9 AM) for 5 - 9 days, starting on d 12-16 of the cycle (day 0 is heat). If no heats are available, feed Altrenogest for 14 days. Inject gilts with Estrumate (IM, 1.0 ml, 9 AM) during the last Altemogest feeding. Inject eCG (Equine Chorionic Gonadotropin, 1500 IU IM) 14 hrs (11 PM) after the last Altrenogest feeding. Inject hCG (human Chorionic Gonadotropin, 1000 IU, IM) 79 hrs after the eCG injection (6 AM). Artificially inseminate 24 and 36 hrs after hCG injection. Collect embryos 50 hrs after the hCG injection (noon).

### Reconstruction of the second stage nuclear transfer embryo.

The karyoplast formed by aspiration of the pronucleus surrounded by a portion of cytoplasm and membrane bound from the first nuclear transfer embryo is placed below the zona pellucida of the second recipient cytoplast in contact with the cytoplast. Fusion of the karyoplast and cytoplast are carried out as below or by any other means. Alternatively the karyoplast, the pronucleus or the pronucleus surrounded by a portion of cytoplasm may be injected into the second nuclear transfer embryo.

Culture of reconstructed embryos.

Reconstructed embryos may be cultured *in vitro* using any suitable culture medium and conditions e.g. NCSU medium supplemented with 10%FCS 37°C in a humidified atmosphere of 5.0% CO₂. Alternatively, as is presently the method of choice in porcine species the reconstructed embryos may be transferred directly to a synchronised final recipient for development to term. The transfer of embryos to a synchronised recipient has been determined to support good embryo and foetal development. In control experiments porcine zygotes were sham manipulated to mimic the zona pellucida damage occurring during the nuclear transfer procedure. These manipulated embryos were then transferred to a suitable recipient. Subsequently the recipient was sacrificed and development of the transferred embryos assessed (Table 7).

Table 7. Embryo development and pregnancy induction using *in vivo* derived micromanipulated porcine zygotes. Two holes were made by enucleation pipette in zona pellucida to mimic nuclear transfer micromanipulation. The embryos were then transferred to the recipients.

| No. of transferred "manipulated" 1 cell embryos | Results |
|---|---|
| 48 | 26 compacted morulae, and 15 16-cell embryos were collected on Day 6. They were cultured in NCSU-23 for 24hrs. 29 embryos developed to blastocyst stage. |
| 34 | Open - Slaughter/Pregnancy Check (SPC) on day 21 |
| 35 | Pregnant, SPC on day 21. Four fetuses and 5 reabsorbtion sites were observed. No corpus luteus (CL's), new follicles were present. Pregnancy would be lost. |

Manipulated embryos were able to develop to blastocysts and healthy foetuses.

### Fusion and Activation

For activation, oocytes were placed between two parallel electrodes in activation medium 0.3 M D-Sorbitol supplemented with0.1mM Mg SO₄ & 0.05 mM CaCl₂ in H₂0, osmolality 280mOsM. Activation was induced by application of two DC pulses of 1kV/cm for 60 µsec at intervals of 5 sec. This activation procedure promotes development of parthenogenetic embryos to the blastocyst stage (see Table 8 below). However, any other suitable medium may be used for example 0.3 M mannitol, 0.1mM MgSO₄, 0.001mM CaCl₂ in distilled water (Willadsen, 1986). Any suitable activation pulse may be used for instance 1 DC pulse of 1.25 kV/cm for 80µs. For fusion manipulated embryos were treated in a similar manner with the addition that the contact surface between the enucleated oocyte and the cell was arranged parallel to the electrodes. Fusion was induced by application of an AC. current of 3V for 5 seconds followed by DC pulses of 1.0 kV/cm for 60 µs at 5 second intervals.

**Table 8. Development to the blastocyst stage of porcine oocytes electrically activated at different ages.**

| Oocyte age. Hrs post hCG | 51.5 hrs (%) | 54 hrs (%) | 57 hrs (%) | 60 hrs (%) |
|---|---|---|---|---|
| 1 experiment | 1/13 (8) | 0/13 | 0/13 (0) | 2/14 (14) |
| 2 experiment | 7/16 (44) | 5/16 (31) | 8/16 (50) | 8/16 (50) |
| Average | 8/29 (28) | 5/29 (17) | 8/29 (28) | 10/30 (33) |

### Embryo culture and assessment (all groups)

Following reconstruction embryos may be cultured by a variety of techniques known to those skilled in the art including; *in vitro* culture in a suitable medium or *in vivo* culture in the ligated oviduct of a suitable recipient host until a stage for transfer to a final surrogate recipient. Alternatively, as is presently the method of choice in porcine species although not essential, the second stage reconstructed embryos may be transferred directly to the oviduct of a suitable synchronised final recipient host for development to term (see Tables 9 and 10).

**Table 9. Development of double nuclear transfer porcine embryos. first stage nuclear embryos were reconstructed by pizzo injection of diploid primary fibroblasts into in vivo matured enucleated porcine oocyte. second stage nuclear transfer used in vivo produced porcine zygotes. Reconstructed embryos were transferred to the oviduct of a synchronised recipient sow and recovered on day 6 to assess development**

| Group | Number attempted fusions | Number Lysed (%) | Number Fused (%) | Number of Blastocysts (%) |
|---|---|---|---|---|
| 1 | 23 - fusions | 0 (0) | 13/23 (57) | 4 (31) |

**Table 10: Pregnancy outcome following transfer of double nuclear transfer embryos directly to a synchronised final recipient.**

| Replicate | Number Of Embryos Transferred | Pregnancy And Comments |
|---|---|---|
| 1 | 48 | Pregnant d28, open d34 |
| 2 | 4 | Open d28, Open d35 |
| 3 | 4 | Open d28, Open d35 |
| 4 | 9 | Not tested |

Synchronisation procedure for the preparation of surrogate recipients for nuclear transfer reconstructed embryos.

Same method as used for the preparation of zygotes with the following differences, no eCG, no AI and 1.5 days behind.

### Procedure for the maintenance of pregnancy in surrogate recipients.

In order to maintain pregnancy in the final recipient *in vivo* produced embryos at the same developmental stage as the reconstructed embryos may be transferred with the nuclear transfer embryos. Alternatively, as is presently preferred in porcine species the final recipient may be treated hormonally to maintain pregnancy in the following manner, eCG (1000 IU) on day 9 or day 10 of pregnancy, hCG (500 IU) 3 to 3.5 days later.

### Example 10

### Generation of cloned pigs using somatic cells as nuclear donors.

### Isolation of porcine granulosa cells:

Granulosa cells were harvested from ovarian follicles by needle aspiration of follicular fluid. Animals from which ovaries were collected were prepared identically to those used for oocyte collection (see Oocyte recovery below). Follicles were aspirated by penetrating through the cortex of the ovary using an 18 gauge hypodermic needle attached to a 10 ml syringe. Follicular fluid was collected from follicles between 2 and 5 mm in diameter, was placed into a 15ml conical centrifuge tube, and centrifuged at 1000 rpm for 10 min. The cell pellet was re-suspended into DMEM (Gibco), containing 10% FCS (Summit Biotech), NEAA, 2 ng/ml bFGF and 6 µl/ml gentamycin. Cells were expanded for several days and then cryopreserved.

### Preparation of cells for nuclear transfer.

Donor cells were plated at 1-5 X 10⁴ cells per 35 mm dish and were allowed to reach 100% confluency in DMEM medium supplemented with NEAA (0.1 mM), bFGF (2ng/ml), and 10% fetal calf serum. Contact inhibition was chosen for cell synchronization in order to maximize the number of healthy cells with a diploid DNA content. See cell cycle data for details.

Cells were harvested by trypsinisation and stored in suspension in a calcium-free phosphate buffered version of NCSU-23 medium at 38.5 °C for 20-120 minutes, prior to use as nuclear donors.

### Cell cycle analysis (granulosa cells)

Since MII oocytes were used as recipient cytoplasts for the first step of nuclear transfer (NT), donor cells having a diploid DNA content were chosen to maintain the appropriate coordination of ploidy of the resultant reconstructed embryo. The position within the cell cycle of various populations of cells after 8 days of culture, was determined by measuring the DNA content using flow cytometry. Three populations were analyzed: sub-confluent actively growing granulosa cells, contact inhibited granulosa cells, and granulosa cells serum starved for 48 hours.

The results of this experiment are summarized in Table 11. It was observed that following serum starvation, the granulosa cell population contained a large proportion (7.2%) of structures having a DNA content lower than that consistent with a cell in G1 (they were sub-G1). These structures are characteristic of cells undergoing apoptosis and senescence, and thus indicated that the granulosa cells did not tolerate serum starvation very well. Synchronization of the cells by contact inhibition (100% confluent) was more effective, given that a higher proportion of cells were in G1/G0 (90.3%), and there were fewer apoptotic sub-G1 cells in the population (only 1.6%).

**Table 11. Cell cycle analysis of granulosa cells. Values are expressed as the percentage of the total number of cells in the population.**

| Stage Of Cell Cycle | Non-Starved | Serum Starved | 100% Confluent |
|---|---|---|---|
| G1/G0 | 78.8 | 82.3 | 90.3 |
| S | 3.7 | 1.9 | 2.2 |
| G2/M | 13.9 | 6.7 | 4.5 |
| Sub G1 | 0.7 | 7.2 | 1.6 |

### Synchronization and superovulation of gilts for oocyte and zygote recovery

### Oocyte recoveries.

Crossbred gilts (280-320 lbs.) were synchronized, superovulated and oocytes were collected. Gilts were synchronized by oral administration of 18-20 mg Regu-Mate (Altrenogest, Hoechst) mixed into the feed. Regu-Mate was fed for 5-14 days using a scheme that is dependent on the stage of the estrous cycle. Estrumate (250 µg, Bayer) was administered IM the last day of the Regu-Mate treatment. Superovulation was induced with a 1600 IU IM injection of Pregnant Mare Serum Gonadotropin (PMSG, Diosynth) 15-17 hours after the last Regu-Mate feeding. 1500 units of human Chorionic Gonadotropin (hCG, Intervet America) were administered IM 82 hours after the PMSG injection. Oocytes were collected 50-52 hours after the hCG injection using Dulbecco's phosphate buffered saline containing BSA (4 g/l).

### Pregnancy maintenance

Pregnancy was maintained by using a combination of Pregnant Mare Serum Gonadotropin (PMSG) and Human Chorionic Gonadotropin (hCG). PMSG (1000 IU) was injected IM on d10 of the estrous cycle, d1 being the day of estrus. Human Chorionic Gonadotropin was injected IM 3 to 3.5 days later, on d13 of the cycle.

### Zygote recoveries.

Crossbred gilts (280-320 lbs.) were synchronized, superovulated and zygotes were collected. Gilts were synchronized by oral administration of 18-20 mg Regu-Mate (Altrenogest, Hoechst) mixed into the feed. Regu-Mate was fed for 5-14 days; dependent on the stage of the estrous cycle the gilts were in. Estrumate (250 g, Bayer) was administered IM the last day of the Regu-Mate treatment. Superovulation was induced by a 1600 IU IM injection of Pregnant Mare Serum Gonadotropin (PMSG, Diosynth) 15-17 hours after the last Regu-Mate feeding. 1500 units of Human Chorionic Gonadotropin (hCG, Intervet America) were administered 78 hours after the PMSG injection. The gilts were then either artificially inseminated or bred naturally 24-36 hours after the hCG injection. Zygotes were collected 52-54 hours after the hCG injection using Dulbecco's phosphate buffered saline containing BSA (4 g/l).

### Oocyte enucleation.

Recovered oocytes were maintained at 38°C, washed in phosphate buffered saline (PBS) with 4 g/l BSA and transferred to calcium free phosphate buffered NCSU-23 medium, at 38°C. To remove the chromosomes (enucleation) oocytes were first placed in calcium free phosphate buffered NCSU-23 medium containing 5 µg/ml Cytochalasin B (Sigma) (Cytochalasin B is optional) and 7.5 µg/ml Hoechst 33342 (Sigma) at 38°C for 20 minutes. A small amount of cytoplasm from directly beneath the 1st polar body was then aspirated using a 18 µM glass pipette. Enucleation was confirmed by exposing the aspirated portion of cytoplasm in the pipette to UV light, and checking for the presence of a metaphase plate.

### Embryo reconstruction (Day 1).

Groups of 10-20 oocytes were enucleated and placed into 20µl drops of calcium free phosphate buffered version of the NCSU-23 medium at 38°C under mineral oil (SIGMA). At 53-56 hours post hCG injection, a single cell was placed under the zona pellucida in contact with the enucleated cytoplasm. The couplet was transferred to a fusion chamber (model # BT-453, BTX Inc. San Diego, CA) containing 700µl of 0.3 M mannitol, 0.1mM MgS0₄, 0.1mM CaCl₂ in deionized water. Fusion and activation were induced by application of an AC pulse of 5V for 5 seconds followed by 2 DC pulses of 1.5kV/Cm for 60µsec using an ECM2001 Electrocell Manipulator (BTX Inc., San Diego, CA). Couplets were then washed in bicarbonate buffered NCSU-23 medium, and incubated in this medium for 0.5-1 hour at 38.6°C in a humidified atmosphere consisting of 5% CO₂ in air. Couplets were then checked for fusion at 300X magnification using an inverted microscope. Fused reconstructed embryos were artificially activated using 2 successive DC pulses of 1.2kV/cm for 60µsec each, and cultured overnight.

### Double Nuclear Transfer (Second day)

The double nuclear transfer procedure refers to the reconstruction of enucleated zygotes on day 2, using nuclei produced by day 1 nuclear transfer.

Assessment of nuclear formation from previous day (day 1) of nuclear transfer : Reconstructed embryos from previous day of nuclear transfer were removed from culture and centrifuged in a Biofuge at 13,000 rpm for 15 min to observe nucleus formation. Embryos were evaluated for the presence of a pseudo-pronucleus by observation at 300X using an inverted microscope. Embryos with a pseudo-pronucleus were sorted, counted, and placed back in an NCSU-23-containing culture plate in an incubator at 38.6°C, with 5% CO² in air.

### Zygote Enucleation:

Zygotes were centrifuged at 13,000 rpm for 15 min in Biofuge 13 centrifuge. The zygotes were then placed in phosphate buffered NCSU-23 medium containing 5.0µg/ml Cytochalasin B (Sigma) at 38°C, and incubated for 20 minutes.incubated for 20 minutes. Fertilized zygotes with 2 pronuclei were used for enucleation.

To remove the nuclei, an enucleation pipette (25-35 µm) was inserted through the zona pelucida and into the cytoplasm, so that the opening of the enucleation pipette was close to the pronuclei. Both pronuclei and the polar bodies were aspirated by slowly applying negative pressure on the enucleation line.

### Reconstruction of Enucleated Zygotes:

Day 1 reconstructed embryos were placed into phosphate buffered NCSU-23 medium containing Cytochalasin B and Nocodazole. A 30-45 µm enucleation pipette was used for manipulation. Karyoplasts were prepared by aspirating the psuedo-pronucleus with minimal surrounding cytoplasm. These karyoplast were slowly expelled into the perivitelline space of enucleated zygotes. The couplets were transferred to the fusion chamber containing 700µl of SOR2 medium. Fusion was induced by application of an AC pulse of 5V for 5 seconds followed by 2 DC pulses of 1.2kV/cm for 60gsec. Couplets were then washed with NCSU-23 medium, incubated in this medium for 0.5-1 hour at 38.6°C, 5% CO₂ , and then checked for fusion. Fused couplets were transferred as soon as possible to the oviduct of an estrus-synchronized recipient gilt. Results of nuclear transfer using primary granulosa cells are summarized in Table 12.

**Table 12. Nuclear transfer using porcine granulosa cells**

| | |
|---|---|
| No. reconstructed embryos - Day 1 | 196 |
| No. fused embryos - Day 1 (%) | 124/183 (68) |
| No. reconstructed embryos - Day 2 | 74 |
| No. fused embryos - Day 2 (%) | 72/74 (97) |
| No. embryos transferred to recipient | 72 |
| No. fetuses detected at day 29 by ultrasound scan | 3¹ |
| No. fetuses detected at day 42 by ultrasound scan | 2² |

| | |
|---|---|
| ¹ at least 3 fetuses; ² at least 2 fetuses. It was difficult to determine the exact number of the fetuses by ultrasound in pigs due to the anatomical position of the pig uterus. | |

Ultrasound images confirmed the pregnancies at day 29 and day 42 of gestation.

## Claims

1. A method of nuclear transfer comprising:
(a) preparing a non-human somatic cell for nuclear transfer by a method comprising modifying the genetic material of the non-human somatic cell by a genetic targeting event at an endogenous locus; and
(b) transferring the genetic material from the non-human somatic cell to a non-human recipient cell.

2. A method as claimed in claim 1, wherein the genetic targeting event is mediated by homologous recombination.

3. A method, as claimed in claim 1 or claim 2, wherein the modification is inactivation, removal or modification of a gene; upregulation of a gene, gene replacement or transgene placement.

4. A method as claimed in any one of claims 1 to 3, wherein the genetic targeting event results in a gene targeted cell clone: randomly targeted cell clone ratio of equal to or greater than 1:100.

5. A method as claimed in any one of claims 1 to 4, wherein the genetic targeting event is carried out at a locus abundantly expressed in the host somatic cell.

6. A method as claimed in any one of claims 1 to 5, wherein a structural gene is placed adjacent to an endogenous promoter.

7. A method as claimed in claim 6, wherein the endogenous promoter is that of a collagen gene.

8. A method as claimed in claim 6, wherein the endogenous promoter is that of a milk protein gene.

9. A method as claimed in claim 6, wherein the endogenous promoter directs abundant expression in fibroblast cells.

10. A method as claimed in claim 6, wherein the endogenous promoter directs abundant expression in endothelial cells.

11. A method as claimed in any one of claims 1 to 10, wherein the genetic targeting event is mediated by lipofection.

12. A method as claimed in any one of claims 1 to 11, wherein the genetic targeting event involves the use of a gene targeting vector which vector comprises a long region of homology to the target locus.

13. A method as claimed in any one of claims 1 to 12, wherein the genetic targeting event involves the use of a gene targeting vector which is in a circular form.

14. A method as claimed in any one of claims 1 to 13, wherein the genetic targeting event includes the artificial induction of gene expression or the induction of chromatin changes in the cell.

15. A method as claimed in any one of claims 1 to 14, wherein the genetic targeting event is facilitated by an agent which inhibits histone deacetylation or by expression in the cell of a factor which stimulates transcription at the target locus.

16. A method as claimed in any one of claims 1 to 15, wherein the non-human somatic cell is a primary somatic cell.

17. A method as claimed in any one of claims I to 16, wherein the non-human somatic cell is an epithelial cell, or a fibroblast cell, or an endothelial cell, or a muscle cell.

18. A method as claimed in any one of claims 1 to 17, wherein the transfer of the genetic material from the non-human somatic cell to a non-human recipient cell provides a non-human animal embryo.

19. A method, as claimed in any one of claims 1 to 18, further comprising the production of a non-human totipotent or pluripotent cloned cell population.

20. A transgenic non-human embryo or a transgenic non-human fetus obtainable by a method as claimed claim 18, wherein the genome contains exogenous DNA.

21. A transgenic non-human cell derived from the non-human embryo or non-human fetus of claim 20.

22. A method according to claim 18, wherein a cell from the non-human animal embryo is used for further nuclear transfer.

23. A method for preparing a transgenic non-human animal, comprising causing a non-human animal to develop to term from the non-human embryo as claimed in claim 20 and, optionally, breeding from the animal.

24. A transgenic animal obtainable by the method as claimed in claim 23.

25. A transgenic animal as claimed in claim 24 which is a sheep, cow, bull, goat, pig, horse, camel, rabbit, or rodent.

26. A transgenic animal which is bred from an animal as claimed in claim 24 or claim 25.

27. A method for obtaining a clonal non-human pluripotent or totipotent cell population comprising culturing a cell line from a non-human transgenic embryo or a non-human transgenic fetus as claimed in claim 20.

28. A clonal non-human pluripotent or totipotent cell population obtainable according to a method as claimed in claim 27.

## Patentansprüche

1. Kerntransferverfahren, umfassend:
(a) Herstellen einer nicht-humanen somatischen Zelle für Kerntransfer durch ein Verfahren, umfassend das Verändern des genetischen Materials der nicht-humanen somatischen Zelle durch ein genetisches Targeting-Ereignis an einem endogenen Locus, und
(b) Transferieren des genetischen Materials von der nicht-humanen somatischen Zelle zu einer nicht-humanen Empfängerzelle.

2. Verfahren wie in Anspruch 1 beansprucht, wobei das genetische Targeting-Ereignis durch homologe Rekombination vermittelt wird.

3. Verfahren wie in Anspruch 1 oder Anspruch 2 beansprucht, wobei die Veränderung Inaktivierung, Entfernen oder Verändern eines Gens, Hochregulierung eines Gens, Genersatz oder Einbringen eines Transgens ist.

4. Verfahren wie in einem der Ansprüche 1 bis 3 beansprucht, wobei das genetische Targeting-Ereignis zu einem Verhältnis zwischen einem Zellklon, bei dem auf eine Gen abgezielt wurde, und einem Zellklon, bei dem zufällig gezielt wurde, (*gene targeted cell clone: randomly targeted cell clone ratio*) von gleich oder größer 1:100 führt.

5. Verfahren wie in einem der Ansprüche 1 bis 4 beansprucht, wobei das genetische Targeting-Ereignis an einem Locus durchgeführt wird, der in der somatischen Wirtszelle reichlich exprimiert wird.

6. Verfahren wie in einem der Ansprüche 1 bis 5 beansprucht, wobei ein strukturelles Gen neben einem endogenen Promotor plaziert wird.

7. Verfahren wie in Anspruch 6 beansprucht, wobei der endogene Promotor der des Kollagen-Gens ist.

8. Verfahren wie in Anspruch 6 beansprucht, wobei der endogene Promotor der eines Milchprotein-Gens ist.

9. Verfahren wie in Anspruch 6 beansprucht, wobei der endogene Promotor reichliche Expression in Fibroblastenzellen steuert.

10. Verfahren wie in Anspruch 6 beansprucht, wobei der endogene Promotor reichliche Expression in endothelialen Zellen steuert.

11. Verfahren wie in einem der Ansprüche 1 bis 10 beansprucht, wobei das genetische Targeting-Ereignis durch Lipofektion vermittelt wird.

12. Verfahren wie in einem der Ansprüche 1 bis 11 beansprucht, wobei das genetische Targeting-Ereignis die Verwendung eines Gentargeting-Vektors einschließt, wobei der Vektor einen langen Homologie-Bereich mit dem Ziellocus umfaßt.

13. Verfahren wie in einem der Ansprüche 1 bis 12 beansprucht, wobei das genetische Targeting-Ereignis die Verwendung eines Gentargeting-Vektors einschließt, der ringförmig ist.

14. Verfahren wie in einem der Ansprüche 1 bis 13 beansprucht, wobei das genetische Targeting-Ereignis die künstliche Induktion der Genexpression oder die Induktion von Chromatinveränderungen in der Zelle beinhaltet.

15. Verfahren wie in einem der Ansprüche 1 bis 14 beansprucht, wobei das genetische Targeting-Ereignis durch ein Mittel, das Histondeacetylierung inhibiert, oder durch Expression eines Faktors in der Zelle, der die Transkription am Ziellocus stimuliert, ermöglicht wird.

16. Verfahren wie in einem der Ansprüche 1 bis 15 beansprucht, wobei die nicht-humane somatische Zelle eine primäre somatische Zelle ist.

17. Verfahren wie in einem der Ansprüche 1 bis 16 beansprucht, wobei die nicht-humane somatische Zelle eine epitheliale Zelle oder eine Fibroblastenzelle oder eine endotheliale Zelle oder eine Muskelzelle ist.

18. Verfahren wie in einem der Ansprüche 1 bis 17 beansprucht, wobei der Transfer des genetischen Materials von der nicht-humanen somatischen Zelle zu einer nicht-humanen Empfängerzelle einen nicht-humanen Tierembryo bildet.

19. Verfahren wie in einem der Ansprüche 1 bis 18 beansprucht, weiter die Herstellung einer nicht-humanen, totipotenten oder pluripotenten, klonierten Zellpopulation umfassend.

20. Transgener, nicht-humaner Embryo oder transgener, nicht-humaner Fötus, erhältlich durch ein Verfahren wie in Anspruch 18 beansprucht, wobei das Genom exogene DNA enthält.

21. Transgene, nicht-humane Zelle, die von dem nicht-humanen Embryo oder nicht-humanen Fötus nach Anspruch 20 stammt.

22. Verfahren nach Anspruch 18, wobei eine Zelle von dem nicht-humanen Tierembryo für weiteren Kerntransfer verwendet wird.

23. Verfahren zur Herstellung eines transgenen, nicht-humanen Tieres, umfassend das Herbeiführen der Entwicklung eines nicht-humanen Tiers zur Reife aus dem nicht-humanen Embryo wie in Anspruch 20 beansprucht und, gegebenenfalls, Züchten von dem Tier.

24. Transgenes Tier, erhältlich durch das Verfahren wie in Anspruch 23 beansprucht.

25. Transgenes Tier wie in Anspruch 24 beansprucht, welches ein Schaf, eine Kuh, ein Stier, eine Ziege, ein Schwein, ein Pferd, ein Kamel, ein Kaninchen oder Nagetier ist.

26. Transgenes Tier, das von einem Tier, wie in Anspruch 24 oder Anspruch 25 beansprucht, gezüchtet wird.

27. Verfahren zum Erhalten einer klonalen, nicht-humanen, pluripotenten oder totipotenten Zellpopulation, umfassend das Kultivieren einer Zelllinie von einem nicht-humanen, transgenen Embryo oder einem nicht-humanen, transgenen Fötus wie in Anspruch 20 beansprucht.

28. Klonale, nicht-humane, pluripotente oder totipotente Zellpopulation, erhältlich nach einem Verfahren wie in Anspruch 27 beansprucht.

## Revendications

1. Procédé de transfert nucléaire comprenant :
a) la préparation d'une cellule somatique non humaine pour le transfert nucléaire à l'aide d'un procédé comprenant la modification du matériel génétique de la cellule somatique non humaine par un événement de ciblage de gène au niveau d'un locus endogène ; et
b) le transfert du matériel génétique provenant de la cellule somatique non humaine dans une cellule receveuse non humaine.

2. Procédé selon la revendication 1, dans lequel l'événement de ciblage de gène est effectué par recombinaison homologue.

3. Procédé selon la revendication 1 ou 2, dans lequel la modification est l'inactivation, la suppression ou la modification d'un gène, la régulation à la hausse d'un gène, le remplacement d'un gène ou l'insertion d'un transgène.

4. Procédé selon l'une des revendications 1 à 3, dans lequel l'événement de ciblage de gène donne lieu à un rapport entre clone de cellule de gène ciblé et clone de cellule ciblé de façon aléatoire égal ou supérieur à 1:100.

5. Procédé selon l'une des revendications 1 à 4, dans lequel l'événement de ciblage de gène a lieu dans un locus abondamment exprimé dans la cellule somatique hôte.

6. Procédé selon l'une des revendications 1 à 5, dans lequel un gène structural est placé de manière adjacente à un promoteur endogène.

7. Procédé selon la revendication 6, dans lequel le promoteur endogène est celui d'un gène du collagène.

8. Procédé selon la revendication 6, dans lequel le promoteur endogène est celui d'un gène d'une protéine du lait.

9. Procédé selon la revendication 6, dans lequel le promoteur endogène dirige une expression abondante dans des cellules de fibroblaste.

10. Procédé selon la revendication 6, dans lequel le promoteur endogène dirige une expression abondante dans des cellules endothéliales.

11. Procédé selon l'une des revendications 1 à 10, dans lequel l'événement de ciblage de gène est effectué par lipofection.

12. Procédé selon l'une des revendications 1 à 11, dans lequel l'événement de ciblage de gène implique l'utilisation d'un vecteur de ciblage de gène, lequel vecteur comporte une région longue d'homologie avec le locus cible.

13. Procédé selon l'une des revendications 1 à 12, dans lequel l'événement de ciblage de gène implique l'utilisation d'un vecteur de ciblage de gène, lequel vecteur présente une forme circulaire.

14. Procédé selon l'une des revendications 1 à 13, dans lequel l'événement de ciblage de gène comprend l'induction artificielle de l'expression du gène ou l'induction de changements dans la chromatine au sein de la cellule.

15. Procédé selon l'une des revendications 1 à 14, dans lequel l'événement de ciblage de gène est favorisé par un agent inhibant la désacétylation de l'histone ou par l'expression dans la cellule d'un facteur qui stimule la transcription au niveau du locus cible.

16. Procédé selon l'une des revendications 1 à 15, dans lequel la cellule somatique non humaine est une cellule somatique primaire.

17. Procédé selon l'une des revendications 1 à 16, dans lequel la cellule somatique non humaine est une cellule épithéliale ou une cellule fibroblaste, ou une cellule endothéliale, ou encore une cellule musculaire.

18. Procédé selon l'une des revendications 1 à 17, dans lequel le transfert du matériel génétique de la cellule somatique non humaine à la cellule receveuse non humaine produit un embryon animal non humain.

19. Procédé selon l'une des revendications 1 à 18, comprenant en outre la production d'une population cellulaire clonée non humaine totipotente ou pluripotente.

20. Embryon transgénique non humain ou foetus transgénique non humain, pouvant être obtenu à l'aide d'un procédé selon la revendication 18, dans lequel le génome contient de l'ADN exogène.

21. Cellule transgénique non humaine issue de l'embryon non humain ou du foetus non humain selon la revendication 20.

22. Procédé selon la revendication 18, dans lequel on utilise une cellule de l'embryon animal non humain pour un transfert nucléaire ultérieur.

23. Procédé d'obtention d'un animal transgénique non humain, comprenant l'induction du développement à terme d'un animal non humain à partir de l'embryon non humain tel que revendiqué dans la revendication 20, puis éventuellement la reproduction à partir de l'animal.

24. Animal transgénique pouvant être obtenu à l'aide du procédé selon la revendication 23.

25. Animal transgénique selon la revendication 24, qui est un mouton, une vache, un taureau, une chèvre, un porc, un cheval, un chameau, un lapin, ou un rongeur.

26. Animal transgénique reproduit à partir d'un animal selon la revendication 24 ou 25.

27. Procédé d'obtention d'une population cellulaire clonée non humaine pluripotente ou totipotente comprenant la culture d'une lignée cellulaire issue d'un embryon transgénique non humain ou d'un foetus transgénique non humain, tel que revendiqué selon la revendication 20.

28. Population cellulaire clonée non humaine pluripotente ou totipotente pouvant être obtenue à l'aide d'un procédé tel que revendiqué selon la revendication 27.
